# EUROPÄISCHE PATENTSCHRIFT

(11) **EP 2 585 555 B1**
(45) Veröffentlichungstag und Bekanntmachung des Hinweises auf die Patenterteilung: **30.07.2014**
(21) Anmeldenummer: 11724555.5
(22) Anmeldetag: 01.06.2011
(51) Int. Cl.: C09K 19/18, C09K 19/32, C09K 19/20, C07C 69/602, C09K 19/30, C09K 19/04, C09K 19/54, G02F 1/1333

(54) **POLYMERISIERBARE VERBINDUNGEN UND IHRE VERWENDUNG IN FLÜSSIGKRISTALLANZEIGEN**
POLYMERIZIBLE COMPOUNDS AND USE THEREOF IN LIQUID CRYSTAL DISPLAYS
COMPOSES POLYMERISABLES ET UTILISATION DE CEUX-CI DANS DES ECRANS A CRISTAUX LIQUIDES

(30) Priorität: 25.06.2010 DE 102010025091
(43) Veröffentlichungstag der Anmeldung: 01.05.2013
(73) Patentinhaber: Merck Patent GmbH, 64293 Darmstadt (DE)
(72) Erfinder: TAUGERBECK, Andreas, 64285 Darmstadt (DE); GOETZ, Achim, 64665 Alsbach-Hähnlein (DE); DEROW, Stephan, 64347 Griesheim (DE)
(74) Vertreter: Derow, Stephan
(86) Internationale Anmeldenummer: PCT/EP2011/002726
(87) Internationale Veröffentlichungsnummer: WO 2011/160765

(56) Entgegenhaltungen:
- EP-A1- 2 218 764
- WO-A1-2009/086911
- DE-A1- 10 064 291
- DE-A1-102008 036 248
- DE-A1-102009 011 652
- US-A1- 2008 143 943
- US-A1- 2008 236 727
- US-A1- 2009 109 392
- US-A1- 2009 324 855
- YAO Y-H ET AL: "SYNTHESIS OF UV-CURABLE LIQUID CRYSTALLINE DIACRYLATES FOR THE APPLICATION OF POLARIZED ELECTROLUMINESCENCE", LIQUID CRYSTALS: AN INTERNATIONAL JOURNAL OF SCIENCE AND TECHNOLOGY, TAYLOR & FRANCIS, GB, Bd. 33, Nr. 1, 1. Januar 2006 (2006-01-01) , Seiten 33-39, XP001239489, ISSN: 0267-8292, DOI: 10.1080/02678290500450931
- SEKINE C ET AL: "HIGH BIREFRINGENCE PHOTOPOLYMERIZABLE PHENYLACETYLENE LIQUID CRYSTALS", LIQUID CRYSTALS: AN INTERNATIONAL JOURNAL OF SCIENCE AND TECHNOLOGY, TAYLOR & FRANCIS, GB, Bd. 28, Nr. 10, 1. Oktober 2001 (2001-10-01), Seiten 1505-1512, XP001117381, ISSN: 0267-8292, DOI: 10.1080/02678290110068947

## Beschreibung

Die vorliegende Erfindung betrifft polymerisierbare Verbindungen, Verfahren und Zwischenprodukte zu ihrer Herstellung, und ihre Verwendung für optische, elektrooptische und elektronische Zwecke, insbesondere in Flüssigkristall (FK)-Medien und FK-Anzeigen, vor allem in FK-Anzeigen des PS- oder PSA-Typs ("polymer sustained" bzw. "polymer sustained alignment").

Die derzeit verwendeten Flüssigkristallanzeigen (FK-Anzeigen) sind meist solche des TN-Typs ("twisted nematic"). Diese weisen allerdings den Nachteil einer starken Blickwinkelabhängigkeit des Kontrastes auf. Daneben sind sogenannte VA-Anzeigen ("vertically aligned") bekannt, die einen breiteren Blickwinkel aufweisen. Die FK-Zelle einer VA-Anzeige enthält eine Schicht eines FK-Mediums zwischen zwei transparenten Elektroden, wobei das FK-Medium üblicherweise einen negativen Wert der dielektrischen (DK-) Anisotropie aufweist. Die Moleküle der FK-Schicht sind im ausgeschalteten Zustand senkrecht zu den Elektrodenflächen (homöotrop) oder gekippt homöotrop ("tilted") orientiert. Bei Anlegen einer elektrischen Spannung an die beiden Elektroden findet eine Umorientierung der FK-Moleküle parallel zu den Elektrodenflächen statt.

Weiterhin sind OCB-Anzeigen ("optically compensated bend") bekannt, die auf einem Doppelbrechungseffekt beruhen und eine FK-Schicht mit einer sogenannten "bend"-Orientierung und üblicherweise positiver (DK-) Anisotropie aufweisen. Bei Anlegen einer elektrischen Spannung findet eine Umorientierung der FK-Moleküle senkrecht zu den Elektrodenflächen statt. Darüber hinaus enthalten OCB-Anzeigen normalerweise einen oder mehrere doppelbrechende optische Retardationsfilme, um unerwünschte Lichtdurchlässigkeit der "bend"-Zelle im dunklen Zustand zu vermeiden. OCB-Anzeigen besitzen gegenüber TN-Anzeigen einen weiteren Blickwinkel und kürzere Schaltzeiten.

Weiterhin sind sogenannte IPS-Anzeigen ("In-Plane Switching") bekannt, die eine FK-Schicht zwischen zwei Substraten enthalten, wobei die beiden Elektroden nur auf einem der beiden Substrate angeordnet sind, und vorzugsweise ineinander verzahnte, kammförmige Strukturen aufweisen. Dadurch wird bei Anlegen einer Spannung an die Elektroden ein elektrisches Feld zwsichen ihnen erzeugt, welches eine signifikante Komponente parallel zur FK-Schicht aufweist. Dies bewirkt eine Umorientierung der FK-Moleküle in der Schichtebene.

Des weiteren wurden sogenannte FFS-Anzeigen ("Fringe-Field-Switching") vorgeschlagen (siehe u.a. S.H. Jung et al., Jpn. J. Appl. Phys., Band 43, No. 3, 2004, 1028), die ebenfalls zwei Elektroden auf dem gleichen Substrat beinhalten, wovon jedoch im Gegensatz zu IPS-Anzeigen nur eine als kammförmig strukturierte Elektrode ausgebildet ist, und die andere Elektrode unstrukturiert ist. Dadurch wird ein starkes sogenanntes "fringe field" erzeugt, also ein starkes elektrisches Feld nahe am Rand der Elektroden und in der gesamten Zelle ein elektrisches Feld, welches sowohl eine starke vertikale als auch eine starke horizontale Komponente aufweist. Sowohl IPS-Anzeigen als auch FFS-Anzeigen weisen eine geringe Blickwinkelabhängigkeit des Kontrastes auf.

In VA-Anzeigen des neueren Typs ist die einheitliche Ausrichtung der FK-Moleküle auf mehrere kleinere Domänen innerhalb der FK-Zelle beschränkt. Zwischen diesen Domänen, auch als Tilt-Domänen (engl. "tilt domains") bezeichnet, können Disklinationen existieren. VA-Anzeigen mit Tilt-Domänen weisen, verglichen mit herkömmlichen VA-Anzeigen, eine größere Blickwinkelunabhängigkeit des Kontrastes und der Graustufen auf. Außerdem sind solche Anzeigen einfacher herzustellen, da eine zusätzliche Behandlung der Elektrodenoberfläche zur einheitlichen Orientierung der Moleküle im eingeschalteten Zustand, wie z.B. durch Reiben, nicht mehr notwendig ist. Stattdessen wird die Vorzugsrichtung des Kipp- oder Tiltwinkels (engl. "pretilt") durch eine spezielle Ausgestaltung der Elektroden kontrolliert.

In den sogenannten MVA-Anzeigen ("multidomain vertical alignment") wird dies üblicherweise dadurch erreicht, dass die Elektroden Erhebungen oder Vorsprünge (engl. "protrusions") aufweisen, die einen lokalen pretilt verursachen. Als Folge werden die FK-Moleküle beim Anlegen einer Spannung in verschiedenen, definierten Regionen der Zelle in unterschiedliche Richtungen parallel zu den Elektrodenflächen orientiert. Dadurch wird ein "kontrolliertes" Schalten erreicht und das Entstehen störender Disklinationslinien vermieden. Diese Anordnung verbessert zwar den Blickwinkel der Anzeige, führt aber zu einer Verringerung ihrer Lichtdurchlässigkeit.

Eine Weiterentwicklung von MVA verwendet Protrusions nur auf einer Elektroden-Seite, die gegenüberliegende Elektrode weist hingegen Schlitze (engl. "slits") auf, was die Lichtdurchlässigkeit verbessert. Die geschlitzten Elektroden erzeugen beim Anlegen einer Spannung ein inhomogenes elektrisches Feld in der FK-Zelle, so dass weiterhin ein kontrolliertes Schalten erreicht wird. Zur weiteren Verbesserung der Lichtdurchlässigkeit können die Abstände zwischen den slits und protrusions vergrößert werden, was jedoch wiederum zu einer Verlängerung der Schaltzeiten führt.

Beim sogenannten PVA (Patterned VA) kommt man ganz ohne Protrusions aus, indem man beide Elektroden auf den gegenüberliegenden Seiten durch Schlitze strukturiert, was zu einem erhöhten Kontrast und verbesserter Lichtdurchlässigkeit führt, aber technologisch schwierig ist und das Display empfindlicher gegen mechanische Einflüsse macht (Klopfen, engl. "tapping", etc.). Für viele Anwendungen, wie beispielsweise Monitore und vor allem TV-Bildschirme, ist jedoch eine Verkürzung der Schaltzeiten sowie eine Verbesserung des Kontrastes und der Luminanz (Transmission) der Anzeige gefragt.

Eine Weiterentwicklung stellen die sogenannten PS-bzw. PSA-Anzeigen ("Polymer Sustained" bzw. "Polymer Sustained Alignment") dar, für die auch gelegentlich der Begriff "Polymer Stabilized" verwendet wird. In diesen Anzeigen wird dem FK-Medium eine geringe Menge (zum Beispiel 0.3 Gew.%, typischerweise <1 Gew.%) einer oder mehrerer polymerisierbarer Verbindung(en) zugesetzt, welche nach Einfüllen in die FK-Zelle mit oder ohne angelegte elektrische Spannung zwischen den Elektroden in situ polymerisiert bzw. vernetzt wird, üblicherweise durch UV-Photopolymerisation. Als besonders geeignet hat sich der Zusatz von polymerisierbaren mesogenen oder flüssigkristallinen Verbindungen, auch als reaktive Mesogene oder "RM"s bezeichnet, zur FK-Mischung erwiesen.

Nachfolgend wir der Begriff "PSA", falls nicht anders angegeben, stellvertretend für PS-Anzeigen und PSA-Anzeigen verwendet.

Mittlerweile wird das PS(A)-Prinzip in diversen klassischen FK-Anzeigen angewendet. So sind beispielsweise PSA-VA-, PSA-OCB-, PSA-IPS-, PSA-FFS- und PSA-TN-Anzeigen bekannt. Die Polymerisation der polymerisierbaren Verbindung(en) erfolgt bei PSA-VA- und PSA-OCB-Anzeigen vorzugsweise bei angelegter elektrischer Spannung, bei PSA-IPS-Anzeigen mit oder ohne angelegte elektrische Spannung. Wie man in Testzellen nachweisen kann, führt das PS(A)-Verfahren zu einem pretilt in der Zelle. Bei PSA-OCB-Anzeigen beispielsweise kann man erreichen, dass die Bend-Struktur stabilisiert wird, so dass man ohne Offset-Spannung auskommt oder diese reduzieren kann. Im Falle von PSA-VA-Anzeigen wirkt sich der pretilt positiv auf die Schaltzeiten aus. Für PSA-VA-Anzeigen kann ein Standard-MVA- bzw. -PVA Pixel- und Elektroden-Layout verwendet werden. Darüber hinaus kann man aber beispielsweise auch mit nur einer strukturierten Elektrodenseite und ohne Protrusions auskommen, was die Herstellung wesentlich vereinfacht und gleichzeitig zu einem sehr guten Kontrast bei sehr guter Lichtdurchlässigkeit führt.

Außerdem haben sich sogenannte Posi-VA-Anzeigen ("Positiv-VA") als besonders günstige Ausführungsform erwiesen. Die Ausgangsorientierung der Flüssigkristalle im spannungsfreien Ausgangszustand ist hier, ebenso wie bei klassischen VA-Anzeigen, homöotrop, also im wesentlichen senkrecht zu den Substraten. Im Gegensatz zu klassischen VA-Anzeigen werden in Posi-VA-Anzeigen jedoch dielektrisch positive FK-Medien verwendet. Durch Anlegen einer elektrischen Spannung an interdigitale Elektroden, die ein im wesentlichen zur Schicht des FK-Mediums paralleles Feld erzeugen, werden die FK-Moleküle in eine im wesentlichen zu den Substraten parallele Orientierung überführt. Derartige interdigitale Elektroden werden auch üblicherweise bei IPS-Anzeigen verwendet. Auch bei Posi-VA-Anzeigen hat sich eine entsprechende Polymerstabilisierung (PSA) als vorteilhaft erwiesen, wodurch eine erhebliche Reduzierung der Schaltzeiten realisiert werden konnte.

PSA-VA-Anzeigen sind beispielsweise in JP 10-036847 A, EP 1 170 626 A2, US 6,861,107, US 7,169,449, US 2004/0191428 A1, US 2006/0066793 A1 und US 2006/0103804 A1 beschrieben. PSA-OCB-Anzeigen sind beispielsweise in T.-J- Chen et al., Jpn. J. Appl. Phys. 45, 2006, 2702-2704 und S. H. Kim, L.-C- Chien, Jpn. J. Appl. Phys. 43, 2004, 7643-7647 beschrieben. PSA-IPS-Anzeigen sind zum Beispiel in US 6,177,972 und Appl. Phys. Lett. 1999, 75(21), 3264 beschrieben. PSA-TN-Anzeigen sind zum Beispiel in Optics Express 2004, 12(7), 1221 beschrieben.

PSA-Anzeigen können ebenso wie die oben beschriebenen konventionallen FK-Anzeigen als Aktivmatrix- oder Passivmatrix-Anzeigen betrieben werden. Bei Aktivmatrix-Anzeigen erfolgt die Ansteuerung einzelner Bildpunkte üblicherweise durch integrierte, nicht-lineare aktive Elemente wie beispielsweise Transistoren (z.B. Dünnfilmtransistoren, engl. "thin film transistor" bzw. "TFT"), bei Passivmatrix-Anzeigen üblicherweise nach dem Multiplex-Verfahren, wobei beide Verfahren aus dem Stand der Technik bekannt sind.

Insbesondere für Monitor- und vor allem TV-Anwendungen ist nach wie vor die Optimierung der Schaltzeiten, wie aber auch des Kontrastes und der Luminanz (also auch Transmission) der FK-Anzeige gefragt. Hier kann das PSA-Verfahren entscheidende Vorteile bringen. Insbesondere bei PSA-VA-, PSA-IPS-, PSA-FFS- und PSA-Posi-VA-Anzeigen kann man ohne nennenswerte Einbußen sonstiger Parameter eine Verkürzung der Schaltzeiten erreichen, die mit einem in Testzellen messbaren pretilt korrelieren.

Im Stand der Technik werden beispielsweise polymerisierbare Verbindungen der folgenen Formel verwendet 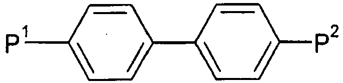 worin P eine polymerisierbare Gruppe, üblicherweise eine Acrylat- oder Methacrylategruppe bedeutet, wie beispielsweise in US 7,169,449 beschrieben.

Es ergibt sich jedoch das Problem, dass nicht alle Kombinationen bestehend aus FK-Mischung (nachfolgend auch als "FK-Hostmischung" bezeichnet) + polymerisierbarer Komponente (typischerweise RMs) für PSA-Anzeigen geeignet sind, weil sich zum Beispiel kein oder kein ausreichender Tilt einstellt, oder weil zum Beispiel die sogenannte "Voltage Holding Ratio" (VHR oder HR) für TFT-Displayanwendungen unzureichend ist. Zudem hat sich gezeigt, dass bei Verwendung in PSA-Anzeigen die aus dem Stand der Technik bekannten FK-Mischungen und RMs noch einige Nachteile aufweisen. So eignet sich nicht jedes bekannte, in FK-Mischungen lösliche RM zur Verwendung in PSA-Anzeigen. Ausserdem ist es oft schwierig, neben der direkten Messung des pretilts in der PSA-Anzeige ein geeignetes Auswahlkriterium für das RM zu finden. Noch kleiner wird die Auswahl geeigneter RMs, wenn eine Polymerisation mittels UV-Licht ohne den Zusatz von Photoinitiatoren gewünscht ist, was für bestimmte Anwendungen von Vorteil sein kann.

Darüber hinaus sollte die gewählte Kombination FK-Hostmischung/RM eine möglichst geringe Rotationsviskosität sowie möglichst gute elektrische Eigenschaften aufweisen, insbesondere sollte sie eine möglichst hohe VHR besitzen. Bei PSA-Anzeigen ist vor allem eine hohe VHR nach Bestrahlung mit UV-Licht erforderlich, da die UV-Belichtung ein notwendiger Teil des Herstellungsprozesses der Anzeige ist, aber auch als normale Belastung im Betrieb der fertigen Anzeige auftritt.

Insbesondere wäre es wünschenswert, neue Materialien für PSA-Anzeigen zur Verfügung zu haben, die einen besonders kleinen pretilt-Winkel erzeugen. Hierbei sind Materialien bevorzugt, die während der Polymerisation bei gleicher Belichtungszeit einen niedrigeren pretilt-Winkel erzeugen als die bisher bekannten Materialien, und/oder durch deren Verwendung der mit den bekannten Materialien erzielbare (höhere) pretilt-Winkel bereits nach kürzerer Belichtungszeit erreicht werden kann. Dadurch könnten die Produktionszeit (engl. "tact time") der Anzeige verkürzt und die Kosten des Produktionsprozesses verringert werden.

Ein weiteres Problem bei der Herstellung von PSA-Anzeigen ist das Vorhandensein bzw. die Entfernung von Restmengen unpolymerisierter RMs insbesondere nach dem Polymerisationsschritt zur Erzeugung des pretilt-Winkels in der Anzeige. Beispielsweise können solche nicht abreagierten RMs die Eigenschaften der Anzeige nachteilig beeinflussen, indem sie z.B. nach Fertigstellung der Anzeige während des Betriebes unkontrolliert polymerisieren.

So zeigen die aus dem Stand der Technik bekannten PSA-Anzeigen oft den unerwünschten Effekt des sogenannten "image sticking" oder "image burn", d.h. dass das in der FK-Anzeige durch vorübergehende Ansteuerung einzelner Bildpunkte (pixel) erzeugte Bild auch nach Abschalten des elektrischen Feldes in diesen Bildpunkten, oder nach Ansteuerung anderer Bildpunkte, noch sichtbar bleibt.

Dieses "image sticking" kann einerseits auftreten, wenn FK-Hostmischungen mit einem geringen VHR verwendet wird. In diesen kann die UV-Komponente des Tageslichtes oder der Hintergrundbeleuchtung unerwünschte Zerfallsreaktionen der FK-Moleküle und dadurch die Erzeugung von ionischen oder radikalischen Verunreinigungen auslösen. Diese können sich insbesondere an den Elektroden bzw. den Orientierungsschichten anreichern und dort die effektive angelegte Spannung reduzieren. Dieser Effekt ist auch bei herkömmlichen FK-Anzeigen ohne Polymerkomponente zu beobachten.

In PSA-Anzeigen wird darüber hinaus oft ein zusätzlicher "image sticking"-Effekt beobachtet, der durch die Gegenwart von unpolymerisierten RMs hervorgerufen wird. Dabei wird durch UV-Licht aus der Umgebung oder von der Hintergrundbeleuchtung eine unkontrollierte Polymerisation der Rest-RMs ausgelöst. In den geschalteten Displaybereichen wird dadurch nach mehreren Ansteuerungszyklen der Tiltwinkel verändert. Als Resultat kann eine Transmissionsänderung in den geschalteten Bereichen auftreten, während sie in den ungeschalteten Bereichen unverändert bleibt.

Es ist deshalb wünschenswert, dass die Polymerisation der RMs bei der Herstellung der PSA-Anzeige möglichst vollständig abläuft und die Anwesenheit von unpolymerisierten RMs in der Anzeige möglichst ausgeschlossen oder auf ein Minimum reduziert wird. Hierzu werden Materialien benötigt, die eine möglichst effektive und vollständige Polymerisation ermöglichen. Zudem wäre ein kontrolliertes Abreagieren dieser Restmengen wünschenswert. Dies wäre einfacher, wenn das RM schneller und effektiver polymerisiert als die bisher bekannten Materialien.

Es besteht somit immer noch ein großer Bedarf an PSA-Anzeigen, insbesondere vom VA- und OCB-Typ, sowie FK-Medien und polymerisierbaren Verbindungen zur Verwendung in solchen Anzeigen, welche die oben beschriebenen Nachteile nicht oder nur in geringem Maße zeigen und verbesserte Eigenschaften besitzen. Zudem besteht ein großer Bedarf nach PSA-Anzeigen, sowie Materialien zur Verwendung in PSA-Anzeigen, die vorteilhate Eigenschaften aufweisen, insbesondere einen hohen spezifischen Widerstand bei gleichzeitig großem Arbeitstemperaturbereich, kurze Schaltzeiten auch bei tiefen Temperaturen und niedriger Schwellenspannung, einen niedrigen pretilt-Winkel, eine Vielzahl von Graustufen, einen hohen Kontrast und einen weiten Blickwinkel ermöglichen, sowie hohe Werte der "voltage holding ratio" (VHR) nach UV-Belastung und der Tieftemperaturstabilität, auch als "LTS" (low temperature stability) bezeichnet, d.h. der Stabilität der FK-Mischung gegen spontane Auskristallisation einzelner Komponenten.

Der Erfindung liegt die Aufgabe zugrunde, neue geeignete Materialien, insbesondere RMs und diese enthaltende FK-Medien, für die Verwendung in PSA-Anzeigen bereitzustellen, die die oben angegebenen Nachteile nicht oder in geringerem Maße aufweisen, möglichst schnell und vollständig polymerisieren, eine möglichst schnelle Einstellung eines niedrigen pretilt-Winkels ermöglichen, das Auftreten von "image sticking" in der Anzeige verringern oder vermeiden, und vorzugsweise gleichzeitig sehr hohe spezifische Widerstände, niedrige Schwellenspannungen und niedrige Schaltzeiten ermöglichen. Zudem sollten die FK-Medien günstige FK-Phaseneigenschaften sowie hohe VHR- und LTS-Werte aufweisen. Weitere Aufgabe der Erfindung ist die Bereitstellung von neuen RMs, insbesondere für optische, elektrooptische und elektronische Anwendungen, sowie von geeigneten Verfahren und Zwischenprodukten zu ihrer Herstellung.

Insbesondere liegt der Erfindung die Aufgabe zugrunde, polymerisierbare Verbindungen bereitzustellen, die nach Photopolymerisation einen größeren maximalen Pretilt erzeugen, was zu einem schnelleren Erreichen des gewünschten Pretilts und so zu deutlich verkürzten Zeiten bei der Herstellung der FK-Anzeige führt

Diese Aufgabe wurde erfindungsgemäß gelöst durch die Bereitstellung von Materialien, Verfahren und FK-Anzeigen wie in der vorliegenden Anmeldung beschrieben. Insbesondere wurde überraschend gefunden, dass die oben beschriebenen Aufgaben teilweise oder vollständig gelöst werden können, indem man PSA-Anzeigen bereitstellt, die eine oder mehrere polymerisierte erfindungsgemäße Verbindungen enthalten, bzw. indem man zur Herstellung solcher PSA-Anzeigen FK-Medien verwendet, welche eine oder mehrere erfindungsgemäße polymerisierbare Verbindungen enthalten.

Die Verwendung solcher polymerisierbarer Verbindungen in erfindungsgemäßen FK-Medien und PSA-Anzeigen führt zu einem besonders schnellen Erreichen des gewünschten Pretilts und zu deutlich verkürzten Zeiten bei der Herstellung der Anzeige. Dies konnte in Verbindung mit einem FK-Medium mittels belichtungszeitabhängiger Pretilt-Messungen in VA-Tilt-Messzellen nachgewiesen werden. Insbesondere konnte ein Pretilt ohne den Zusatz von Photoinitiator erreicht werden.

Da die erfindungsgemäßen polymerisierbaren Verbindungen in den PSA-Anzeigen eine deutlich höhere Polymerisationsgeschwindigkeit zeigen, verbleiben auch weniger unreagierte Restmengen in der FK-Zelle, wodurch deren elektrooptische Eigenschaften verbessert werden, und das kontrollierte Abreagieren dieser Restmengen einfacher wird.

Die Verwendung von erfindungsgemäßen polymerisierbaren Verbindungen in PSA-Anzeigen zur schnellen Einstellung eines Tiltwinkels durch in situ-Polymerisation im elektrischen Feld wurde im Stand der Technik weder beschrieben noch nahegelegt.
WO 2009/086911 A1 offenbart monoreaktive polymerisierbare Tolanverbindungen mit einer terminalen Isothiocyanatgruppe. DE 100 64 291 A1 offenbart direaktive polymerisierbare Tolanverbindungen. Yao et al., Liquid Crystals 2006, 33(1), 33-39 und Sekine et al., Liquid Crystals 2001, 28(1), 1505-1512 offenbaren polymerisierbare Tolanverbindungen sowie Polymere erhältlich durch deren Polymerisation. EP 2 218 764 A1 offenbart eine chirale Mischung reaktiver Mesogene, welche auch polymerisierbare Tolanverbindungen enthält. US 2008/0236727 A1, US 2009/0324855 A1 und US 2009/0109392 A1 offenbaren FK-Medien enthaltend polymerisierbare Verbindungen sowie FK-Anzeigen enthaltend solche Medien. US 2008/0143943 A1 offenbart eine polymerisierbare FK-Mischung enthaltend eine polymerisierbare Tolanverbindung, sowie deren Verwendung zur Herstellung von optisch biaxialen Polymerfilmen. DE 10 2009 011 652 A1 und D11 DE 10 2008 036 248 A1 offenbaren PSA-Anzeigen. Die oben genannten Dokumente offenbaren jedoch weder polymerisierbare Tolanverbindungen gemäß der vorliegenden Erfindung zur Verwendung in PSA-Anzeigen, noch legen sie diese nahe.

Zudem wurde völlig überraschend gefunden, dass erfindungsgemäße polymerisierbare Verbindungen bei Verwendung in PSA-Anzeigen eine deutlich schnellere Tiltwinkel-Generierung und eine schnellere und vollständigere Polymerisation zeigen, als aus dem Stand der Technik bekannte polymerisierbare Verbindungen. Dies konnte durch direkte Vergleichsversuche bestätigt werden. Dieses Ergebnis war durch den Stand der Technik weder beschrieben noch nahegelegt.

Gegenstand der Erfindung ist somit die Verwendung von Verbindungen der Formel I (nachfolgend auch als "polymerisierbare erfindungsgemäße Verbindungen" bezeichnet)

P-(Sp)ₛ₁-A¹-Z¹-A²(-Z²-A³)ₘ-(Sp)ₛ₂-P I

worin die einzelnen Reste folgende Bedeutung besitzen
- P: bei jedem Auftreten gleich oder verschieden eine polymerisierbare Gruppe,
- Sp: bei jedem Auftreten gleich oder verschieden eine Abstandsgruppe,
- s1, s2: jeweils unabhängig voneinander 0 oder 1,
- A¹, A², A³: jeweils unabhängig voneinander 1,4-Phenylen, Naphthalin-1,4-diyl oder Naphthalin-2,6-diyl, wobei in diesen Gruppen auch eine oder mehrere CH-Gruppen durch N ersetzt sein können, Phenanthren-2,7-diyl, Anthracen-2,7-diyl, 9,10-Dihydrophenanthren-2,7-diyl, 9H-Fluoren-2,7-diyl, 9,9-Dimethylfluoren-2,7-diyl oder Dibenzofuran-3,7-diyl, wobei alle diese Gruppen unsubstituiert oder durch L ein- oder mehrfach substituiert sein können,
- m: 0, 1 oder 2,
- L: bei jedem Auftreten gleich oder verschieden P-, P-Sp-, OH, CH₂OH, Halogen, -CN, -NO₂, -NCO, -NCS, -OCN, -SCN, -C(=O)N(R^{x})₂, -C(=O)Y¹, -C(=O)R^{x}, -N(R^{x})₂, optional substituiertes Silyl oder eine optional substituierte Kohlenstoffgruppe oder Kohlenwasserstoffgruppe,
- R^{x}: P-, P-Sp-, H, Halogen, geradkettiges, verzweigtes oder cyclisches Alkyl mit 1 bis 25, vorzugsweise 1 bis 12 C-Atomen, worin auch eine oder mehrere nicht benachbarte CH₂-Gruppen durch -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, P- oder P-Sp- ersetzt sein können,
- Y¹: Halogen,
- Z¹, Z²: jeweils unabhängig voneinander -CO-O-, -OCO-, -CY=CY-, - C=C- oder eine Einfachbindung,
- Y: bei jedem Auftreten gleich oder verschieden H oder F,
wobei mindestens einer der Reste Z¹ und Z² -C≡C- bedeutet,

in Flüssigkristall-(FK-)medien und FK-Anzeigen des PS- oder PSA-(polymer sustained alignment) Typs.

Ein weiterer Gegenstand der Erfindung ist ein FK-Medium enthaltend eine oder mehrere Verbindungen der Formel I und eine oder mehrere zusätzliche Verbindungen, welche auch mesogen, flüssigkristallin und/oder polymerisierbar sein können, wie in Anspruch 9 oder 10 definiert.

Ein weiterer Gegenstand der Erfindung ist ein FK-Medium wie in Anspruch 9 oder 10 definiert, enthaltend ein Polymer erhältlich durch Polymerisation einer oder mehrerer Verbindungen der Formel I und eine oder mehrere zusätzliche Verbindungen, welche auch mesogen, flüssigkristallin und/oder polymerisierbar sein können.

Ein weiterer Gegenstand der Erfindung ist ein FK-Medium enthaltend
- eine polymerisierbare Komponente A), enthaltend eine oder mehrere Verbindungen der Formel I, sowie
- eine flüssigkristalline Komponente B), im Folgenden auch als "FK-Hostmischung" bezeichnet, enthaltend eine oder mehrere, vorzugsweise zwei oder mehr niedermolekulare (monomere und unpolymerisierbare) Verbindungen wie vor- und nachstehend beschrieben, wie in Anspruch 9 oder 10 definiert.

Ein weiterer Gegenstand der Erfindung ist ein Verfahren zur Herstellung eines FK-Mediums wie vor- und nachstehend beschrieben, indem man eine oder mehrere niedermolekulare flüssigkristalline Verbindungen, oder eine FK-Hostmischung wie vor- und nachstehend beschrieben, mit einer oder mehreren Verbindungen der Formel I und gegebenenfalls mit weiteren flüssigkristallinen Verbindungen und/oder Additiven, mischt.

Ein weiterer Gegenstand der Erfindung ist die Verwendung von Verbindungen der Formel I und erfindungsgemäßen FK-Medien in PS-und PSA-Anzeigen, insbesondere die Verwendung in PS- und PSA-Anzeigen enthaltend ein FK-Medium, zur Erzeugung eines Tiltwinkels im FK-Medium durch in situ-Polymerisation der Verbindung(en) der Formel I in der PSA-Anzeige, vorzugsweise unter Anlegen eines elektrischen oder magnetischen Feldes.

Ein weiterer Gegenstand der Erfindung ist eine FK-Anzeige enthaltend eine oder mehrere Verbindungen der Formel I oder ein erfindungsgemäßes FK-Medium, insbesondere eine PS- oder PSA-Anzeige, besonders bevorzugt eine PSA-VA-, PSA-OCB-, PSA-IPS-, PSA-FFS- PSA-Posi-VA- oder PSA-TN-Anzeige.

Ein weiterer Gegenstand der Erfindung ist eine FK-Anzeige des PS- oder PSA-Typs, enthaltend eine FK-Zelle mit zwei Substraten und zwei Elektroden, wobei mindestens ein Substrat lichtdurchlässig ist und mindestens ein Substrat eine oder zwei Elektroden aufweist, sowie einer zwischen den Substraten befindlichen Schicht eines FK-Mediums enthaltend eine polymerisierte Komponente und eine niedermolekulare Komponente, wobei die polymerisierte Komponente erhältlich ist durch Polymerisation einer oder mehrerer polymerisierbarer Verbindungen zwischen den Substraten der FK-Zelle im FK-Medium, vorzugsweise unter Anlegen einer elektrischen Spannung an die Elektroden, wobei mindestens eine der polymerisierbaren Verbindungen eine Verbindung der Formel I ist.

Ein weiterer Gegenstand der Erfindung ist ein Verfahren zur Herstellung einer FK-Anzeige wie vor- und nachstehend beschrieben, indem man ein FK-Medium, enthaltend eine oder mehrere niedermolekulare flüssigkristalline Verbindungen oder eine FK-Hostmischung wie vor- und nachstehend beschrieben sowie eine oder mehrere Verbindungen der Formel I, in eine FK-Zelle mit zwei Substraten und zwei Elektroden wie vor- und nachstehend beschrieben füllt, und die polymerisierbaren Verbindungen, vorzugsweise unter Anlegen einer elektrischen Spannung an die Elektroden, polymerisiert.

Die erfindungsgemäßen PS- und PSA-Anzeigen weisen zwei Elektroden, vorzugsweise in Form von transparenten Schichten, auf, wobei diese auf einem oder beiden der Substrate aufgebracht sind, die die FK-Zelle bilden. Dabei ist entweder jeweils eine Elektrode auf je einem der beiden Substrate aufgebracht, wie zum Beispiel in erfindungsgemäßen PSA-VA-, PSA-OCB- oder PSA-TN-Anzeigen, oder beide Elektroden sind auf nur einem der beiden Substrate aufgebracht, während das andere Substrat keine Elektrode aufweist, wie zum Beispiel in erfindungsgemäßen PSA-Posi-VA-, PSA-IPS- oder PSA-FFS-Anzeigen.

Weiterer Gegenstand der Erfindung sind neue Verbindungen der Formel I, Verfahren zu ihrer Herstellung, sowie in diesen Verfahren verwendete oder daraus erhaltene neue Zwischenprodukte.

Vor- und nachstehend gelten folgende Bedeutungen:
Die Begriffe "Tilt" und "Tiltwinkel" beziehen sich auf eine gekippte oder geneigte Orientierung der FK-Moleküle eines FK-Mediums relativ zu den Oberflächen der Zelle in einer FK-Anzeige (hier vorzugsweise einer PS- oder PSA-Anzeige). Der Tiltwinkel bezeichnet dabei den durchschnittlichen Winkel (<90°) zwischen den Moleküllängsachsen der FK-Moleküle (FK-Direktor) und der Oberfläche der planparallelen Trägerplatten, welche die FK-Zelle bilden. Ein niedriger Wert des Tiltwinkels (d.h. eine große Abweichung vom 90°-Winkel) entspricht dabei einem großen Tilt. Eine geeignete Methode zur Messung des Tiltwinkels findet sich in den Beispielen. Soweit nicht anders angegeben, beziehen sich vor- und nachstehend offenbarte Werte des Tiltwinkels auf diese Messmethode.

Der Begriff "mesogene Gruppe" ist dem Fachmann bekannt und in der Literatur beschrieben, und bedeutet eine Gruppe, die durch die Anisotropie ihrer anziehenden und abstoßenden Wechselwirkungen wesentlich dazu beiträgt, in niedermolekularen oder polymeren Substanzen eine Flüssigkristall(FK-)Phase hervorzurufen. Verbindungen enthaltend mesogene Gruppen (mesogene Verbindungen) müssen nicht unbedingt selbst eine FK-Phase aufweisen. Es ist auch möglich, dass mesogene Verbindungen FK-Phasenverhalten nur nach Vermischung mit anderen Verbindungen und/oder nach Polymerisation zeigen. Typische mesogene Gruppen sind beispielsweise starre stäbchen- oder scheibchenförmige Einheiten. Ein Überblick über die im Zusammenhang mit mesogenen bzw. FK-Verbindungen verwendeten Begriffe und Definitionen findet sich in Pure Appl. Chem. 73(5), 888 (2001) und C. Tschierske, G. Pelzl, S. Diele, Angew. Chem. 2004, 116, 6340-6368.

Der Begriff "Abstandsgruppe" (engl. "spacer" oder "spacer group"), vor-und nachstehend auch als "Sp" bezeichnet, ist dem Fachmann bekannt und in der Literatur beschrieben, siehe beispielsweise Pure Appl. Chem. 73(5), 888 (2001) und C. Tschierske, G. Pelzl, S. Diele, Angew. Chem. 2004, 116, 6340-6368. Falls nicht anders angegeben, bezeichnet der Begriff "Abstandsgruppe" bzw. "Spacer" vor- und nachstehend eine flexible Gruppe, die in einer polymerisierbaren mesogenen Verbindung die mesogene Gruppe und die polymerisierbare(n) Gruppe(n) miteinander verbindet.

Der Begriff "reaktives Mesogen" oder "RM" bezeichnet eine Verbindung enthaltend eine mesogene Gruppe und eine oder mehrere funktionelle Gruppen, die zur Polymerisation geeignet sind (auch als polymerisierbare Gruppe oder Gruppe P bezeichnet).

Die Begriffe "niedermolekulare Verbindung" und "unpolymerisierbare Verbindung" bezeichnen, üblicherweise monomere, Verbindungen, die keine funktionelle Gruppe aufweisen, welche zur Polymerisation unter den üblichen dem Fachmann bekannten Bedingungen, insbesondere unter den zur Polymerisation der RMs verwendeten Bedingungen, geeignet ist.

Die Begriffe "niedermolekulare Verbindung" und "unpolymerisierbare Verbindung" bezeichnen, üblicherweise monomere, Verbindungen, die keine funktionelle Gruppe aufweisen, welche zur Polymerisation unter den üblichen dem Fachmann bekannten Bedingungen, insbesondere unter den zur Polymerisation der RMs verwendeten Bedingungen, geeignet ist.

Der Begriff "organische Gruppe" bedeutet eine Kohlenstoff- oder Kohlenwasserstoffgruppe.

Der Begriff "Kohlenstoffgruppe" bedeutet eine ein- oder mehrbindige organische Gruppe enthaltend mindestens ein Kohlenstoffatom, vorzugsweise 1 bis 40 C-Atome, wobei diese entweder keine weiteren Atome enthält (wie z.B. -C≡C-), oder gegebenenfalls ein oder mehrere weitere Atome wie beispielsweise N, O, S, P, Si, Se, As, Te oder Ge enthält (z.B. Carbonyl etc.). Der Begriff "Kohlenwasserstoffgruppe" bedeutet eine Kohlenstoffgruppe, die zusätzlich ein oder mehrere H-Atome und gegebenenfalls ein oder mehrere Heteroatome wie beispielsweise N, O, S, P, Si, Se, As, Te oder Ge enthält.

"Halogen" bedeutet F, Cl, Br oder I.

Eine Kohlenstoff- oder Kohlenwasserstoffgruppe kann eine gesättigte oder ungesättigte Gruppe sein. Ungesättigte Gruppen sind beispielsweise Aryl-, Alkenyl- oder Alkinylgruppen. Ein Kohlenstoff- oder Kohlenwasserstoffrest mit mehr als 3 C-Atomen kann geradkettig, verzweigt und/oder cyclisch sein, und kann auch Spiroverküpfungen oder kondensierte Ringe aufweisen.

Die Begriffe "Alkyl", "Aryl", "Heteroaryl" etc. umfassen auch mehrbindige Gruppen, beispielsweise Alkylen, Arylen, Heteroarylen etc.

Der Begriff "Aryl" bedeutet eine aromatische Kohlenstoffgruppe oder eine davon abgeleitete Gruppe. Der Begriff "Heteroaryl" bedeutet "Aryl" gemäß vorstehender Definition, enthaltend ein oder mehrere Heteroatome.

Bevorzugte Kohlenstoff- und Kohlenwasserstoffgruppen sind gegebenenfalls substituiertes Alkyl, Alkenyl, Alkinyl, Alkoxy, Alkylcarbonyl, Alkoxycarbonyl, Alkylcarbonyloxy und Alkoxycarbonyloxy mit 1 bis 40, vorzugsweise 1 bis 25, besonders bevorzugt 1 bis 18 C-Atomen, gegebenenfalls substituiertes Aryl, Heteroaryl, Aryloxy oder Heteroaryloxy mit 1 bis 40, vorzugsweise 1 bis 25 C-Atomen, oder gegebenenfalls substituiertes Alkylaryl, Arylalkyl, Alkylaryloxy, Arylalkyloxy, Arylcarbonyl, Aryloxycarbonyl, Arylcarbonyloxy und Aryloxycarbonyloxy mit 6 bis 40, vorzugsweise 6 bis 25 C-Atomen.

Weitere bevorzugte Kohlenstoff- und Kohlenwasserstoffgruppen sind C₁-C₄₀ Alkyl, C₂-C₄₀ Alkenyl, C₂-C₄₀ Alkinyl, C₃-C₄₀ Allyl, C₄-C₄₀ Alkyldienyl, C₄-C₄₀ Polyenyl, C₆-C₄₀ Aryl, C₆-C₄₀ Alkylaryl, C₆-C₄₀ Arylalkyl, C₆-C₄₀ Alkylaryloxy, C₆-C₄₀ Arylalkyloxy, C₂-C₄₀ Heteroaryl, C₄-C₄₀ Cycloalkyl, C₄-C₄₀ Cycloalkenyl, etc. Besonders bevorzugt sind C₁-C₂₂ Alkyl, C₂-C₂₂ Alkenyl, C₂ -C₂₂ Alkinyl, C₃-C₂₂ Allyl, C₄-C₂₂ Alkyldienyl, C₆-C₁₂ Aryl, C₆-C₂₀ Arylalkyl und C₂-C₂₀ Heteroaryl.

Weitere bevorzugte Kohlenstoff- und Kohlenwasserstoffgruppen sind geradkettige, verzweigte oder cyclische Alkylreste mit 1 bis 40, vorzugsweise 1 bis 25 C-Atomen, welche unsubstituiert oder durch F, Cl, Br, I oder CN ein- oder mehrfach substituiert sind, und worin ein mehrere nicht benachbarte CH₂-Gruppen jeweils unabhängig voneinander durch - C(R^{x})=C(R^{x})-, -C≡C-, -N(R^{x})-, -O-, -S-, -CO-, -CO-O-, -O-CO-, -O-CO-O-so ersetzt sein können, dass O- und/oder S-Atome nicht direkt miteinander verknüpft sind.

R^{x} bedeutet vorzugsweise H, Halogen, eine geradkettige, verzweigte oder cyclische Alkylkette mit 1 bis 25 C-Atomen, in der auch ein oder mehrere nicht benachbarte C-Atome durch -O-, -S-, -CO-, -CO-O-, -O-CO-, -O-CO-O- ersetzt sein können, wobei auch ein oder mehrere H-Atome durch Fluor ersetzt sein können, eine optional substituierte Aryl- oder Aryloxygruppe mit 6 bis 40 C-Atomen, oder eine optional substituierte Heteroaryl- oder Heteroaryloxygruppe mit 2 bis 40 C-Atomen.

Bevorzugte Alkoxygruppen sind beispielsweise Methoxy, Ethoxy, 2-Methoxyethoxy, n-Propoxy, i-Propoxy, n-Butoxy, i-Butoxy, s-Butoxy, t-Butoxy, 2-Methylbutoxy, n-Pentoxy, n-Hexoxy, n-Heptoxy, n-Octoxy, n-Nonoxy, n-Decoxy, n-Undecoxy, n-Dodecoxy, etc.

Bevorzugte Alkylgruppen sind beispielsweise Methyl, Ethyl, n-Propyl, Isopropyl, n-Butyl, Isobutyl, s-Butyl, t-Butyl, 2-Methylbutyl, n-Pentyl, s-Pentyl, Cyclopentyl, n-Hexyl, Cyclohexyl, 2-Ethylhexyl, n-Heptyl, Cycloheptyl, n-Octyl, Cyclooctyl, n-Nonyl, n-Decyl, n-Undecyl, n-Dodecyl, Dodecanyl, Trifluormethyl, Perfluor-n-butyl, 2,2,2-Trifluorethyl, Perfluoroctyl, Perfluorhexyl etc.

Bevorzugte Alkenylgruppen sind beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl etc.

Bevorzugte Alkinylgruppen sind beispielsweise Ethinyl, Propinyl, Butinyl, Pentinyl, Hexinyl, Octinyl etc.

Bevorzugte Alkoxygruppen sind beispielsweise Methoxy, Ethoxy, 2-Methoxyethoxy, n-Propoxy, i-Propoxy, n-Butoxy, i-Butoxy, s-Butoxy, t-Butoxy, 2-Methylbutoxy, n-Pentoxy, n-Hexoxy, n-Heptoxy, n-Octoxy, n-Nonoxy, n-Decoxy, n-Undecoxy, n-Dodecoxy, etc.

Bevorzugte Aminogruppen sind beispielsweise Dimethylamino, Methylamino, Methylphenylamino, Phenylamino, etc.

Aryl- und Heteroarylgruppen können einkernig oder mehrkernig sein, d.h. sie können einen Ring (wie z.B. Phenyl) oder zwei oder mehr Ringe aufweisen, welche auch anelliert (wie z.B. Naphthyl) oder kovalent verknüpft sein können (wie z.B. Biphenyl), oder eine Kombination von anellierten und verknüpften Ringen beinhalten. Heteroarylgruppen enthalten ein oder mehrere Heteroatome, vorzugsweise ausgewählt aus O, N, S und Se.

Besonders bevorzugt sind ein-, zwei- oder dreikernige Arylgruppen mit 6 bis 25 C-Atomen sowie ein-, zwei- oder dreikernige Heteroarylgruppen mit 2 bis 25 C-Atomen, welche optional anellierte Ringe enthalten und optional substituiert sind. Ferner bevorzugt sind 5-, 6- oder 7-gliedrige Aryl- und Heteroarylgruppen, worin auch eine oder mehrere CH-Gruppen durch N, S oder O so ersetzt sein können, dass O-Atome und/oder S-Atome nicht direkt miteinander verknüpft sind.

Bevorzugte Arylgruppen sind beispielsweise Phenyl, Biphenyl, Terphenyl, [1,1':3',1"]Terphenyl-2'-yl, Naphthyl, Anthracen, Binaphthyl, Phenanthren, Pyren, Dihydropyren, Chrysen, Perylen, Tetracen, Pentacen, Benzpyren, Fluoren, Inden, Indenofluoren, Spirobifluoren, etc.

Bevorzugte Heteroarylgruppen sind beispielsweise 5-gliedrige Ringe wie Pyrrol, Pyrazol, Imidazol, 1,2,3-Triazol, 1,2,4-Triazol, Tetrazol, Furan, Thiophen, Selenophen, Oxazol, Isoxazol, 1,2-Thiazol, 1,3-Thiazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 6-gliedrige Ringe wie Pyridin, Pyridazin, Pyrimidin, Pyrazin, 1,3,5-Triazin, 1,2,4-Triazin, 1,2,3-Triazin, 1,2,4,5-Tetrazin, 1,2,3,4-Tetrazin, 1,2,3,5-Tetrazin, oder kondensierte Gruppen wie Indol, Isoindol, Indolizin, Indazol, Benzimidazol, Benzotriazol, Purin, Naphthimidazol, Phenanthrimidazol, Pyridimidazol, Pyrazinimidazol, Chinoxalinimidazol, Benzoxazol, Naphthoxazol, Anthroxazol, Phenanthroxazol, Isoxazol, Benzothiazol, Benzofuran, Isobenzofuran, Dibenzofuran, Chinolin, Isochinolin, Pteridin, Benzo-5,6-chinolin, Benzo-6,7-chinolin, Benzo-7,8-chinolin, Benzoisochinolin, Acridin, Phenothiazin, Phenoxazin, Benzopyridazin, Benzopyrimidin, Chinoxalin, Phenazin, Naphthyridin, Azacarbazol, Benzocarbolin, Phenanthridin, Phenanthrolin, Thieno[2,3b]thiophen, Thieno[3,2b]thiophen, Dithienothiophen, Isobenzothiophen, Dibenzothiophen, Benzothiadiazothiophen, oder Kombinationen dieser Gruppen. Die Heteroarylgruppen können auch mit Alkyl, Alkoxy, Thioalkyl, Fluor, Fluoralkyl oder weiteren Aryl- oder Heteroarylgruppen substituiert sein.

Die (nicht-aromatischen) alicyclischen und heterocyclischen Gruppen umfassen sowohl gesättigte Ringe, d.h. solche die ausschließlich Einfachbindungen enthalten, als auch teilweise ungesättigte Ringe, d.h. solche die auch Mehrfachbindungen enthalten können. Heterocyclische Ringe enthalten ein oder mehrere Heteroatome, vorzugsweise ausgewählt aus Si, O, N, S und Se.

Die (nicht-aromatischen) alicyclischen und heterocyclischen Gruppen können einkernig sein, d.h. nur einen Ring enthalten (wie z.B. Cyclohexan), oder mehrkernig sein, d.h. mehrere Ringe enthalten (wie z.B. Decahydronaphthalin oder Bicyclooctan). Besonders bevorzugt sind gesättigte Gruppen. Ferner bevorzugt sind ein-, zwei- oder dreikernige Gruppen mit 3 bis 25 C-Atomen, welche optional anellierte Ringe enthalten und optional substituiert sind. Ferner bevorzugt sind 5-, 6-, 7-oder 8-gliedrige carbocyclische Gruppen worin auch ein oder mehrere C-Atome durch Si ersetzt sein können und/oder eine oder mehrere CH-Gruppen durch N ersetzt sein können und/oder eine oder mehrere nichtbenachbarte CH₂-Gruppen durch -O- und/oder -S- ersetzt sein können.

Bevorzugte alicyclische und heterocyclische Gruppen sind beispielsweise 5-gliedrige Gruppen wie Cyclopentan, Tetrahydrofuran, Tetrahydrothiofuran, Pyrrolidin, 6-gliedrige Gruppen wie Cyclohexan, Silinan, Cyclohexen, Tetrahydropyran, Tetrahydrothiopyran, 1,3-Dioxan, 1,3-Dithian, Piperidin, 7- gliedrige Gruppen wie Cycloheptan, und anellierte Gruppen wie Tetrahydronaphthalin, Decahydronaphthalin, Indan, Bicyclo[1.1.1]pentan-1,3-diyl, Bicyclo[2.2.2]octan-1,4-diyl, Spiro[3.3]heptan-2,6-diyl, Octahydro-4,7-methano-indan-2,5-diyl.

Bevorzugte Substituenten sind beispielsweise löslichkeitsfördernde Gruppen wie Alkyl oder Alkoxy, elektronenziehende Gruppen wie Fluor, Nitro oder Nitril, oder Substituenten zur Erhöhung der Glastemperatur (Tg) im Polymer, insbesondere voluminöse Gruppen wie z.B. t-Butyl oder gegebenenfalls substituierte Arylgruppen.

Bevorzugte Substituenten, vor- und nachstehend auch als "L" bezeichnet, sind beispielsweise F, Cl, Br, I, OH, -CN, -NO₂, -NCO, -NCS, -OCN, - SCN, -C(=O)N(R^{x})₂, -C(=O)Y¹, -C(=O)R^{x}, - C(=O)OR^{x}, -N(R^{x})₂, worin R^{x} die oben angegebene Bedeutung hat und Y¹ Halogen bedeutet, optional substituiertes Silyl, optional substituiertes Aryl oder Heteroaryl mit 4 bis 40, vorzugsweise 4 bis 20 C Atomen, und geradkettiges oder verzweigtes Alkyl, Alkenyl, Alkinyl, Alkoxy, Alkylcarbonyl, Alkoxycarbonyl, Alkylcarbonyloxy oder Alkoxycarbonyloxy mit 1 bis 25 C-Atomen, worin ein oder mehrere H-Atome gegebenenfalls durch F oder Cl ersetzt sein können.

"Substituiertes Silyl oder Aryl" bedeutet vorzugsweise durch Halogen, - CN, R⁰, -OR⁰, -CO-R⁰, -CO-O-R⁰, -O-CO-R⁰ oder -O-CO-O-R⁰ substituiert, worin R⁰ die oben angegebene Bedeutung hat.

Besonders bevorzugte Substituenten L sind beispielsweise F, Cl, CN, NO₂, CH₃, C₂H₅, OCH₃, OC₂H₅, COCH₃, COC₂H₅, COOCH₃, COOC₂H₅, CF₃, OCF₃, OCHF₂, OC₂F₅, ferner Phenyl. 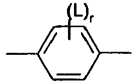 ist vorzugsweise 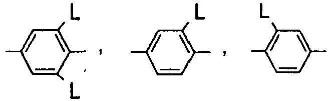 oder 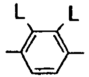 worin L eine der oben angegebenen Bedeutungen hat.

Die polymerisierbare Gruppe P ist eine Gruppe, die für eine Polymerisationsreaktion, wie beispielsweise die radikalische oder ionische Kettenpolymerisation, Polyaddition oder Polykondensation, oder für eine polymeranaloge Umsetzung, beispielsweise die Addition oder Kondensation an eine Polymerhauptkette, geeignet ist. Besonders bevorzugt sind Gruppen für die Kettenpolymerisation, insbesondere solche enthaltend eine C=C-Doppelbindung oder -C≡C-Dreifachbindung, sowie zur Polymerisation unter Ringöffnung geeignete Gruppen wie beispielsweise Oxetan- oder Epoxygruppen

Bevorzugte Gruppen P sind ausgewählt aus der Gruppe bestehend aus CH₂=CW¹-CO-O-, CH₂=CW¹-CO-, 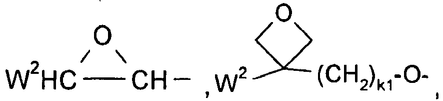 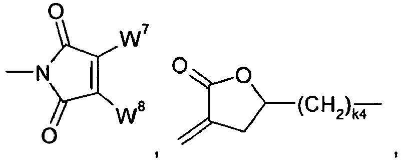 CH₂=CW²-(O)ₖ₃-, CW¹=CH-CO-(O)ₖ₃-, CW'=CH-CO-NH-, CH₂=CW¹-CO-NH-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH-, HOOC-, OCN-, und W⁴W⁵W⁶Si-, worin W¹ H, F, Cl, CN, CF₃, Phenyl oder Alkyl mit 1 bis 5 C-Atomen, insbesondere H, F, Cl oder CH₃ bedeutet, W² und W³ jeweils unabhängig voneinander H oder Alkyl mit 1 bis 5 C-Atomen, insbesondere H, Methyl, Ethyl oder n-Propyl bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet, welches optional mit einem oder mehreren, von P-Sp- verschiedenen Resten L wie oben definiert substiuiert ist, k₁, k₂ und k₃ jeweils unabhängig voneinander 0 oder 1 bedeuten, k₃ vorzugsweise 1 bedeutet, und k₄ eine ganze Zahl von 1 bis 10 bedeutet.

Besonders bevorzugte Gruppen P sind ausgewählt aus der Gruppe bestehend aus CH₂=CW¹-CO-O-, CH₂=CW¹-CO-, 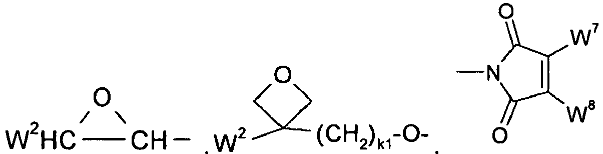 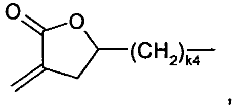 CH₂=CW²-O-, CW¹=CH-CO-(O)ₖ₃-, CW¹=CH-CO-NH-, CH₂=CW¹-CO-NH-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, CH₂=CW¹-CO-NH-, CH₂=CH-(COO)ₖ₁-Phe-(O)ₖ₂-, CH₂=CH-(CO)ₖ₁-Phe-(O)ₖ₂-, Phe-CH=CH- und W⁴W⁵W⁶Si-, worin W¹ H, F, Cl, CN, CF₃, Phenyl oder Alkyl mit 1 bis 5 C-Atomen, insbesondere H, F, Cl oder CH₃ bedeutet, W² und W³ jeweils unabhängig voneinander H oder Alkyl mit 1, 2, 3, 4 oder 5 C-Atomen, insbesondere H, Methyl, Ethyl oder n-Propyl bedeuten, W⁴, W⁵ und W⁶ jeweils unabhängig voneinander Cl, Oxaalkyl oder Oxacarbonylalkyl mit 1 bis 5 C-Atomen bedeuten, W⁷ und W⁸ jeweils unabhängig voneinander H, Cl oder Alkyl mit 1 bis 5 C-Atomen bedeuten, Phe 1,4-Phenylen bedeutet, k₁, k₂ und k₃ jeweils unabhängig voneinander 0 oder 1 bedeuten, k₃ vorzugsweise 1 bedeutet, und k₄ eine ganze Zahl von 1 bis 10 bedeutet.

Ganz besonders bevorzugte Gruppen P sind sind ausgewählt aus der Gruppe bestehend aus CH₂=CW¹-CO-O-, insbesondere CH₂=CH-CO-O-, CH₂=C(CH₃)-CO-O- und CH₂=CF-CO-O-, ferner CH₂=CH-O-, (CH₂=CH)₂CH-O-CO-, (CH₂=CH)₂CH-O-, 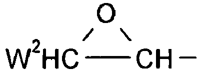 und

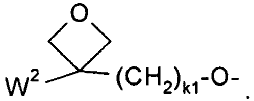

Weitere ganz besonders bevorzugte Gruppen P sind ausgewählt aus der Gruppe bestehend aus Vinyloxy-, Acrylat-, Methacrylat-, Fluoracrylat-, Chloracrylat-, Oxetan-, 3-Ethyloxetan- und Epoxygruppen, und bedeuten besonders bevorzugt eine Acrylat- oder Methacrylatgruppe.

Bevorzugte Abstandsgruppen Sp sind ausgewählt aus der Formel Sp"-X", so dass der Rest " P-Sp- " der Formel " P-Sp"-X"- " entspricht, wobei
- Sp": Alkylen mit 1 bis 20, vorzugsweise 1 bis 12 C-Atomen bedeutet, welches optional durch F, Cl, Br, I oder CN ein- oder mehrfach substituiert ist, und worin auch eine oder mehrere nicht benachbarte CH₂-Gruppen jeweils unabhängig voneinander so durch -O-, -S-, -NH-, -N(R⁰)-, -Si(R⁰⁰R⁰⁰⁰)-, -CO-, -CO-O-, -O-CO-, -O-CO-O-, -S-CO-, -CO-S-, -N(R⁰⁰)-CO-O-, -O-CO-N(R⁰⁰)-, -N(R⁰⁰)-CO-N(R⁰⁰)-, -CH=CH- oder -C≡C- ersetzt sein können, dass O- und/oder S-Atome nicht direkt miteinander verknüpft sind,
- X": -O-, -S-, -CO-, -CO-O-, -O-CO-, -O-CO-O-, -CO-N(R⁰⁰)-, -N(R⁰⁰)-CO-, -N(R⁰⁰)-CO-N(R⁰⁰)-, -OCH₂-, -CH₂O-, -SCH₂-, -CH₂S-, -CF₂O-, -OCF₂-, -CF₂S-, -SCF₂-, -CF₂CH₂-, -CH₂CF₂-, -CF₂CF₂-, -CH=N-, -N=CH-, -N=N-, -CH=CR⁰-, -CY²=CY³-, -C≡C-, -CH=CH-CO-O-, -O-CO-CH=CH- oder eine Einfachbindung bedeutet,
- R⁰⁰ und R⁰⁰⁰: jeweils unabhängig voneinander H oder Alkyl mit 1 bis 12 C-Atomen bedeuten, und
- Y² und Y³: jeweils unabhängig voneinander H, F, Cl oder CN bedeuten.

X' ist vorzugsweise -O-, -S -CO-, -COO-, -OCO-, -O-COO-, -CO-NR0⁻, - NR⁰-CO-, -NR⁰-CO-NR⁰- oder eine Einfachbindung.

Typische Abstandsgruppen Sp" sind beispielsweise -(CH₂)ₚ₁-, -(CH₂CH₂O)_{q1} - CH₂CH₂-, -CH₂CH₂-S-CH₂CH₂-, -CH₂CH₂-NH-CH₂CH₂- oder -(SiR⁰⁰R⁰⁰⁰-O)ₚ₁-, worin p1 eine ganze Zahl von 1 bis 12 ist, q1 eine ganze Zahl von 1 bis 3 ist, und R⁰⁰ und R⁰⁰⁰ die oben angegebenen Bedeutungen besitzen.

Besonders bevorzugte Gruppen -Sp"-X"- sind -(CH₂)ₚ₁-, -(CH₂)ₚ₁-O-, -(CH₂)ₚ₁-O-CO-, -(CH₂)ₚ₁-O-CO-O-, worin p1 und q1 die oben angebene Bedeutung haben.

Besonders bevorzugte Gruppen Sp" sind beispielsweise jeweils geradkettiges Ethylen, Propylen, Butylen, Pentylen, Hexylen, Heptylen, Octylen, Nonylen, Decylen, Undecylen, Dodecylen, Octadecylen, Ethylenoxyethylen, Methylenoxybutylen, Ethylenthioethylen, Ethylen-N-methyl-iminoethylen, 1-Methylalkylen, Ethenylen, Propenylen und Butenylen.

In einer weiteren bevorzugten Ausführungsform der Erfindung bedeutet P in Formel I einen Rest mit zwei oder mehreren polymerisierbaren Gruppen (multifunktionelle polymerisierbare Reste). Geeignete Reste dieses Typs, sowie diese enthaltende polymerisierbare Verbindungen und ihre Herstellung sind beispielsweise in US 7,060,200 B1 oder US 2006/0172090 A1 beschrieben. Besonders bevorzugt sind multifunktionelle polymerisierbare Reste ausgewählt aus folgenden Formeln

-X-alkyl-CHP¹-CH₂-CH₂P² I*a

-X-alkyl-C(CH₂P¹)(CH₂P²)-CH₂P³ I*b

-X-alkyl-CHP¹CHP²-CH₂P³ I*c

-X-alkyl-C(CH₂P¹)(CH₂P²)-CₐₐH₂ₐₐ₊₁ I*d

-X-alkyl-CHP¹-CH₂P² I*e

-X-alkyl-CHP¹P² I*f

-X-alkyl-CP¹P²-CₐₐH₂ₐₐ₊₁ I*g

-X-alkyl-C(CH₂P¹)(CH₂P²)-CH₂OCH₂-C(CH₂P³)(CH₂P⁴)CH₂P⁵ I*h

-X-alkyl-CH((CH₂)ₐₐP¹)((CH₂)_{bb}P²) I*i

-X-alkyl-CHP¹CHP²-CₐₐH₂ₐₐ₊₁ I*k

-X'-alky(-C(CH₃)(CH₂P¹)(CH₂P²) I*m

worin
- alkyl: eine Einfachbindung oder geradkettiges oder verzweigtes Alkylen mit 1 bis 12 C-Atomen bedeutet, worin eine oder mehrere nicht benachbarte CH₂-Gruppen jeweils unabhängig voneinander durch -C(R⁰⁰)=C(R⁰⁰⁰)-, -C≡C-, -N(R⁰⁰)-, -O-, -S-, - CO-, -CO-O-, -O-CO-, -O-CO-O- so ersetzt sein können, dass O-und/oder S-Atome nicht direkt miteinander verknüpft sind, und worin auch ein oder mehrere H-Atome durch F, Cl oder CN ersetzt sein können, wobei R⁰⁰ und R⁰⁰⁰ die oben angegebene Bedeutung haben,
aa und bb jeweils unabhängig voneinander 0, 1, 2, 3, 4, 5 oder 6 bedeuten,
- X: eine der für X' angegebenen Bedeutungen besitzt, und
- P¹⁻⁵: jeweils unabhängig voneinander eine der für P angegebenen Bedeutungen besitzen.

Die polymerisierbaren Verbindungen der Formel I sind dadurch gekennzeichnet, dass mindestens einer der in den Verbindungen enthaltenen Reste Z¹ und Z² eine C≡C-Dreifachbindung bedeutet. Es hat sich überraschend gezeigt, dass bei Verwendung dieser Verbindungen in erfindungsgemäßen FK-Medien und PSA-Anzeigen der gewünschte Pretilt besonders schnell erreicht und Herstellungszeit der Anzeige deutlich verkürzt werden kann. Die erfindungsgemäßen polymerisierbaren Verbindungen zeigen in den PSA-Anzeigen eine deutlich höhere Polymerisationsgeschwindigkeit, dadurch verbleiben weniger unreagierte Restmengen in der FK-Zelle, wodurch deren elektrooptische Eigenschaften verbessert werden und das kontrollierte Abreagieren dieser Restmengen einfacher wird.

Besonders bevorzugt sind Verbindungen der Formel I, worin
- A¹, A², A³: jeweils unabhängig voneinander 1,4-Phenylen, Naphthalin-2,6-diyl, Phenanthren-2,7-diyl oder Anthracen-2,7-diyl, wobei alle diese Gruppen unsubstituiert oder durch L ein- oder mehrfach substituiert sein können,
- L: P-, P-Sp-, OH, CH₂OH, F, Cl, Br, I, -CN, -NO₂, -NCO, -NCS, -OCN, -SCN, -C(=O)N(R^{x})₂, -C(=O)Y¹, -C(=O)R^{x}, -N(R^{x})₂, optional substituiertes Silyl, optional substituiertes Aryl mit 6 bis 20 C Atomen, geradkettiges oder verzweigtes Alkyl oder Alkoxy 1 bis 25, vorzugsweise 1 bis 12, C-Atomen, oder geradkettiges oder verzweigtes Alkenyl, Alkinyl, Alkylcarbonyl, Alkoxycarbonyl, Alkylcarbonyloxy oder Alkoxycarbonyloxy mit 2 bis 25, vorzugsweise 2 bis 12, C-Atomen, worin in allen diesen Gruppen auch ein oder mehrere H-Atome durch F, Cl, P oder P-Sp- ersetzt sein können,
- Y¹: Halogen, und
- R^{x}: P-, P-Sp-, H, Halogen, geradkettiges, verzweigtes oder cyclisches Alkyl mit 1 bis 25, vorzugsweise 1 bis 12 C-Atomen, worin auch eine oder mehrere nicht benachbarte CH₂-Gruppen durch -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, P- oder P-Sp- ersetzt sein können,
bedeuten.

Weitere bevorzugte Verbindungen der Formel I sowie deren vor- und nachstehend angegebenen Unterformeln sind ausgewählt aus einer oder mehreren der nachfolgenden bevorzugten Ausführungsformen, welche beliebig miteineander kombiniert werden können, worin
- P bei jedem Auftreten gleich oder verschieden Acrylat (CH₂=CH-C(O)-O-) oder Methacrylat (CH₂=C(CH₃)-C(O)-O-) bedeutet,
- s1 1 und s2 0 bedeutet oder s1 0 und s2 1 bedeutet,
- s1 und s2 0 bedeuten,
- Sp -(CH₂)ₚ₁-, -(CH₂)ₚ₁-O-, -(CH₂)ₚ₁-O-CO- oder -(CH₂)ₚ₁-O-CO-O-, vorzugsweise -(CH₂)ₚ₁- oder -(CH₂)ₚ₁-O- bedeutet, worin p1 eine ganze Zahl von 1 bis 12, vorzugsweise von 1 bis 5, besonders bevorzugt von 1 bis 3 bedeutet,
- Sp und/oder Sp" ein Alkylenrest mit 1 bis 5, vorzugsweise 1 bis 3 C-Atomen bedeuten,
- A¹, A² und A³ jeweils unabhängig voneinander und bei jedem Auftreten gleich oder verschieden Phenylen-1,4-diyl oder Naphthalin-2,6-diyl, welches auch ein- oder mehrfach durch L wie vor- und nachstehend beschrieben substituiert sein kann,
- L eine polymerisierbare Gruppe weder bedeutet noch enthält,
- L eine unpolymerisierbare Gruppe bedeutet, vorzugsweise ausgewählt aus F, Cl, -CN und geradkettigem oder verzweigtem Alkyl mit 1 bis 25, besonders bevorzugt 1 bis 10 C-Atomen, worin auch eine oder mehrere nicht benachbarte CH₂-Gruppen jeweils unabhängig voneinander durch -C(R⁰⁰)=C(R⁰⁰⁰)-, -C≡C-, -N(R⁰⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, Br, I oder CN ersetzt sein können,
- L F, Cl, Br, I, -CN, -NO₂, -NCO, -NCS, -OCN oder -SCN, vorzugsweise F, bedeutet,
- L geradkettiges oder verzweigtes Alkyl oder Alkoxy mit 1 bis 12 C-Atomen, oder geradkettiges oder verzweigtes Alkenyl, Alkinyl, Alkylcarbonyl, Alkoxycarbonyl, Alkylcarbonyloxy oder Alkoxycarbonyloxy mit 2 bis 12 C-Atomen, worin in allen diesen Gruppen auch ein oder mehrere H-Atome durch F, Cl oder P-Spersetzt sein können, bedeutet,
- L P- oder P-Sp bedeutet,
- mindestens einer der Reste Z¹ und Z² -C=C- bedeutet und die anderen Reste Z¹ und Z² jeweils unabhängig voneinander -C≡C-, - CO-O-, -O-CO- oder eine Einfachbindung bedeuten,
- mindestens einer der Reste Z¹ und Z² -C≡C- bedeutet und die anderen Reste Z¹ und Z² jeweils unabhängig voneinander -C≡C-oder eine Einfachbindung bedeuten,
- alle in den Verbindungen der Formel I auftretenden Reste Z¹ und Z² -C≡C- bedeuten,
- m 0 oder 1 bedeutet,
- m 0 bedeutet,
- m 1 bedeutet und Z² -CO-O- oder -O-CO- bedeutet,
- m 1 bedeutet und Z² -C≡C- oder eine Einfachbindung bedeutet.

Besonders bevorzugte Verbindungen der Formel I sind ausgewählt aus der Gruppe bestehend aus den folgenden Unterformeln 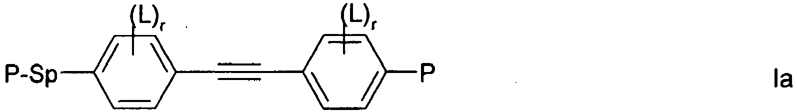 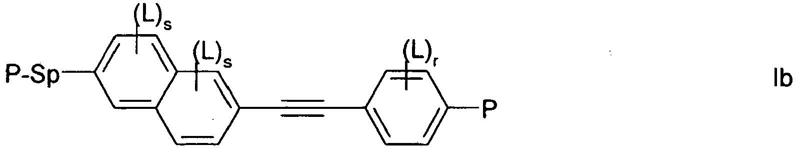 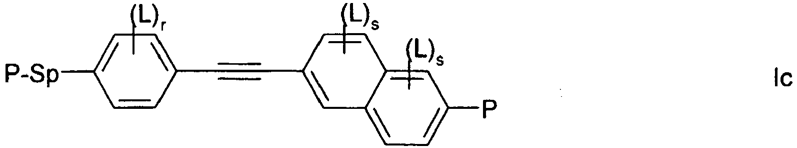 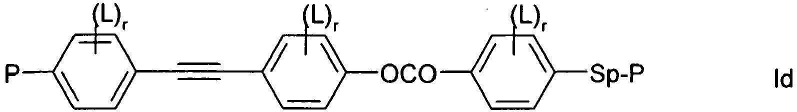 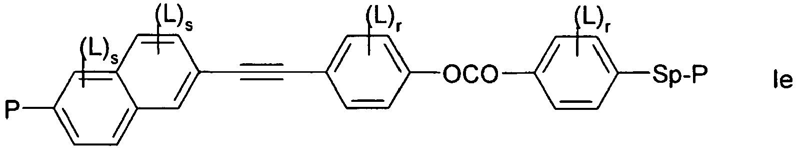 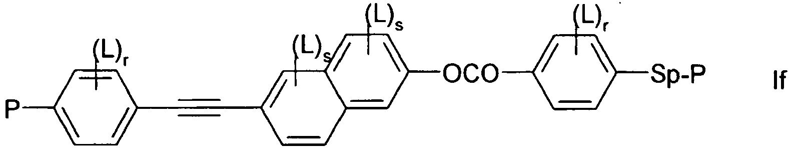 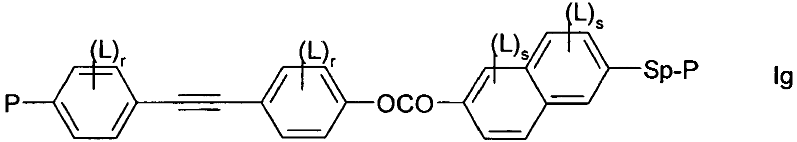 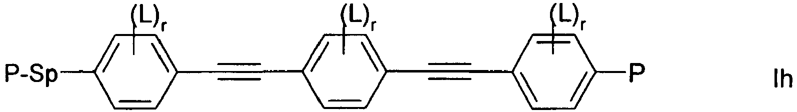 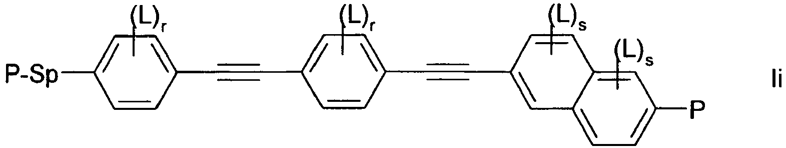 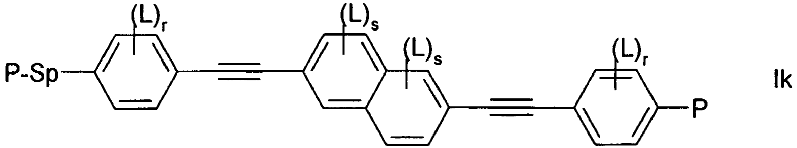 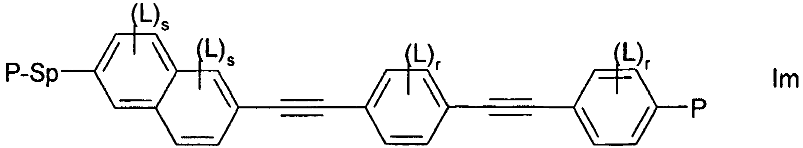 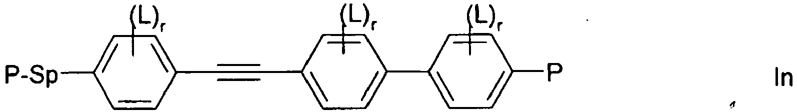 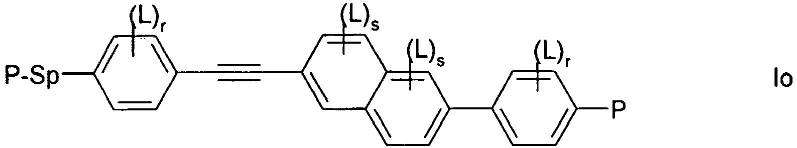 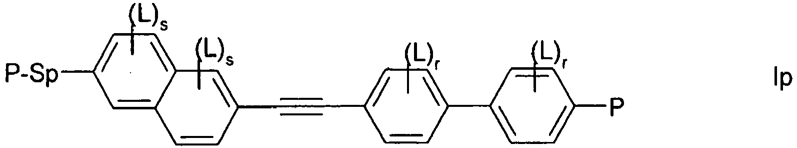 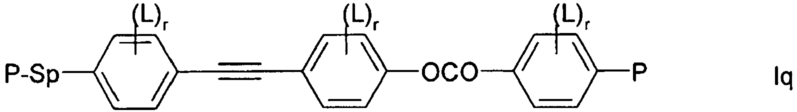 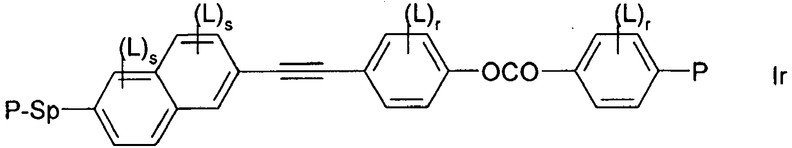 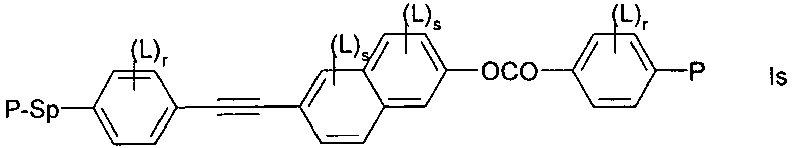 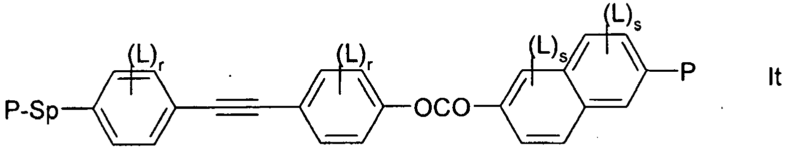 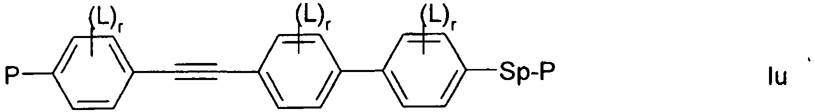 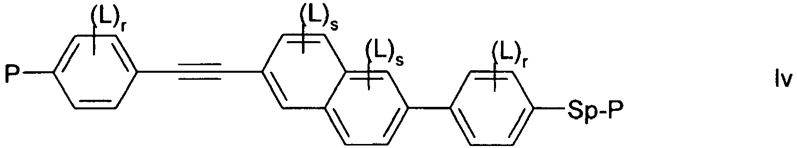 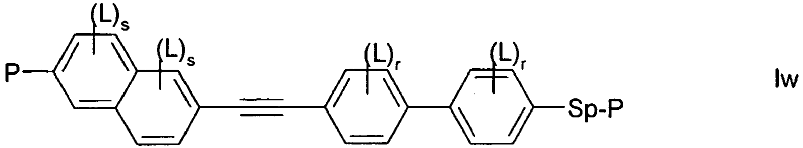 worin P, Sp und L eine der vor-und nachstehend angegebenen Bedeutungen besitzen, r 0, 1, 2, 3 oder 4 und s 0, 1, 2 oder 3 bedeuten.

P bedeutet in den Verbindungen der Formel I sowie den Unterformeln la-Ip vorzugsweise Acrylat oder Methacrylat, ferner Fluoracrylat.

Sp bedeutet in den Verbindungen der Formeln I sowie den Unterformeln Ia-Ip vorzugsweise -(CH₂)ₚ₁-, -(CH₂)ₚ₁-O-, -(CH₂)ₚ₁-O-CO- oder -(CH₂)ₚ₁-O-CO-O-, oder deren Spiegelbilder, worin p1 eine ganze Zahl von 1 bis 12, vorzugsweise von 1 bis 6, besonders bevorzugt 1, 2, 3 oder 4 bedeutet, und wobei die Verknüpfung mit dem benachbarten Benzolring über das O-Atom erfolgt.

Ein weiterer Gegenstand der Erfindung sind neue Verbindungen der Formel I.

Ein weiterer Gegenstand der Erfindung sind neue Zwischenprodukte zur Herstellung von Verbindungen der Formel I, ausgewählt aus Formel II

G-O-(Sp)ₛ₁-A¹-Z¹-A²(-Z²-A³)ₘ-(Sp)ₛ₂-O-G' II

worin Sp, s1, s2, A¹, A², A³, Z¹, Z² und m die in Formel I oder vor- und nachstehend angegebene Bedeutung besitzen, und G und G' jeweils unabhängig voneinander ein H-Atom oder eine Schutzgruppe bedeuten.

Geeignete Schutzgruppen G sind dem Fachmann bekannt. Bevorzugte Schutzguppen sind Alkyl-, Acyl- und Alkylsilyl- oder Arylsilylgruppen, 2-Tetrahydropyranyl oder Methoxymethyl.

Besonders bevorzugte Zwischenprodukte der Formel II sind ausgewählt aus der Gruppe bestehend aus den oben genannten Unterformeln Ia-Ip, worin "P-" G-O- und "-P" -O-G' bedeutet, wobei G und G' vorzugsweise H bedeuten.

Besonders geeignete und bevorzugte Verfahren zur Herstellung von Verbindungen und Zwischenprodukten der Formel I und II sowie deren Unterformeln sind in folgenden Schemata beispielhaft dargestellt und enthalten vorzugsweise einen oder mehrere der nachfolgend beschriebenen Schritte.

Die Verbindungen und Zwischenprodukte der Formel I und II sowie deren Unterformeln können in Analogie zu dem Fachmann bekannten und in Standardwerken der organischen Chemie beschriebenen Verfahren, wie beispielsweise in Houben-Weyl, Methoden der organischen Chemie, Thieme-Verlag, Stuttgart, hergestellt werden.

Beispielsweise erfolgt die Synthese von Verbindungen der Formel I durch Veresterung oder Veretherung der Zwischenprodukte der Formel II mit entsprechenden Säuren, Säurederivaten, oder halogenierten Verbindungen enthaltend eine Gruppe P. Wie in Schema 1 beispielhaft dargestellt, können Verbindungen der Formel I, worin P beispielsweise eine Acrylat- oder Methacrylatgruppe ist, durch Veresterung der entsprechenden Alkohole der Formel II, worin G=G'=H mit Säurederivaten wie zum Beispiel (Meth)acrylsäurechlorid oder (Meth)acrylsäureanhydrid in Gegenwart einer Base und ggf. 4-(N,N-Dimethylamino)pyridin (DMAP) erhalten werden. Weiterhin können die Alkohole auch mit (Meth)acrylsäure in Gegenwart eines wasserentziehenden Mittels verestert werden, beispielsweise nach Steglich mit Dicyclohexylcarbodiimid (DCC).

Ein weiterer Gegenstand der Erfindung ist somit ein Verfahren zur Herstellung einer Verbindung nach Formel I, indem man eine Verbindung der Formel II mit entsprechenden Säuren, Säurederivaten, oder halogenierten Verbindungen enthaltend eine Gruppe P in Gegenwart eines wasserentziehenden Reagens verestert oder verethert.

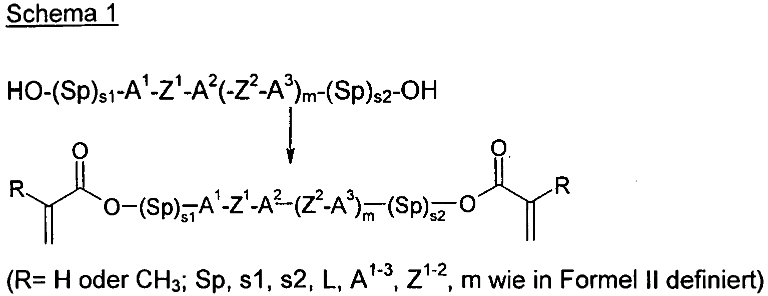

Die Synthese des Tolangrundkörpers in den Verbindungen der Formel I und II erfolgt nach dem Fachmann bekannten und in der Literatur beschriebenen Verfahren. Einige besonders geeignete Varianten sind nachfolgend beispielhaft dargestellt (Schema 2): So lassen sich Arylhalogenide und Triflate (X = Hal, OSO₂CF₃) in einer Vielzahl von übergangsmetallkatalysierten Kreuzkupplungsreaktionen zu Tolanen und analogen Diarylacetylenen umsetzen, z.B. nach Sonogashira (Y = H, s. z.B. R. Chinchilla, C. Najera, Chem. Rev. 2007, 107, 874-922; J. Tsuji, Palladium Reagent and Catalysts; Wiley: New York, 1995), nach Stille (Y = Sn(Alkyl)₃, s. z.B. V. Farina, V. Krishnamurthy, W.J. Scott, The Stille reaction. Organic Reactions (Hoboken, NJ, United States) (1997), Bd. 50.), in Reaktionen vom Kumada-Typ (Y = MgHal, T. Kamikawa, T. Hayashi, J. Org. Chem. 1998, 63(24), 8922-8925) oder nach Negishi (Y = ZnHal, s. z.B. R. Rossi et al., Tetrahedron 59 (2003) 2067-2081).

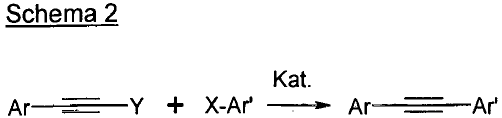

Die Arylacetylene sind ihrerseits durch Sonogashira-Kupplung mit Silylacetylenen zugänglich. (Schema 3). Die in einem ersten Schritt erhaltenen Arylsilylacetylene werden dabei z. B. mit Tetrabutylammoniumfluorid oder Olahs Reagenz zu den Zielverbindungen entschützt.

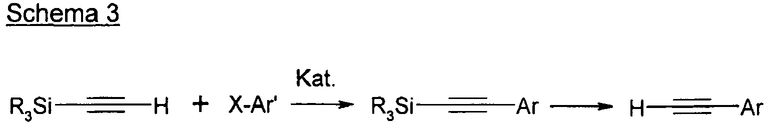

Ein weiterer Zugang zu Arylacetylenen ist durch die Umsetzung von Arylcarbaldehyden nach Corey und Fuchs gegeben (Tetrahedron Lett. 1972, 36, 3769 - 3772; Schema 4).

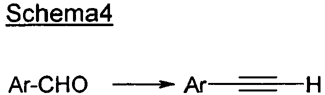

Zur formalen Eliminierung von Wasser aus Ketoenolen zu terminalen Alkinen (Schema 5) s. z. B. E. Negishi et al., Organic Syntheses, 1986, 64, 44-49; I.M.Lyapkalo, Synlett 2009, 4, 558-561).

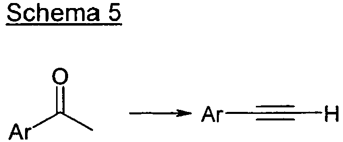

Die vor- und nachstehend beschriebenen Verfahren zur Herstellung von Verbindungen der Formel I und II, insbesondere zur Herstellung von Verbindungen der Formel I aus Verbindungen der Formel II, sind ein weiterer Gegenstand der Erfindung.

Zur Herstellung von PSA-Anzeigen werden die polymerisierbaren Verbindungen im FK-Medium zwischen den Substraten der FK-Anzeige unter Anlegen einer Spannung durch in-situ-Polymerisation polymerisiert oder vernetzt (falls eine Verbindung zwei oder mehr polymerisierbare Gruppen enthält). Die Polymerisation kann in einem Schritt durchgeführt werden. Es ist auch möglich, zunächst in einem ersten Schritt die Polymerisation unter Anlegen einer Spannung durchzuführen, um einen pretilt-Winkel zu erzeugen, und anschließend in einem zweiten Polymerisationsschritt ohne anliegende Spannung die im ersten Schritt nicht abreagierten Verbindungen zu polymerisieren bzw. zu vernetzen ("end curing").

Geeignete und bevorzugte Polymerisationsmethoden sind beispielsweise die thermische oder Photopolymerisation, vorzugsweise Photopolymerisation, insbesondere UV-Photopolymerisation. Dabei können gegebenfalls auch ein oder mehrere Initiatoren zugesetzt werden. Geeignete Bedingungen für die Polymerisation, sowie geeignete Arten und Mengen der Initiatoren, sind dem Fachmann bekannt und in der Literatur beschrieben. Für die radikalische Polymerisation eignen sich zum Beispiel die kommerziell erhältlichen Photoinitiatoren Irgacure651®, Irgacure184®, Irgacure907®, Irgacure369®, oder Darocure1173® (Ciba AG). Falls ein Initiator eingesetzt wird, beträgt dessen Anteil vorzugsweise 0,001 bis 5 Gew.-%, besonders bevorzugt 0,001 bis 1 Gew.-%.

Die erfindungsgemäßen polymerisierbaren Verbindungen eignen sich auch für die Polymerisation ohne Initiator, was erhebliche Vorteile mit sich bringt, wie beispielsweise geringere Materialkosten und insbesondere eine geringere Verunreinigung des FK-Mediums durch mögliche Restmengen des Initiators oder dessen Abbauprodukte. Die Polymerisation kann somit auch ohne Zusatz eines Initiators erfolgen. Somit enthält das FK-Medium in einer bevorzugten Ausführungsform keinen Polymerisationsinitiator.

Die polymerisierbare Komponente A) oder das FK-Medium können auch einen oder mehrere Stabilisatoren enthalten, um eine unerwünschte spontane Polymerisation der RMs, beispielsweise während der Lagerung oder des Transports, zu verhindern. Geeignete Arten und Mengen der Stabilisatoren sind dem Fachmann bekannt und in der Literatur beschrieben. Besonders geeignet sind zum Beispiel die kommerziell erhältlichen Stabilisatoren der Serie Irganox® (Ciba AG), wie beispielsweise Irganox® 1076. Falls Stabilisatoren eingesetzt werden, beträgt deren Anteil, bezogen auf die Gesamtmenge der RMs beziehungsweise der polymerisierbaren Komponente A), vorzugsweise 10 - 10,000 ppm, besonders bevorzugt 50 - 500 ppm.

Die erfindungsgemäßen FK-Medien zur Verwendung in PSA-Anzeigen enthalten vorzugsweise < 5 Gew.-%, besonders bevorzugt < 1 Gew.-%, ganz besonders bevorzugt < 0.5 Gew.-% an polymerisierbaren Verbindungen, insbesondere polymerisierbaren Verbindungen der oben genannten Formeln I und deren Unterformeln.

Besonders bevorzugt sind FK-Medien enthaltend eine, zwei oder drei erfindungsgemäße polymerisierbare Verbindungen.

Ferner bevorzugt sind FK-Medien, worin die polymerisierbare Komponente (Komponente A) ausschließlich erfindungsgemäße polymerisierbare Verbindungen enthält.

Ferner bevorzugt sind FK-Medien, worin Komponente B) eine FK-Verbindung oder eine FK-Mischung ist, die eine nematische Flüssigkristallphase aufweist.

Ferner bevorzugt sind achirale erfindungsgemäße polymerisierbare Verbindungen, sowie FK-Medien worin die Verbindungen der Komponente A) und/oder B) ausschließlich aus der Gruppe bestehend aus achiralen Verbindungen ausgewählt sind.

Ferner bevorzugt sind FK-Medien, worin die polymerisierbare Komponente bzw. Komponente A) eine oder mehrere erfindungsgemäße polymerisierbare Verbindungen mit einer polymerisierbaren Gruppe (monoreaktiv) und eine oder mehrere erfindungsgemäße polymerisierbare Verbindungen mit zwei oder mehr, vorzugsweise zwei polymerisierbaren Gruppen (di- oder multireaktiv) enthält.

Ferner bevorzugt sind PSA-Anzeigen und FK-Medien, worin die polymerisierbare Komponente bzw. Komponente A) ausschließlich erfindungsgemäße polymerisierbare Verbindungen mit zwei polymerisierbaren Gruppen (direaktiv) enthält.

Der Anteil der polymerisierbaren Komponente bzw. Komponente A) in den erfindungsgemäßen FK-Medien ist vorzugsweise < 5%, besonders bevorzugt < 1 %, ganz besonders bevorzugt < 0.5 %.

Der Anteil der flüssigkristallinen Komponente bzw. Komponente B) in den erfindungsgemäßen FK-Medien ist vorzugsweise > 95%, besonders bevorzugt > 99%.

Die erfindungsgemäßen polymerisierbaren Verbindungen können einzeln polymerisiert werden, es können aber auch Mischungen polymerisiert werden, welche zwei oder mehr erfindungsgemäße polymerisierbare Verbindungen enthalten, oder Mischungen enthaltend eine oder mehrere erfindungsgemäße polymerisierbare Verbindungen und eine oder mehrere weitere polymerisierbare Verbindungen (Comonomere), welche vorzugsweise mesogen oder flüssigkristallin sind. Bei Polymerisation solcher Mischungen entstehen Copolymere. Die vor- und nachstehend genannten polymerisierbaren Mischungen sind ein weiterer Gegenstand der Erfindung. Die polymerisierbaren Verbindungen und Comonomere sind mesogen oder nicht-mesogen, vorzugsweise mesogen oder flüssigkristallin.

Geeignete und bevorzugte mesogene Comonomere, besonders für die Verwendung in PSA-Anzeigen, sind beispielsweise ausgewählt aus den folgenden Formeln: 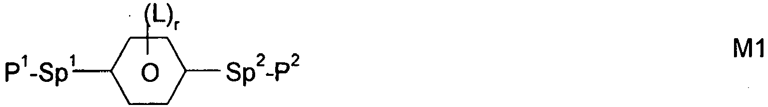 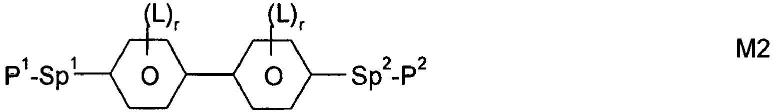 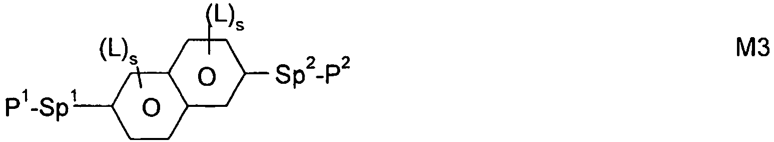 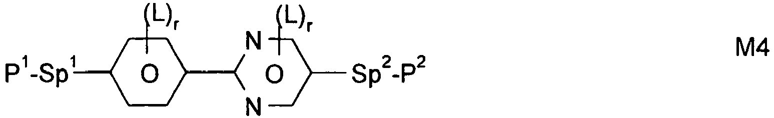 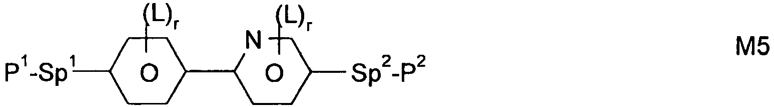 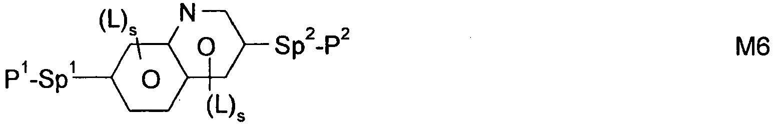 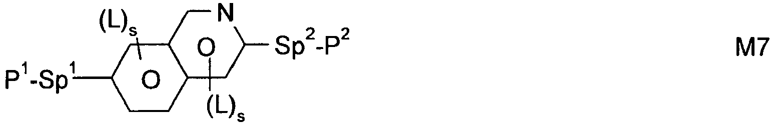 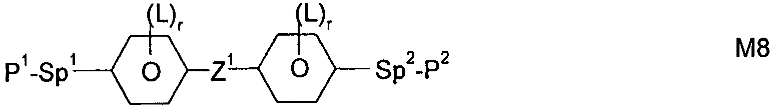 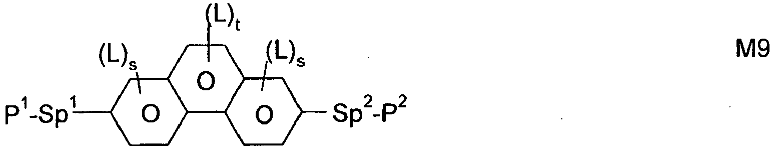 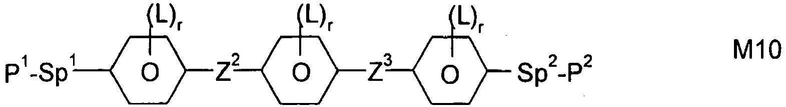 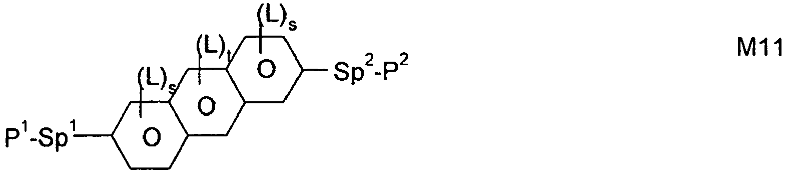 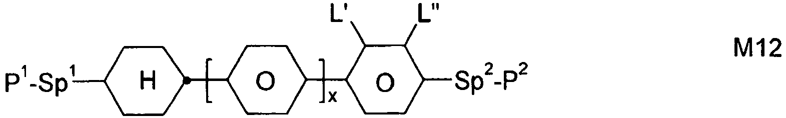 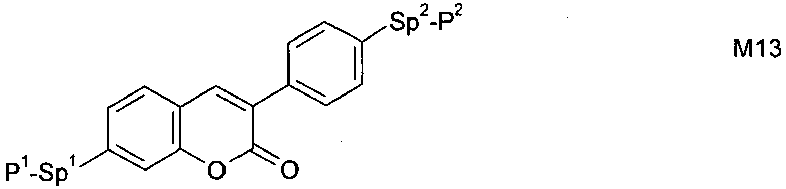 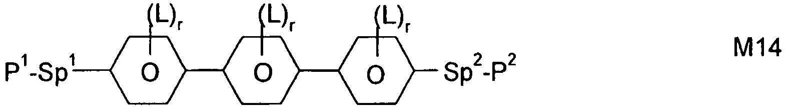 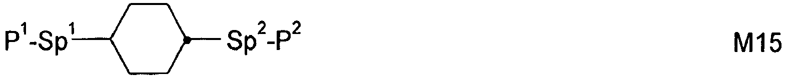 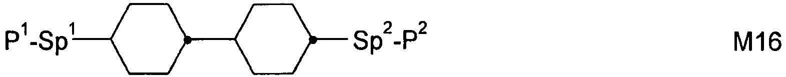 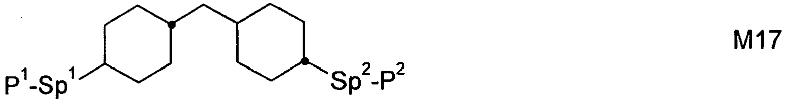 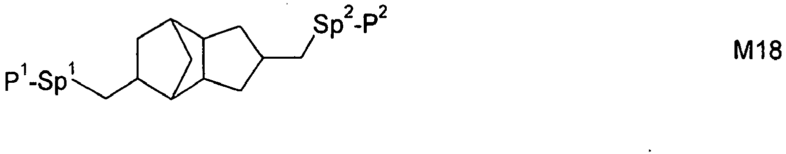 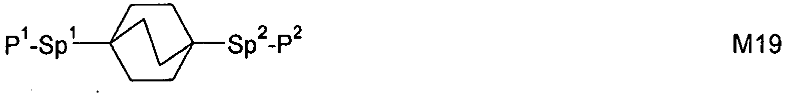 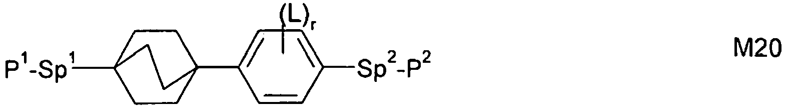 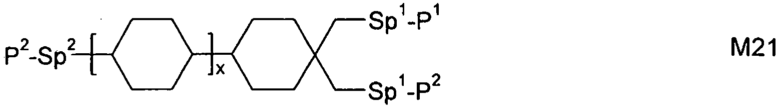 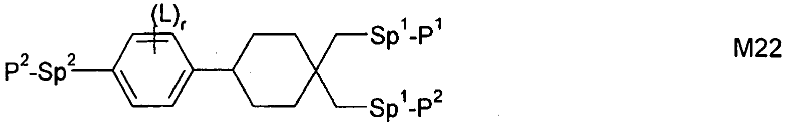 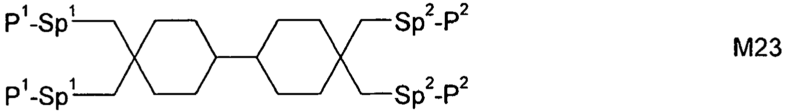 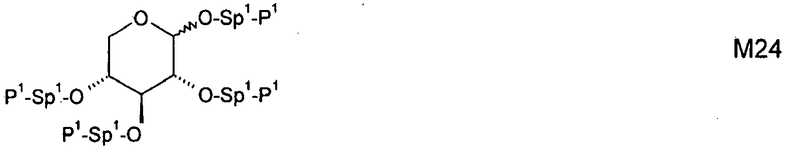 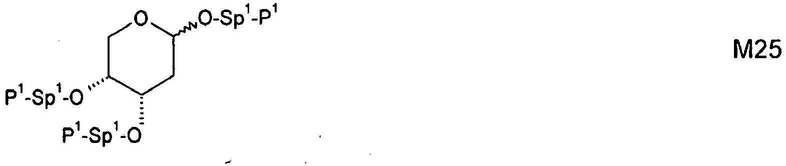 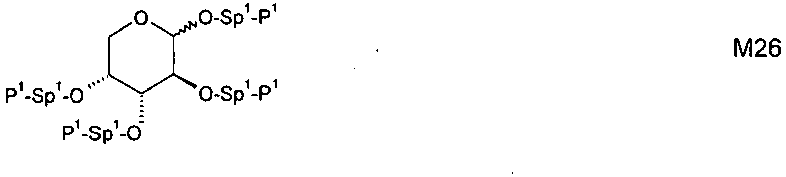 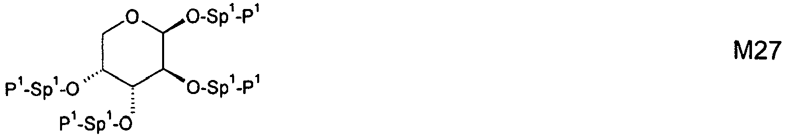 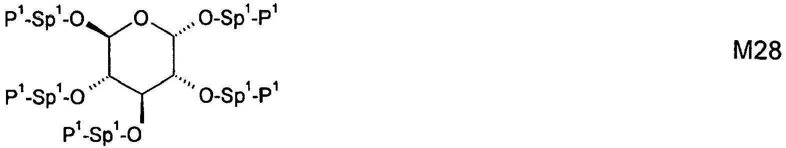 worin die einzelnen Reste folgende Bedeutung besitzen:
- P¹ und P²: jeweils unabhängig voneinander eine polymerisierbare Gruppe, vorzugsweise mit einer der vor- und nachstehend für P angegebenen Bedeutungen, besonders bevorzugt eine Acrylat-, Methacrylat-, Fluoracrylat-, Oxetan-, Vinyloxy- oder Epoxygruppe,
- Sp¹ und Sp²: jeweils unabhängig voneinander eine Einfachbindung oder eine Abstandsgruppe, vorzugsweise mit einer der vor- und nachstehend für Sp angegebenen Bedeutungen, und besonders bevorzugt -(CH₂)ₚ₁-, -(CH₂)ₚ₁-O-, -(CH₂)ₚ₁-CO-O-oder -(CH₂)ₚ₁-O-CO-O- bedeuten, worin p1 eine ganze Zahl von 1 bis 12 ist, und wobei in den letztgenannten Gruppen die Verknüpfung zur benachbarten Ring über das O-Atom erfolgt,
wobei auch einer oder mehrere der Reste P¹-Sp¹- und P²-Sp²- R^{aa} bedeuten können, mit der Maßgabe dass mindestens einer der vorhandenen Reste P¹-Sp¹- und P²-Sp²- nicht R^{aa} bedeutet,
- R^{aa}: H, F, Cl, CN oder geradkettiges oder verzweigtes Alkyl mit 1 bis 25 C-Atomen, worin auch eine oder mehrere nicht benachbarte CH₂-Gruppen jeweils unabhängig voneinander durch C(R⁰)=C(R⁰⁰)-, -C≡C-, -N(R⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, CN oder P¹-Sp¹- ersetzt sein können, besonders bevorzugt geradkettiges oder verzweigtes, optional ein- oder mehrfach fluoriertes, Alkyl, Alkoxy, Alkenyl, Alkinyl, Alkylcarbonyl, Alkoxycarbonyl, Alkylcarbonyloxy oder Alkoxycarbonyloxy mit 1 bis 12 C-Atomen (wobei die Alkenyl- und Alkinylreste mindestens zwei und die verzweigten Reste mindestens drei C-Atome aufweisen),
- R⁰, R⁰⁰: jeweils unabhängig voneinander und bei jedem Auftreten gleich oder verschieden H oder Alkyl mit 1 bis 12 C-Atomen,
- R^{y} und R^{z}: jeweils unabhängig voneinander H, F, CH₃ oder CF₃,
- Z¹: -O-, -CO-, -C(R^{y}R^{z})-,oder -CF₂CF₂-,
- Z² und Z³: jeweils unabhängig voneinander -CO-O-, -O-CO-, -CH₂O-, -OCH₂-, -CF₂O-, -OCF₂-, oder -(CH₂)ₙ-, wobei n 2, 3 oder 4 ist,
- L: bei jedem Auftreten gleich oder verschieden F, Cl, CN, oder geradkettiges oder verzweigtes, optional ein- oder mehrfach fluoriertes, Alkyl, Alkoxy, Alkenyl, Alkinyl, Alkylcarbonyl, Alkoxycarbonyl, Alkylcarbonyloxy oder Alkoxycarbonyloxy mit 1 bis 12 C-Atomen vorzugsweise F,
- L' und L": jeweils unabhängig voneinander H, F oder Cl,
- r: 0, 1, 2, 3 oder 4,
- s: 0, 1, 2 oder 3,
- t: 0, 1 oder 2,
- x: 0 oder 1.

Die FK-Medien zur Verwendung in den erfindungsgemäßen FK-Anzeigen enthalten, neben den oben beschriebenen polymerisierbaren Verbindungen, eine FK-Mischung ("Host-Mischung") enthaltend eine oder mehr, vorzugsweise zwei oder mehr niedermolekulare (d.h. monomere bzw. unpolymerisierte) Verbindungen. Letztere sind stabil bzw. unreaktiv gegenüber einer Polymerisationsreaktion unter den zur Polymerisation der polymerisierbaren Verbindungen verwendeten Bedingungen. Prinzipiell eignet sich als Host-Mischung jede zur Verwendung in herkömmlichen VA- und OCB-Anzeigen geeignete FK-Mischung. Geeignete FK-Mischungen sind dem Fachmann bekannt und in der Literatur beschrieben, beispielsweise Mischungen in VA-Anzeigen in EP 1 378 557 A1, und Mischungen für OCB-Anzeigen in EP 1 306 418 A1 und DE 102 24 046 A1.

In einer ersten bevorzugten Ausführungsform der vorliegenden Erfindung enthält das FK-Medium eine FK-Hostmischung basierend auf Verbindungen mit negativer dielektrischer Anisotropie. Solche FK-Medien eignen sich besonders zur Verwendung in PSA-VA-Anzeigen. Besonders bevorzugte Ausführungsformen für solche FK-Medien werden in den folgenden Abschnitten a-x genannt:
a) FK-Medium, welches eine oder mehrere Verbindungen ausgewählt aus der Gruppe bestehend aus den Formeln CY und PY enthält: 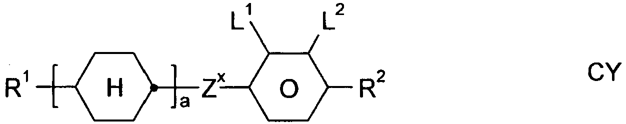 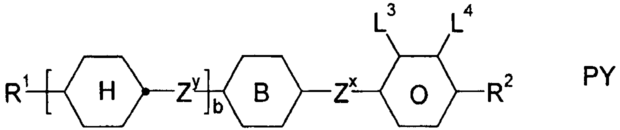 worin die einzelnen Reste folgende Bedeutung besitzen
   - a: 1 oder 2,
   - b: 0 oder 1, 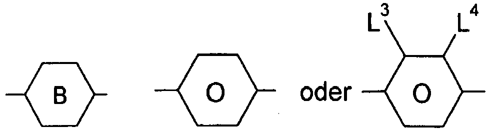
   - R¹ und R²: jeweils unabhängig voneinander Alkyl mit 1 bis 12 C-Atomen, wobei auch eine oder zwei nicht benachbarte CH₂-Gruppen durch -O-, -CH=CH-, -CO-, -OCO- oder - COO- so ersetzt sein können, dass O-Atome nicht direkt miteinander verknüpft sind, vorzugsweise Alkyl oder Alkoxy mit 1 bis 6 C-Atomen,
   - Z^{x} und Z^{y}: jeweils unabhängig voneinander -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, oder eine Einfachbindung, vorzugsweise eine Einfachbindung,
   - L¹⁻⁴: jeweils unabhängig voneinander F, Cl, OCF₃, CF₃, CH₃, CH₂F, CHF₂.

   Vorzugsweise bedeuten beide Reste L¹ und L² F, oder einer der Reste L¹ und L² F und der andere Cl, bzw. beide Reste L³ und L⁴ F, oder einer der Reste L³ und L⁴ F und der andere Cl.
   Die Verbindungen der Formel CY sind vorzugsweise ausgewählt aus der Gruppe bestehend aus folgenden Unterformeln 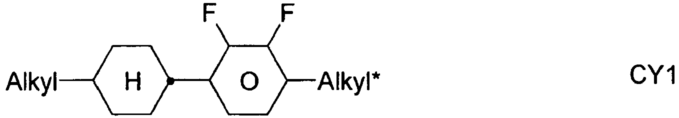 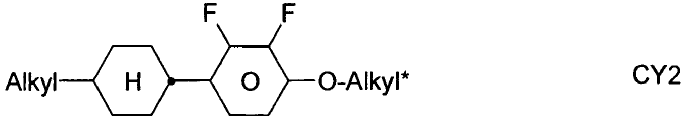 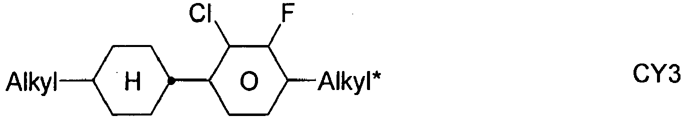 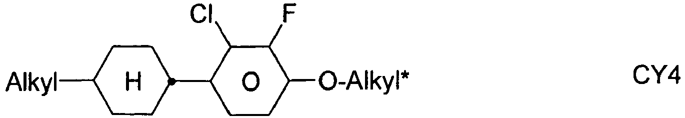 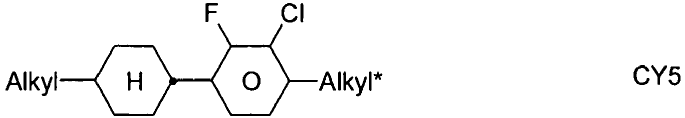 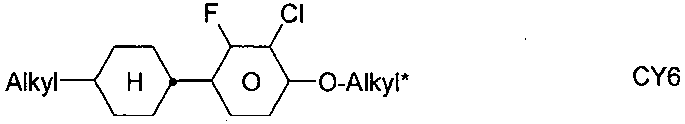 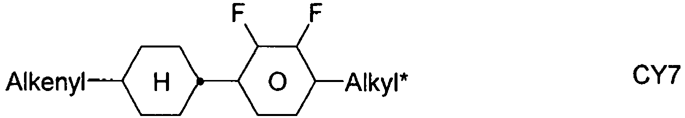 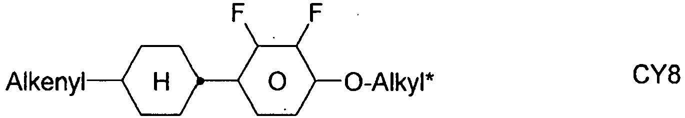 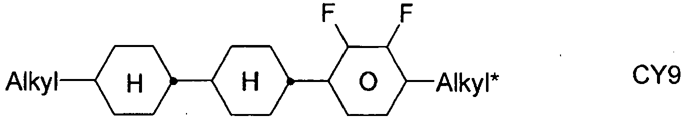 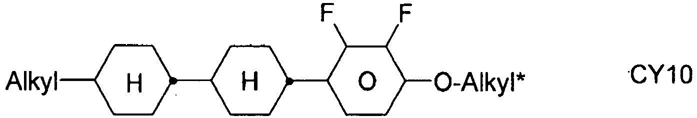 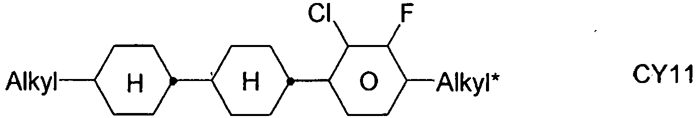 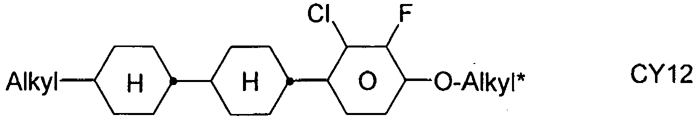 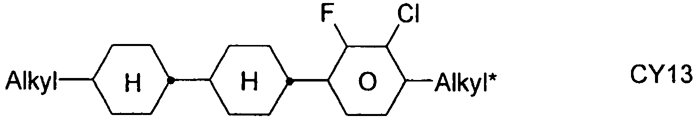 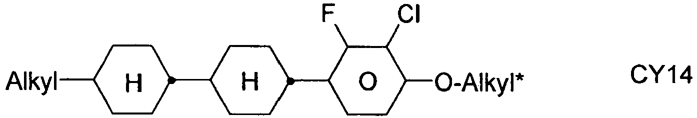 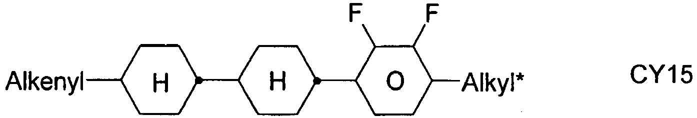 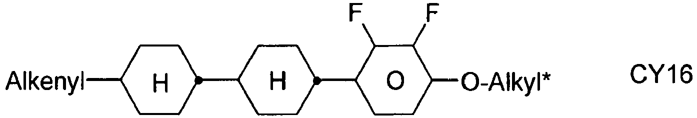 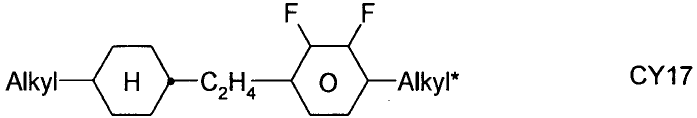 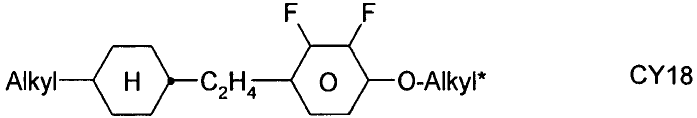 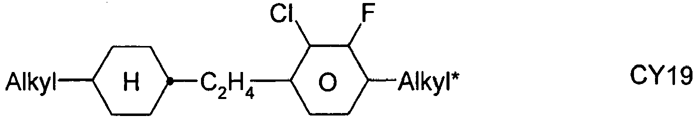 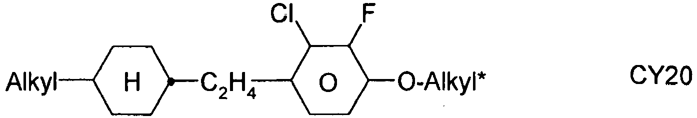 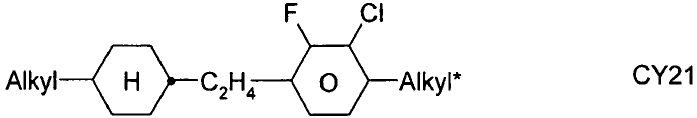 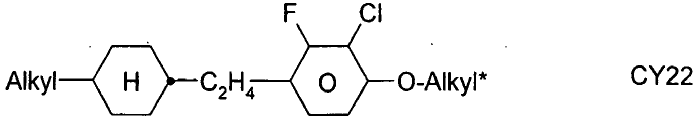 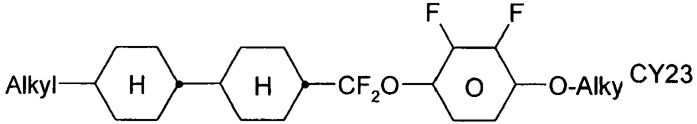 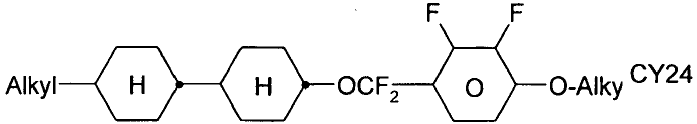 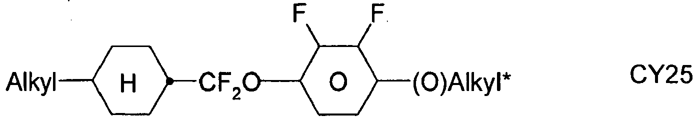 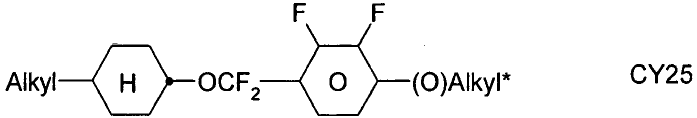 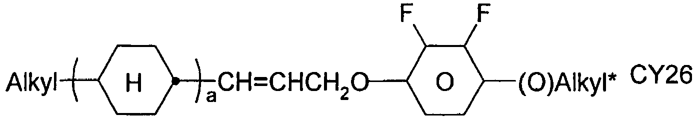 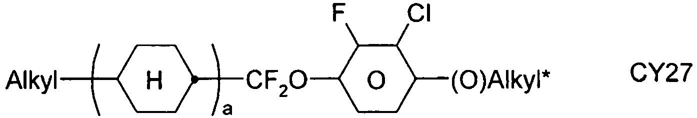 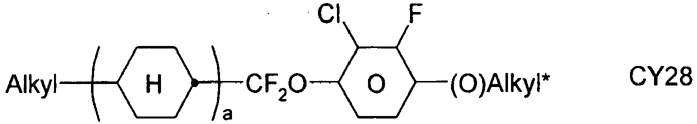 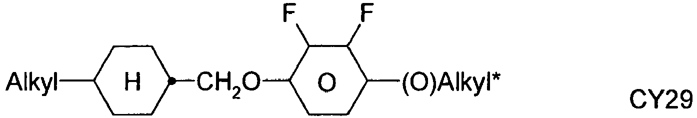 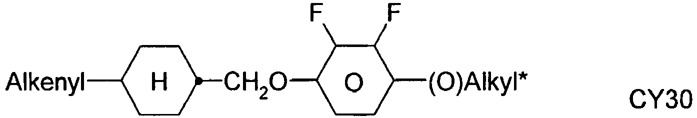 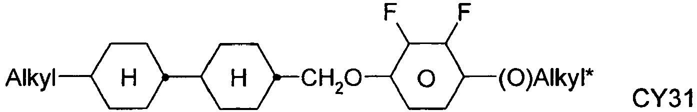 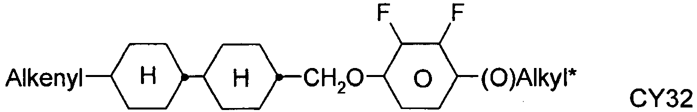 worin a 1 oder 2, Alkyl und Alkyl* jeweils unabhängig voneinander einen geradkettigen Alkylrest mit 1-6 C-Atomen, und Alkenyl einen geradkettigen Alkenylrest mit 2-6 C-Atomen, und (O) ein Sauerstoffatom oder eine Einfachbindung bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
   Die Verbindungen der Formel PY sind vorzugsweise ausgewählt aus der Gruppe bestehend aus folgenden Unterformeln: 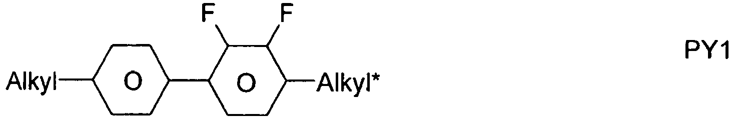 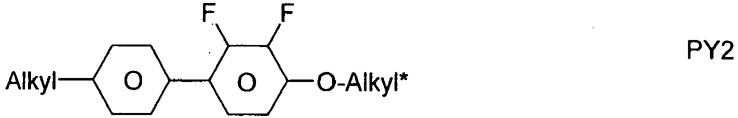 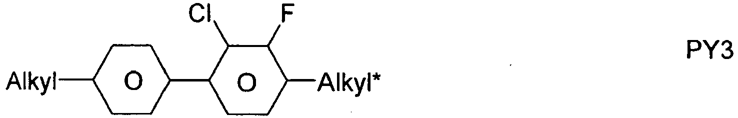 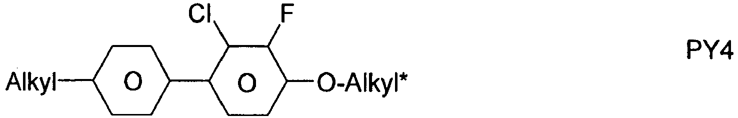 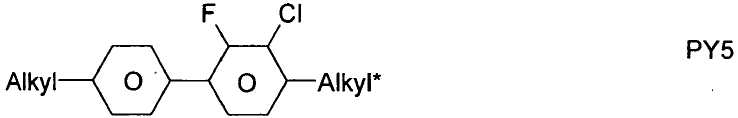 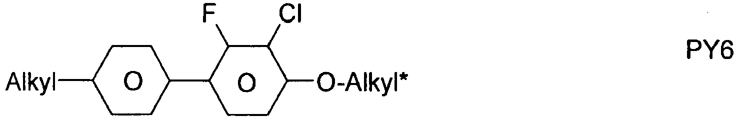 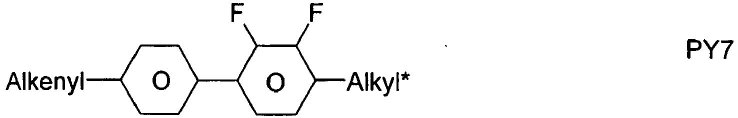 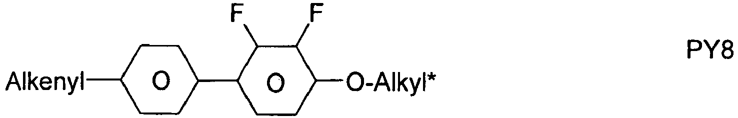 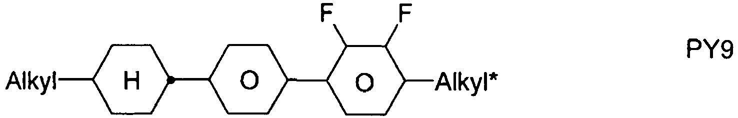 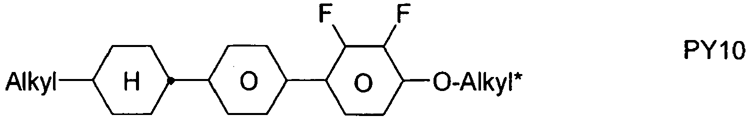 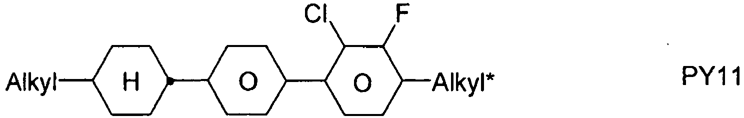 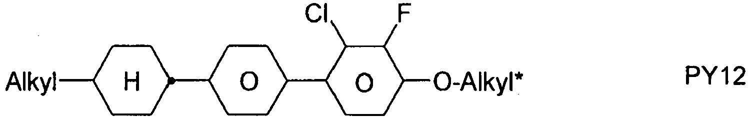 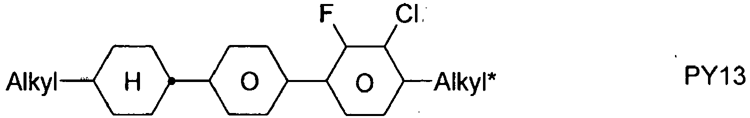 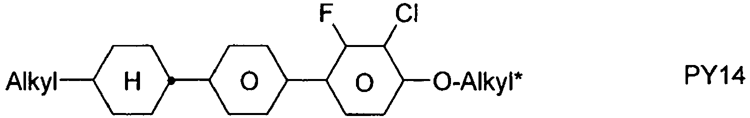 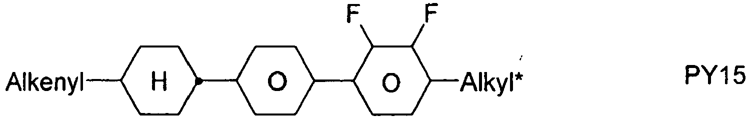 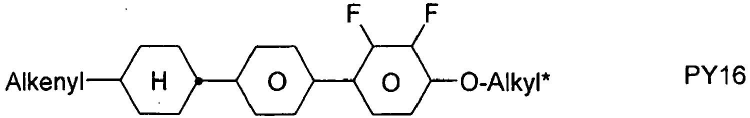 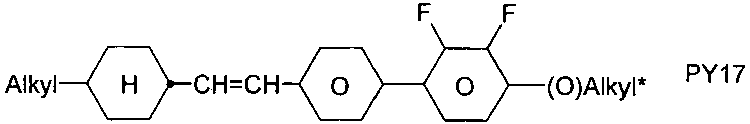 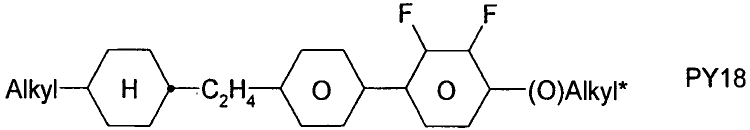 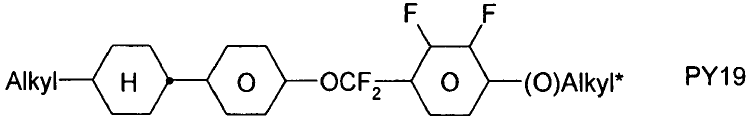 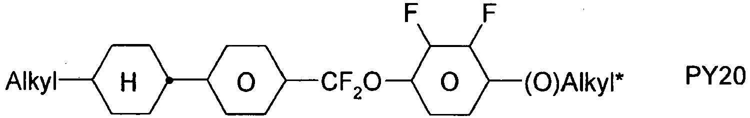 worin Alkyl und Alkyl* jeweils unabhängig voneinander einen geradkettigen Alkylrest mit 1-6 C-Atomen und Alkenyl einen geradkettigen Alkenylrest mit 2-6 C-Atomen, und (O) ein Sauerstoffatom oder eine Einfachbindung bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
b) FK-Medium, welches zusätzlich Verbindungen der Formel eine oder mehrere Verbindungen der folgenden Formel enthält: 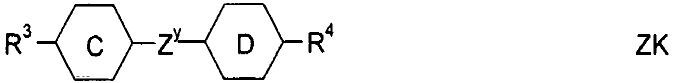 worin die einzelnen Reste folgende Bedeutung besitzen 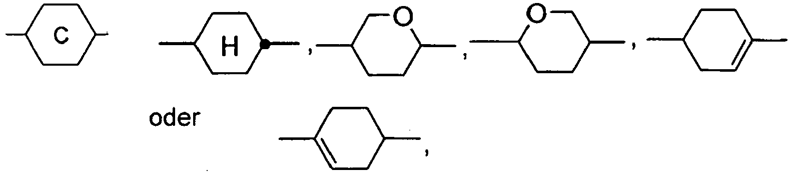 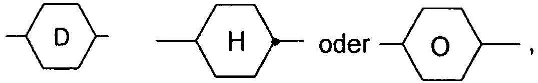
   - R³ und R⁴: jeweils unabhängig voneinander Alkyl mit 1 bis 12 C-Atomen, worin auch eine oder zwei nicht benachbarte CH₂-Gruppen durch -O-, -CH=CH-, -CO-, -O-CO- oder -CO-O- so ersetzt sein können, dass O-Atome nicht direkt miteinander verknüpft sind,
   - Z^{y}: -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, oder eine Einfachbindung, vorzugsweise eine Einfachbindung.

   Die Verbindungen der Formel ZK sind vorzugsweise ausgewählt aus der Gruppe bestehend aus folgenden Unterformeln: 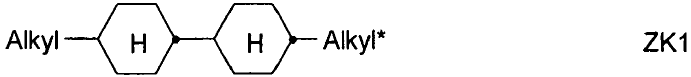 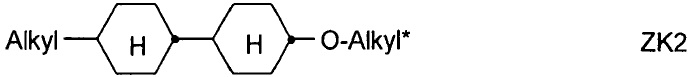 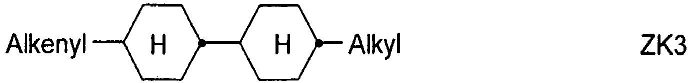 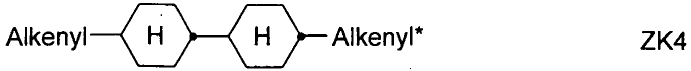 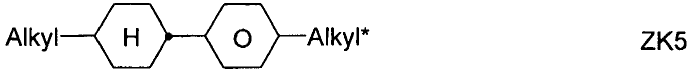 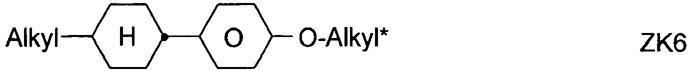 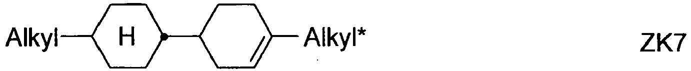 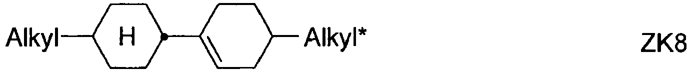 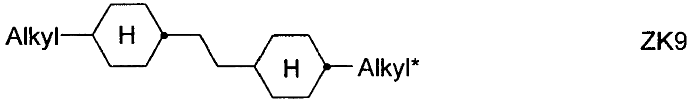 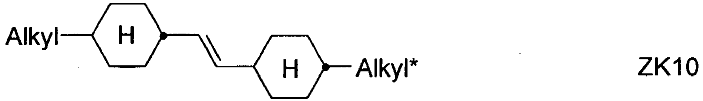 worin Alkyl und Alkyl* jeweils unabhängig voneinander einen geradkettigen Alkylrest mit 1-6 C-Atomen bedeuten, und Alkenyl einen geradkettigen Alkenylrest mit 2-6 C-Atomen bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
c) FK-Medium, welches zusätzlich eine oder mehrere Verbindungen der folgenden Formel enthält: 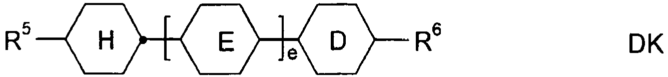 worin die einzelnen Reste bei jedem Auftreten gleich oder verschieden folgende Bedeutung haben:
   - R⁵ und R⁶: jeweils unabhängig voneinander eine der oben für R¹ angegebenen Bedeutungen,

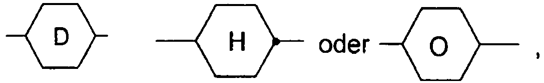

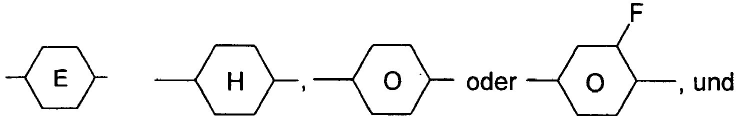

   - e: 1 oder 2.

   Die Verbindungen der Formel DK sind vorzugsweise ausgewählt aus der Gruppe bestehend aus folgenden Unterformeln: 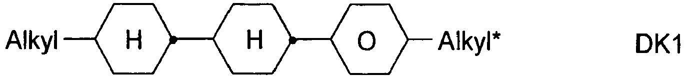 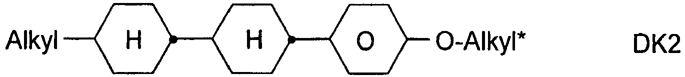 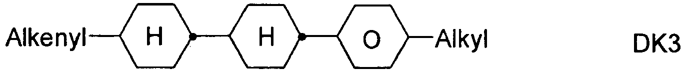 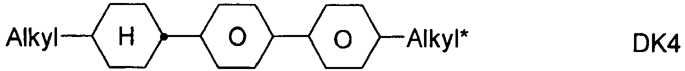 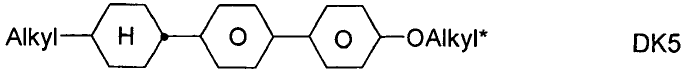 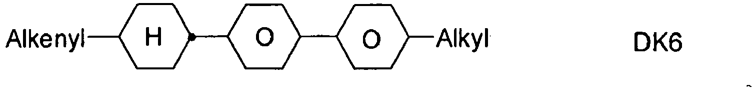 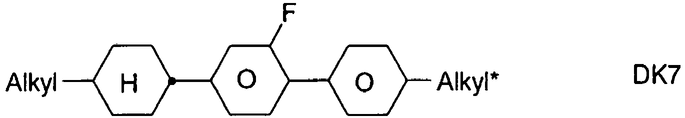 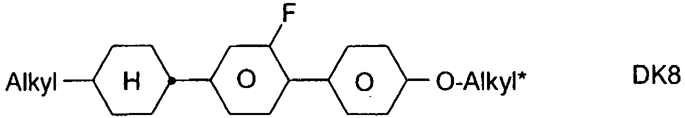 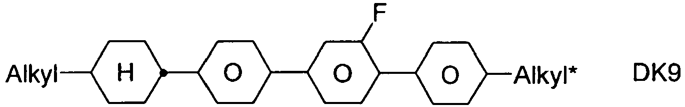 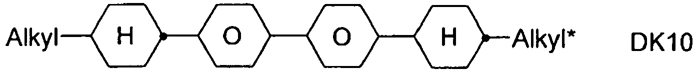 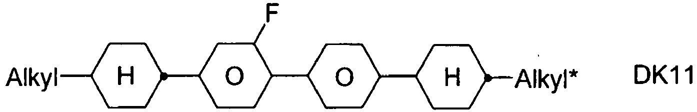 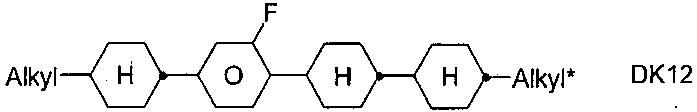 worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
d) FK-Medium, welches zusätzlich eine oder mehrere Verbindungen der folgenden Formel enthält: 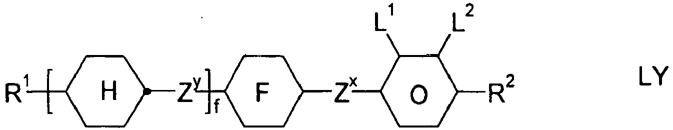 worin die einzelnen Reste folgende Bedeutung besitzen 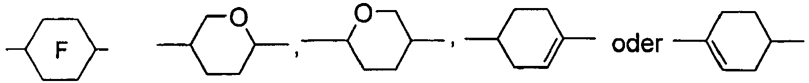
   - f: 0 oder 1,
   - R¹ und R²: jeweils unabhängig voneinander Alkyl mit 1 bis 12 C-Atomen, wobei auch eine oder zwei nicht benachbarte CH₂-Gruppen durch -O-, -CH=CH-, -CO-, -OCO- oder - COO- so ersetzt sein können, dass O-Atome nicht direkt miteinander verknüpft sind,
   - Z^{x} und Z^{y}: jeweils unabhängig voneinander -CH₂CH₂-, -CH=CH-, -CF₂O-, -OCF₂-, -CH₂O-, -OCH₂-, -CO-O-, -O-CO-, -C₂F₄-, -CF=CF-, -CH=CH-CH₂O-, oder eine Einfachbindung, vorzugsweise eine Einfachbindung,
   - L¹ und L²: jeweils unabhängig voneinander F, Cl, OCF₃, CF₃, CH₃, CH₂F, CHF₂.

   Vorzugsweise bedeuten beide Reste L¹ und L² F oder einer der Reste L¹ und L² F und der andere Cl. Die Verbindungen der Formel LY sind vorzugsweise ausgewählt aus der Gruppe bestehend aus folgenden Unterformeln 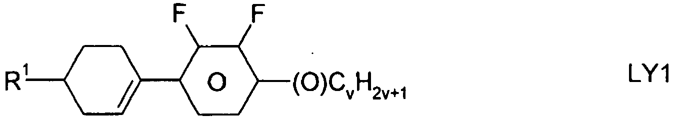 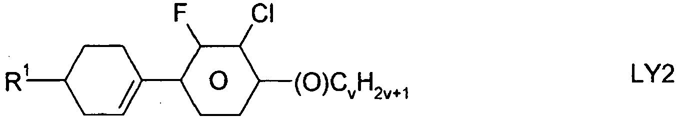 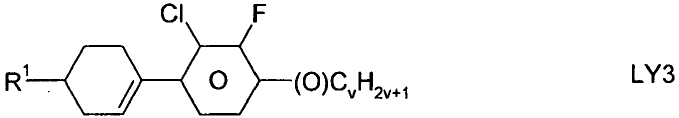 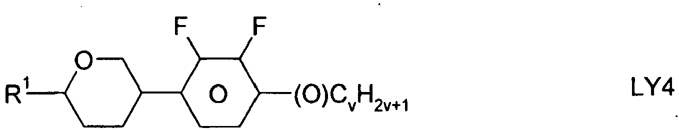 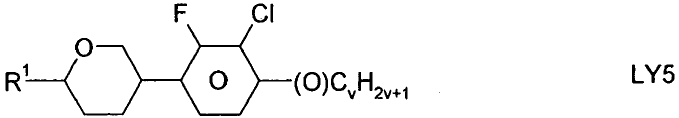 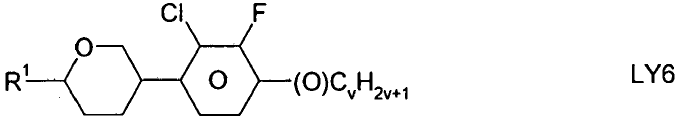 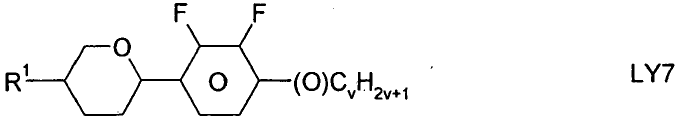 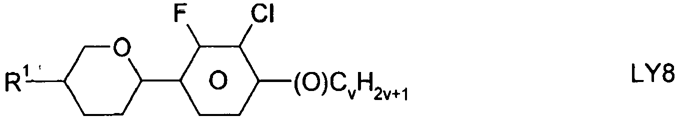 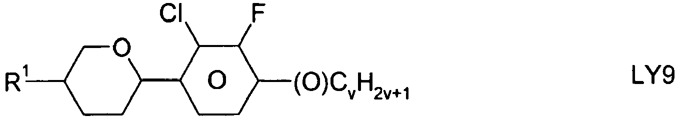 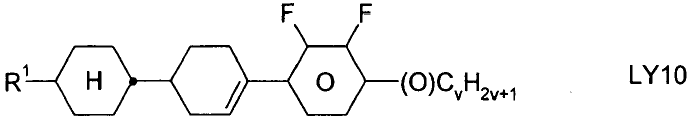 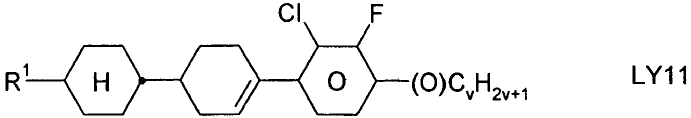 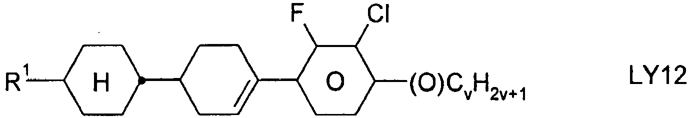 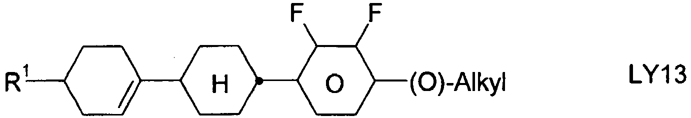 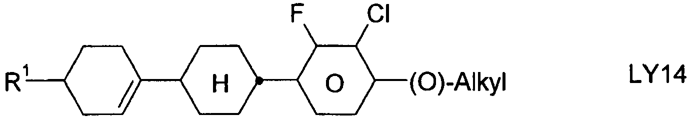 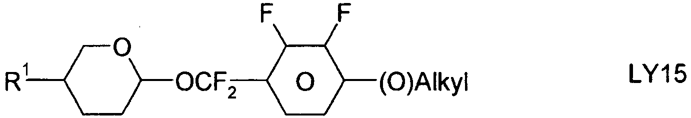 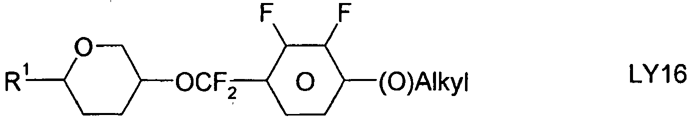 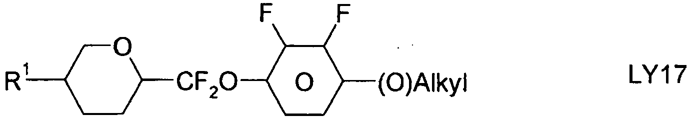 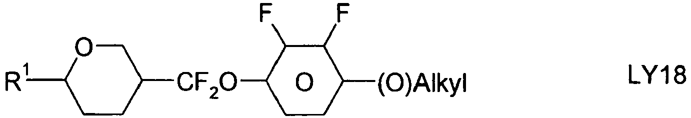 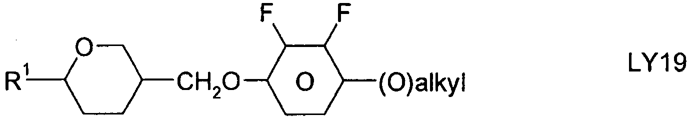 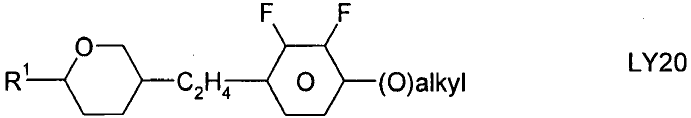 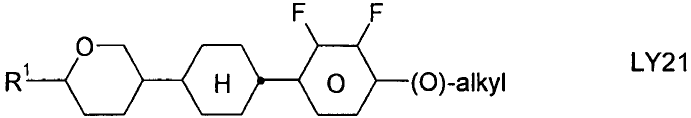 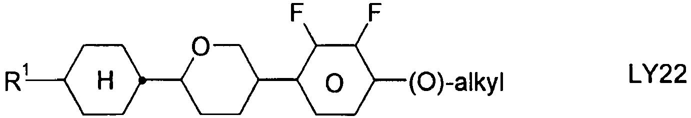 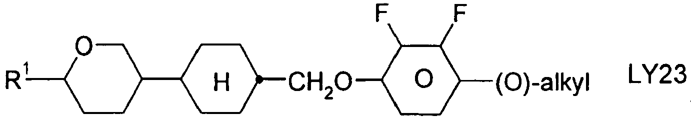 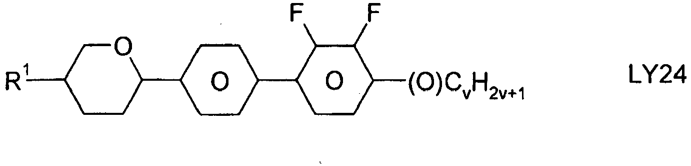 worin R¹ die oben angegebene Bedeutung hat, Alkyl einen geradkettigen Alkylrest mit 1-6 C-Atomen, (O) ein Sauerstoffatom oder eine Einfachbindung und v eine ganze Zahl von 1 bis 6 bedeuten. R¹ bedeutet vorzugsweise geradkettiges Alkyl mit 1 bis 6 C-Atomen oder geradkettiges Alkenyl mit 2 bis 6 C-Atomen, insbesondere CH₃, C₂H₅, n-C₃H₇, n-C₄H₉, n-C₅H₁₁, CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
e) FK-Medium, welches zusätzlich eine oder mehrere Verbindungen ausgewählt aus der Gruppe bestehend aus folgenden Formeln enthält: 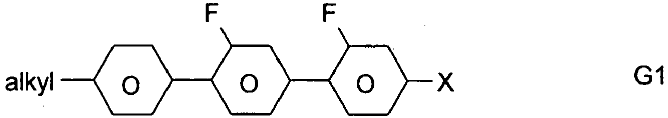 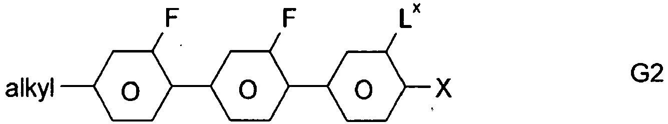 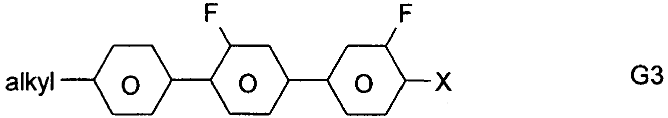 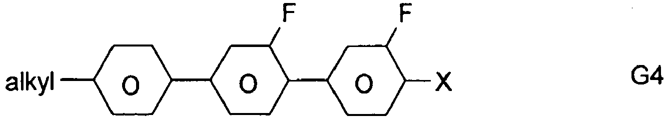 worin alkyl C₁₋₆-alkyl, L^{x} H oder F und X F, Cl, OCF₃, OCHF₂ oder OCH=CF₂ bedeutet. Besonders bevorzugt sind Verbindungen der Formel G1, worin X F bedeutet.
f) FK-Medium, welches zusätzlich eine oder mehrere Verbindungen ausgewählt aus der Gruppe bestehend aus folgenden Formeln enthält: 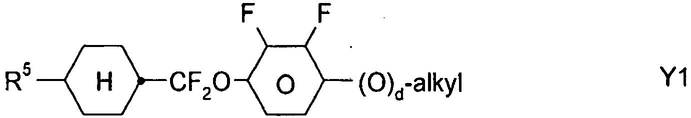 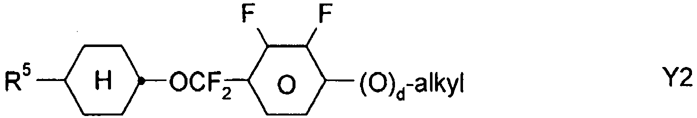 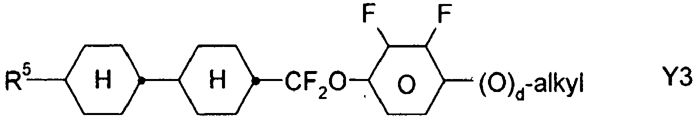 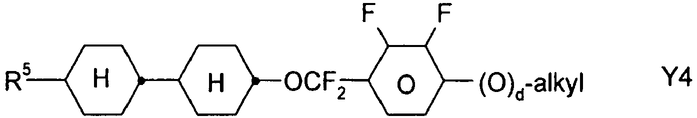 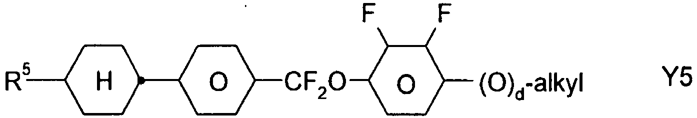 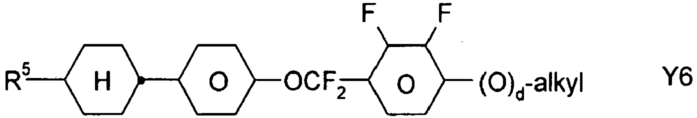 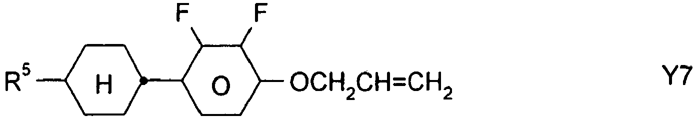 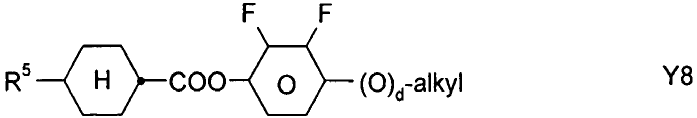 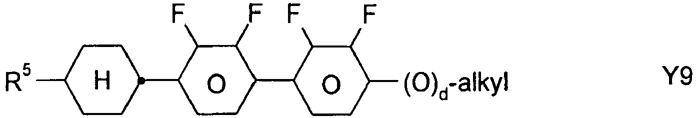 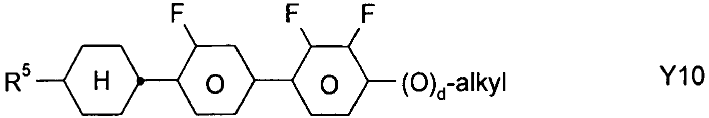 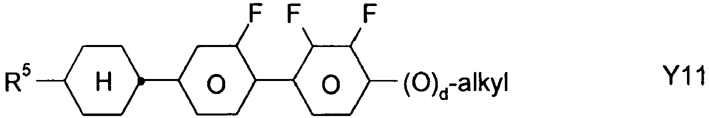 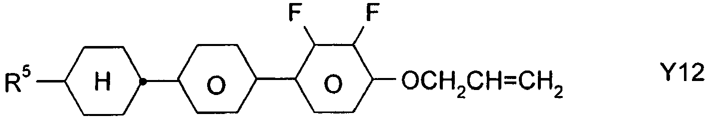 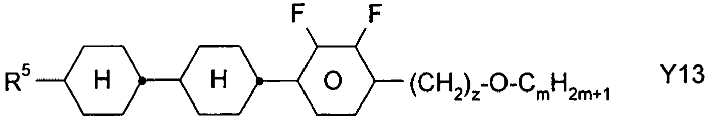 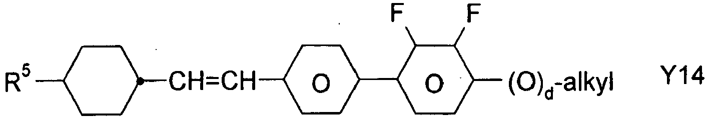 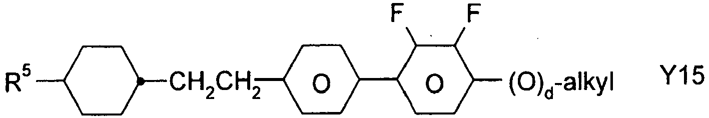 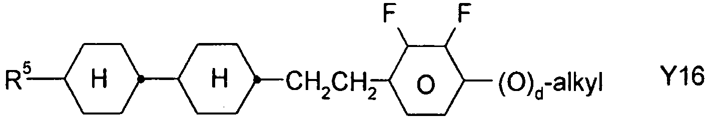 worin R⁵ eine der oben für R¹ angegebenen Bedeutungen besitzt, alkyl C₁₋₆-alkyl, d 0 oder 1, und z und m jeweils unabhängig voneinander eine ganze Zahl von 1 bis 6 bedeuten. R⁵ ist in diesen Verbindungen besonders bevorzugt C₁₋₆-alkyl oder -alkoxy oder C₂₋₆-alkenyl, d ist vorzugsweise 1. Vorzugsweise enthält das erfindungsgemäße FK-Medium eine oder mehrere Verbindungen der oben genannten Formeln in Mengen von ≥ 5 Gew.%.
g) FK-Medium, welches zusätzlich eine oder mehrere Biphenylverbindungen ausgewählt aus der Gruppe bestehend aus folgenden Formeln enthält: 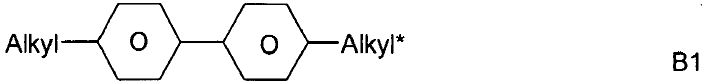 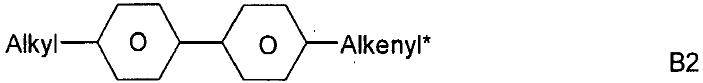 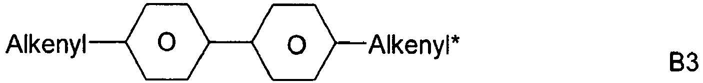 worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
   Der Anteil der Biphenyle der Formeln B1 bis B3 in der FK-Mischung beträgt vorzugsweise mindestens 3 Gew.%, insbesondere ≥ 5 Gew.%.
   Die Verbindungen der Formel B2 sind besonders bevorzugt.
   Die Verbindungen der Formel B1 bis B3 sind vorzugsweise ausgewählt aus der Gruppe bestehend aus folgenden Unterformeln 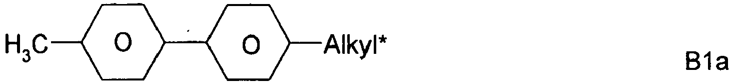 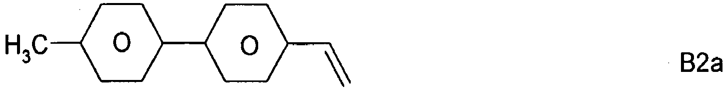 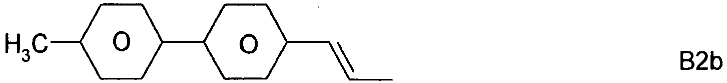 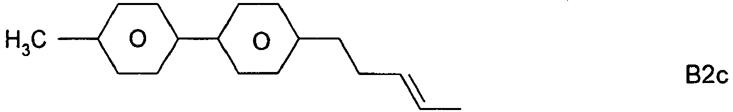 worin Alkyl* einen Alkylrest mit 1-6 C-Atomen bedeutet. Insbesondere bevorzugt enthält das erfindungsgemäße Medium eine oder mehrere Verbindungen der Formeln B1a und/oder B2c.
h) FK-Medium, welches zusätzlich eine oder mehrere Terphenylverbindungen der folgenden Formel enthält: 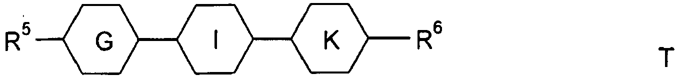 worin R⁵ und R⁶ jeweils unabhängig voneinander eine der oben für R¹ angegebenen Bedeutungen besitzen und 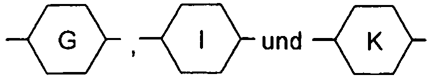 jeweils unabhängig voneinander 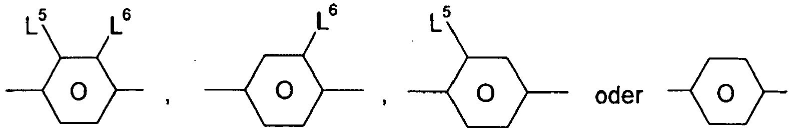
   bedeuten, worin L⁵ F oder Cl, vorzugsweise F, und L⁶ F, Cl, OCF₃, CF₃, CH₃, CH₂F oder CHF₂, vorzugsweise F, bedeuten.
   Die Verbindungen der Formel T sind vorzugsweise ausgewählt aus der Gruppe bestehend aus folgenden Unterformeln 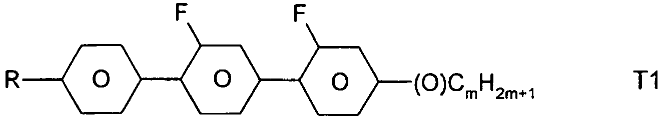 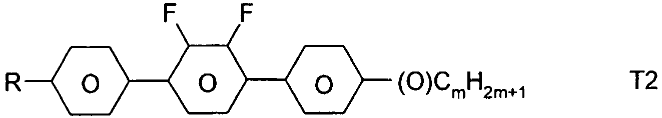 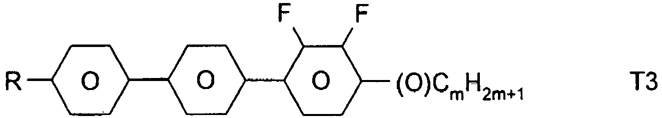 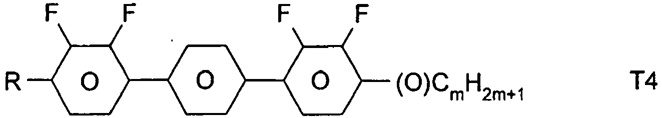 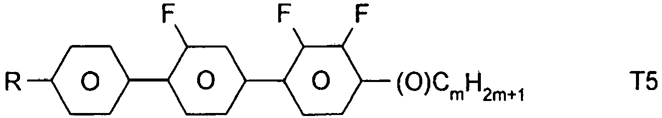 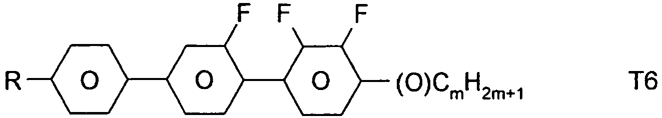 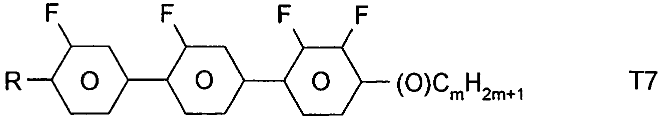 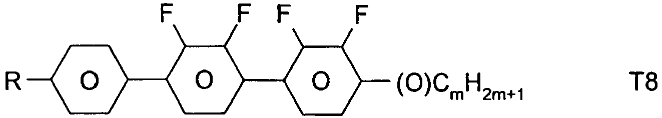 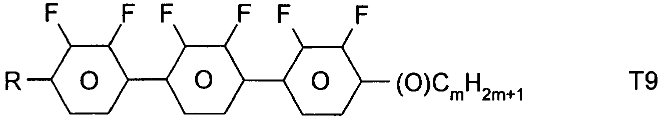 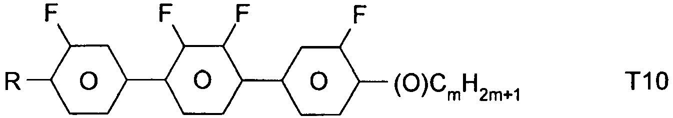 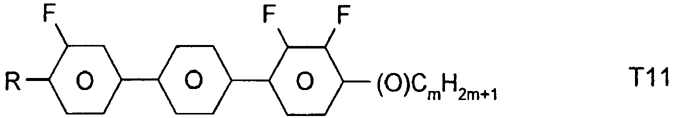 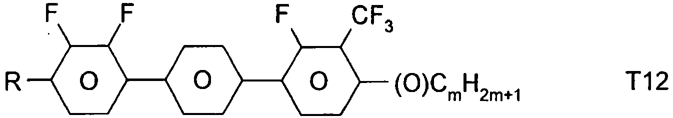 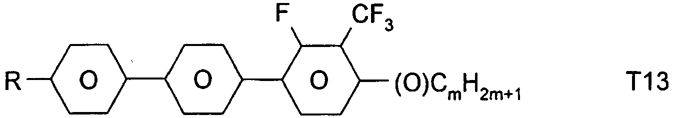 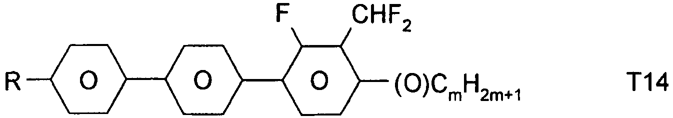 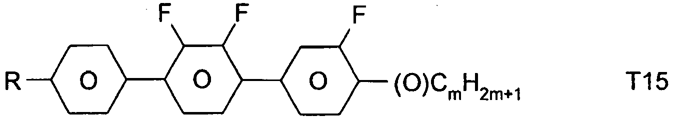 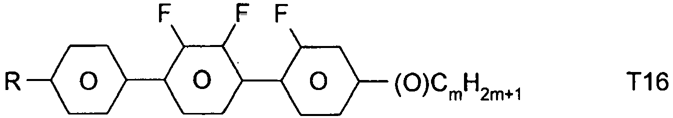 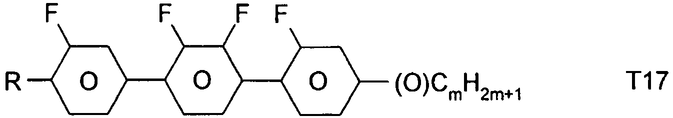 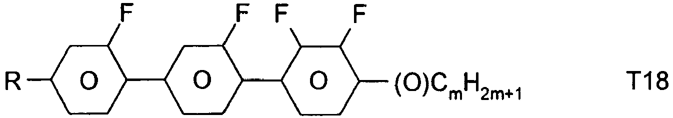 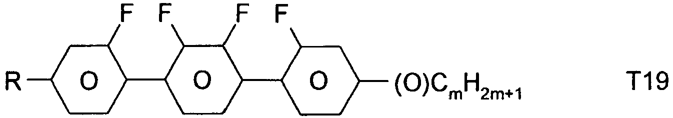 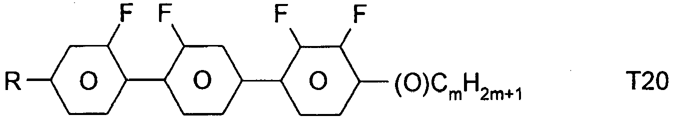 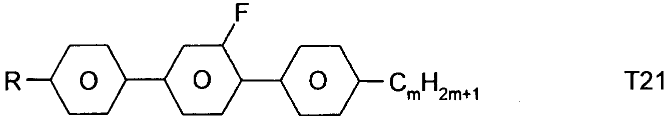 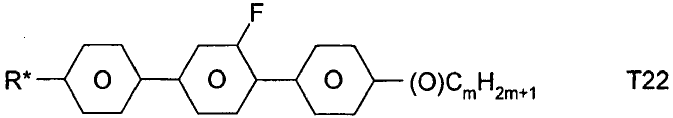 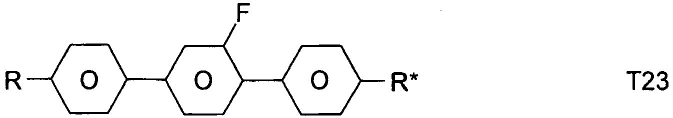 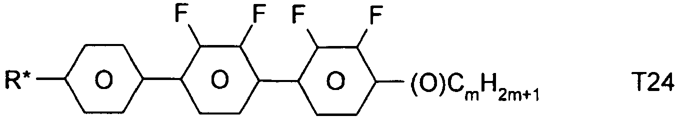 worin R einen geradkettigen Alkyl- oder Alkoxyrest mit 1-7 C-Atomen, R* einen geradkettigen Alkenylrest mit 2-7 C-Atomen, (O) ein Sauerstoffatom oder eine Einfachbindung, und m eine ganze Zahl von 1 bis 6 bedeutet. R* bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
   Vorzugsweise bedeutet R Methyl, Ethyl, Propyl, Butyl, Pentyl, Hexyl Methoxy, Ethoxy, Propoxy, Butoxy oder Pentoxy.
   Das erfindungsgemäße FK-Medium enthält die Terphenyle der Formeln T und deren bevorzugte Unterformeln vorzugsweise in einer Menge von 0,5-30 Gew.%, insbesondere von 1-20 Gew.%.
   Besonders bevorzugt sind Verbindungen der Formeln T1, T2, T3 und T21. In diesen Verbindungen bedeutet R vorzugsweise Alkyl, ferner Alkoxy jeweils mit 1-5 C-Atomen.
   Vorzugsweise werden die Terphenyle in erfindungsgemäßen Mischungen eingesetzt, wenn der Δn-Wert der Mischung ≥ 0,1 sein soll. Bevorzugte Mischungen enthalten 2-20 Gew.% einer oder mehrerer Terphenyl-Verbindungen der Formel T, vorzugsweise ausgewählt aus der Gruppe der Verbindungen T1 bis T22.
i) FK-Medium, welches zusätzlich eine oder mehrere Verbindungen ausgewählt aus der Gruppe bestehend aus folgenden Formeln enthält: 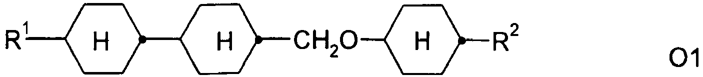 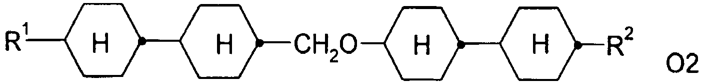 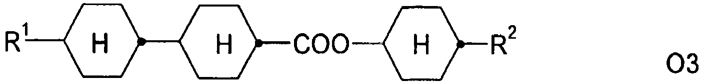 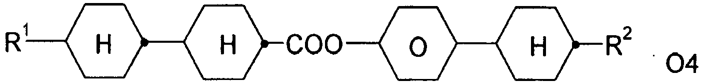 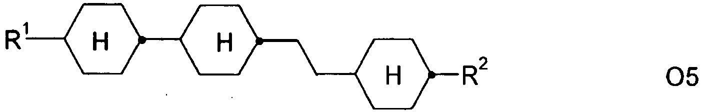 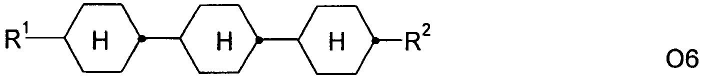 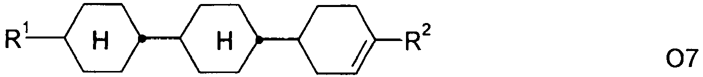 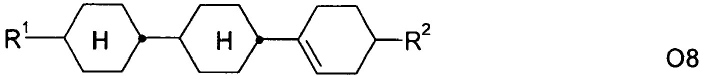 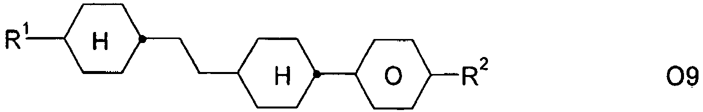 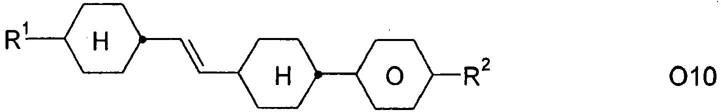 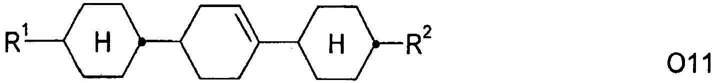 worin R¹ und R² die oben angegebenen Bedeutungen haben, und vorzugsweise jeweils unabhängig voneinander geradkettiges Alkyl mit 1 bis 6 C-Atomen oder geradkettiges Alkenyl mit 2 bis 6 C-Atomen bedeuten.
   Bevorzugte Medien enthalten eine oder mehrere Verbindungen ausgewählt aus den Formeln 01, 03 und 04.
k) FK-Medium, welches zusätzlich eine oder mehrere Verbindungen der folgenden Formel enthält: 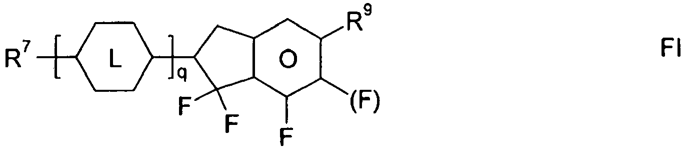 worin 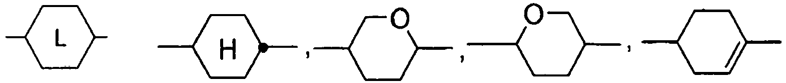 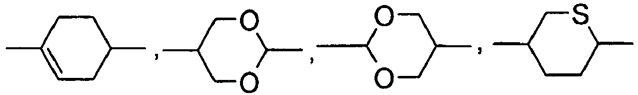 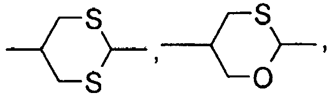 R⁹ H, CH₃, C₂H₅ oder n-C₃H₇, (F) einen optionalen Fluorsubstituenten und q 1, 2 oder 3 bedeutet, und R⁷ eine der für R¹ angegebenen Bedeutungen hat, vorzugsweise in Mengen von > 3 Gew.%, insbesondere ≥ 5 Gew.%, und ganz besonders bevorzugt von 5-30 Gew.%.
   Besonders bevorzugte Verbindungen der Formel IF sind ausgewählt aus der Gruppe bestehend aus folgenden Unterformeln: 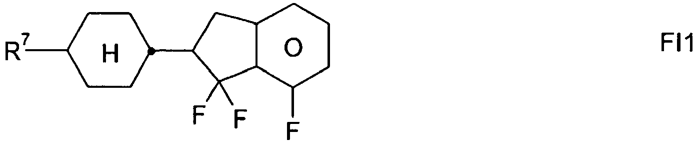 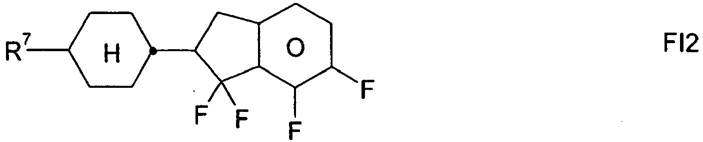 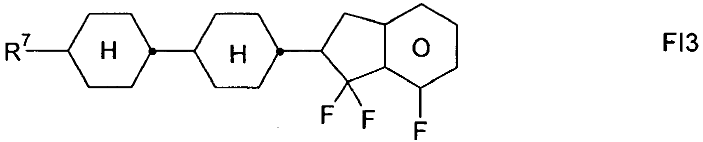 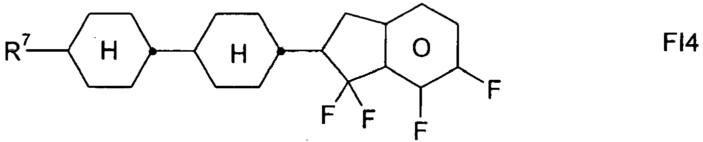 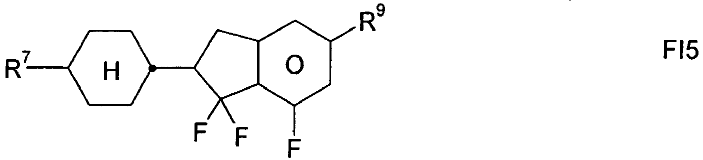 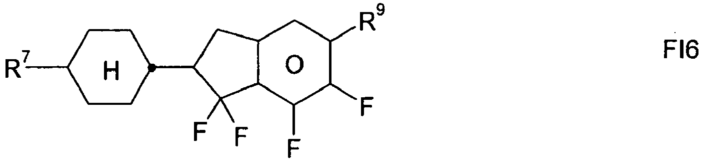 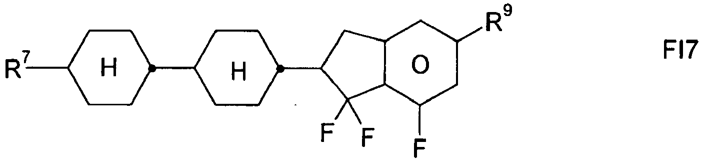 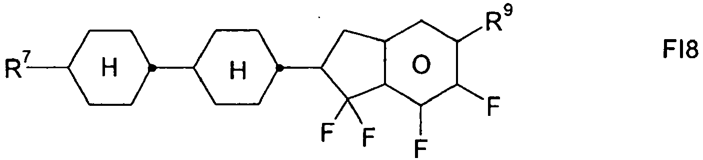 worin R⁷ vorzugsweise geradkettiges Alkyl bedeutet und R⁹ CH₃, C₂H₅ oder n-C₃H₇ bedeutet. Besonders bevorzugt sind die Verbindungen der Formel FI1, FI2 und FI3.
m) FK-Medium, welches zusätzlich eine oder mehrere Verbindungen ausgewählt aus der Gruppe bestehend aus folgenden Formeln enthält: 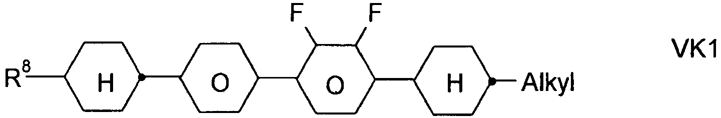 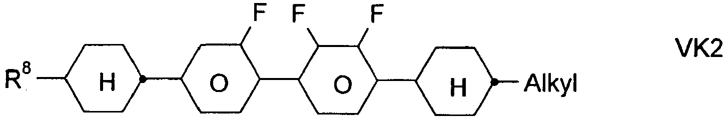 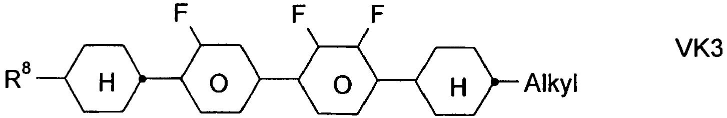 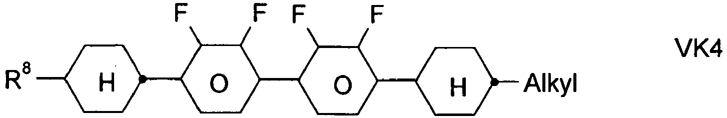 worin R⁸ die für R¹ angegebene Bedeutung hat und Alkyl einen geradkettigen Alkylrest mit 1-6 C-Atomen bedeutet.
n) FK-Medium, welches zusätzlich eine oder mehrere Verbindungen enthält, die eine Tetrahydronaphthyl- oder Naphthyl-Einheit aufweisen, wie z.B. die Verbindungen ausgewählt aus der Gruppe bestehend aus folgenden Formeln: 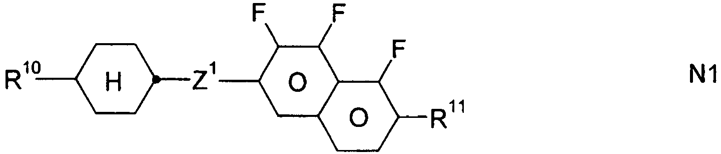 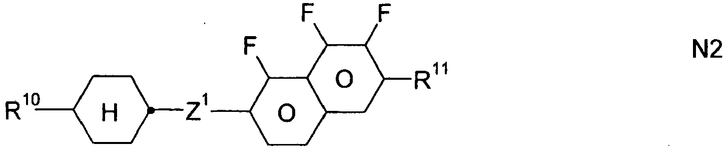 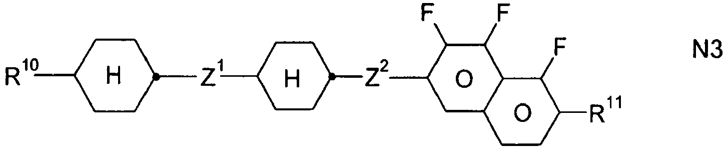 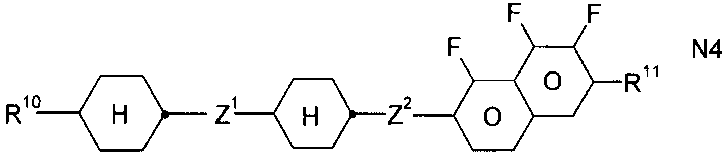 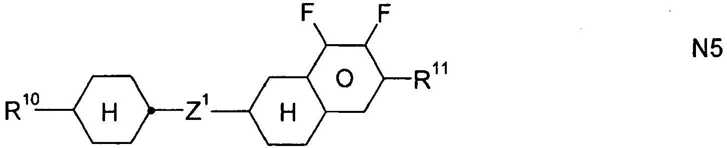 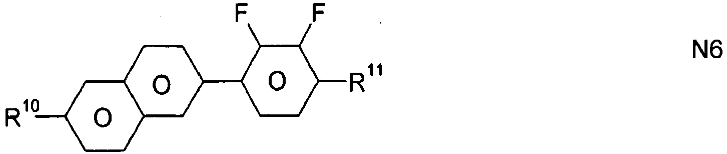 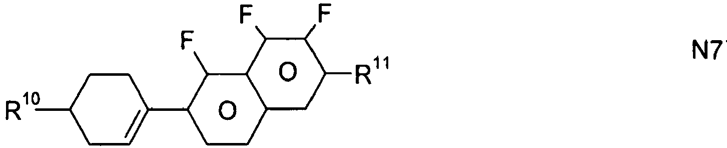 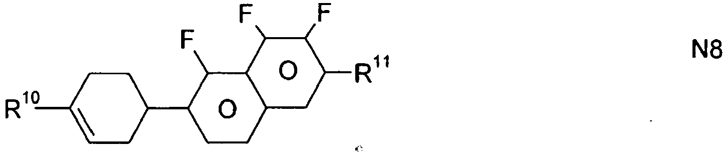 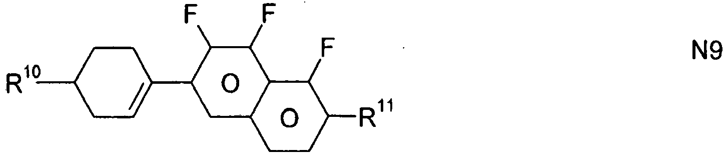 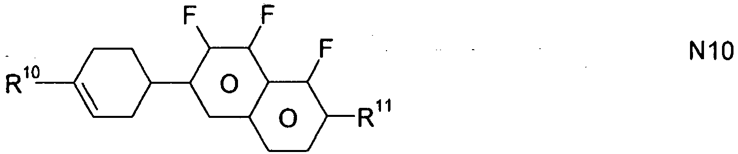 worin R¹⁰ und R¹¹ jeweils unabhängig voneinander eine der für R¹ angegebenen Bedeutungen haben, vorzugsweise geradkettiges Alkyl oder Alkoxy mit 1 bis 6 C-Atomen oder geradkettiges Alkenyl mit 2 bis 6 C-Atomen bedeuten, und Z¹ und Z² jeweils unabhängig voneinander -C₂H₄-, -CH=CH-, -(CH₂)₄-, -(CH₂)₃O-, -O(CH₂)₃-, -CH=CH-CH₂CH₂-, -CH₂CH₂CH=CH-, -CH₂O-, -OCH₂-, -CO-O-, -O-CO-, -C₂F₄-, -CF=CF-, -CF=CH-, -CH=CF-, -CH₂- oder eine Einfachbindung bedeuten.
o) FK-Medium, welches zusätzlich eine oder mehrere eine oder mehrere Difluordibenzochromane und/oder Chromane der folgenden Formeln enthält: 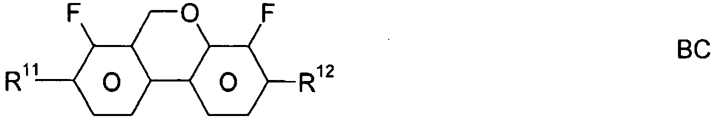 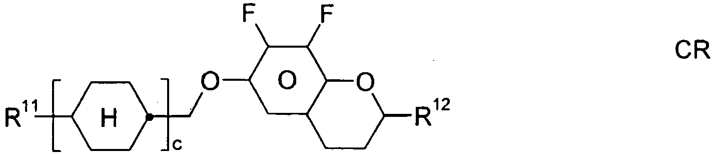 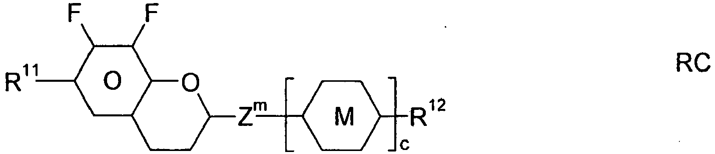 worin R¹¹ und R¹² jeweils unabhängig voneinander die oben angegebene Bedeutung aufweisen, Ring M trans-1,4-Cyclohexylen oder 1,4-Phenylen bedeutet, Z^{m} -C₂H₄-, -CH₂O-, -OCH₂-, -CO-O-oder -O-CO- bedeutet, und c 0 oder 1 bedeutet, vorzugsweise in Mengen von 3 bis 20 Gew.%, insbesondere in Mengen von 3 bis 15 Gew. %.
   Besonders bevorzugte Verbindungen der Formeln BC und CR sind ausgewählt aus der Gruppe bestehend aus folgenden Unterformeln: 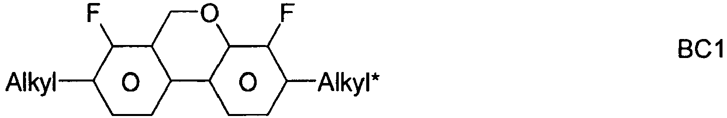 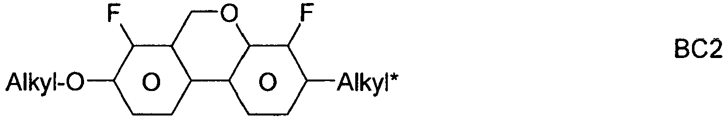 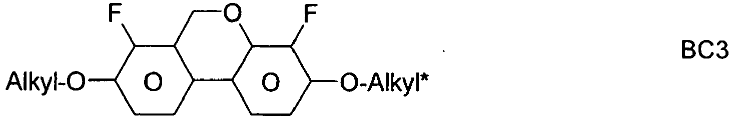 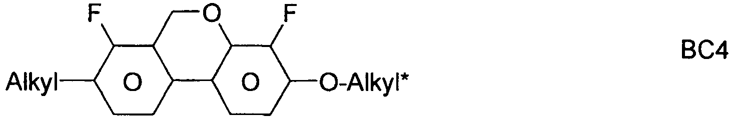 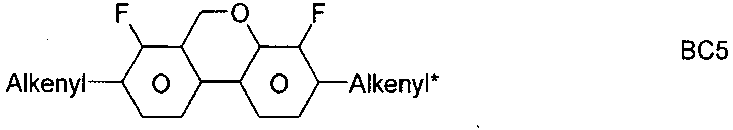 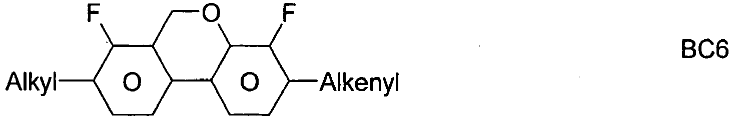 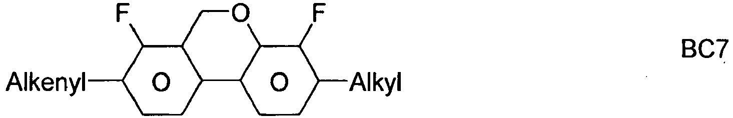 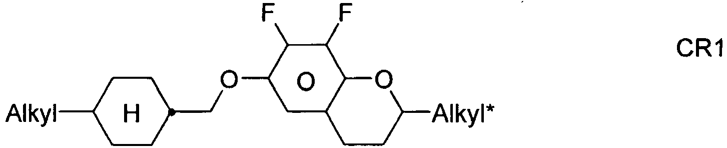 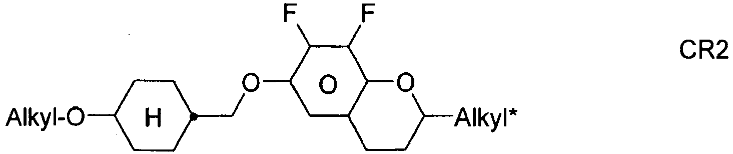 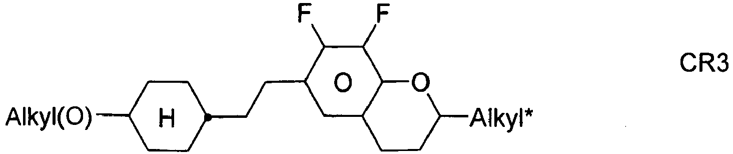 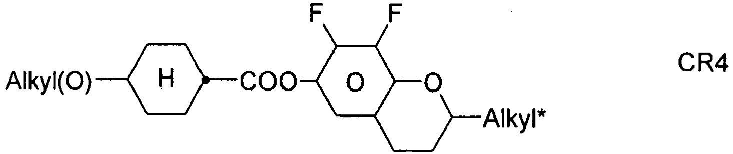 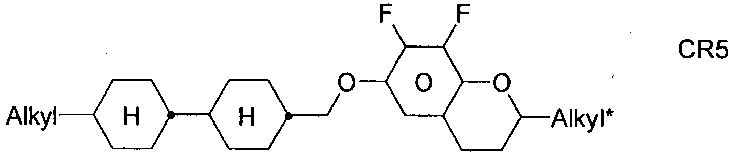 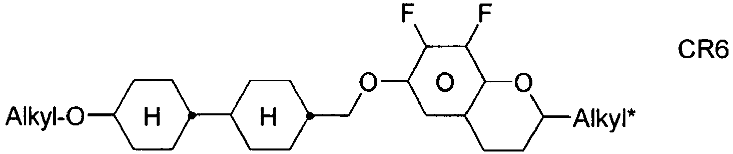 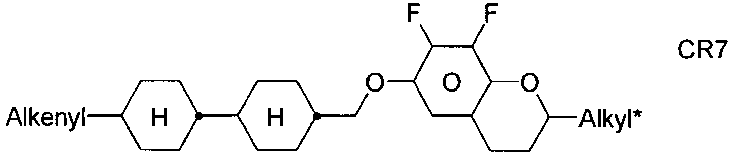 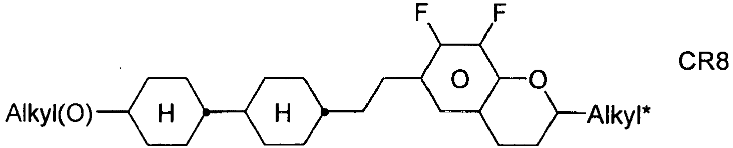 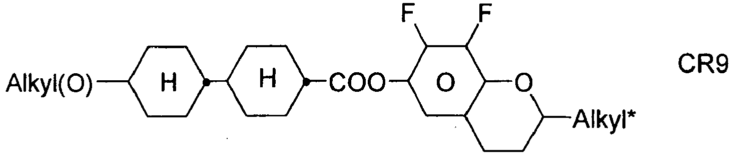 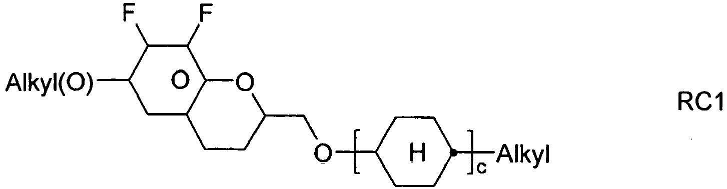 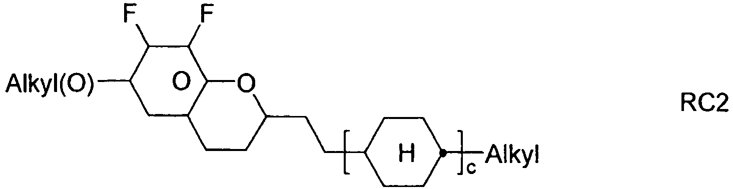 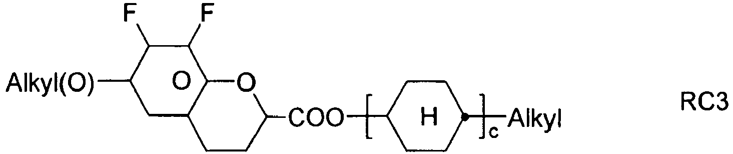 worin Alkyl und Alkyl* jeweils unabhängig voneinander einen geradkettigen Alkylrest mit 1-6 C-Atomen, (O) ein Sauerstoffatom oder eine Einfachbindung, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
   Ganz besonders bevorzugt sind Mischungen enthaltend eine, zwei oder drei Verbindungen der Formel BC-2.
p) FK-Medium, welches zusätzlich eine oder mehrere fluorierte Phenanthrene und/oder Dibenzofurane der folgenden Formeln enthält: 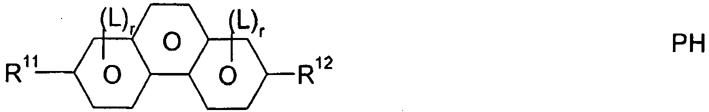 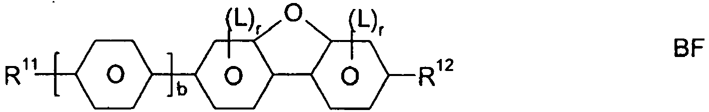 worin R¹¹ und R¹² jeweils unabhängig voneinander die oben angegebenen Bedeutungen besitzen, b 0 oder 1, L F und r 1, 2 oder 3 bedeutet.
   Besonders bevorzugte Verbindungen der Formeln PH und BF sind ausgewählt aus der Gruppe bestehend aus folgenden Unterformeln: 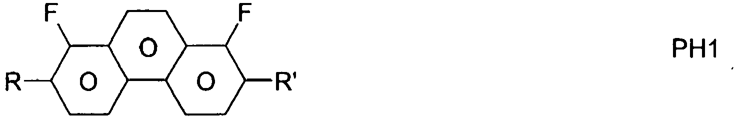 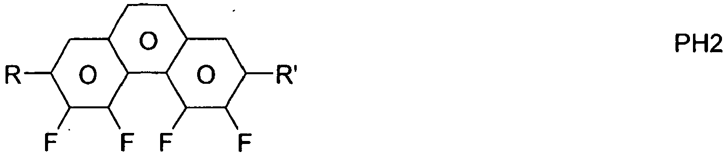 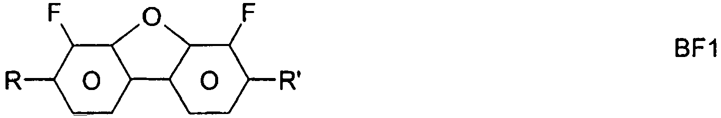 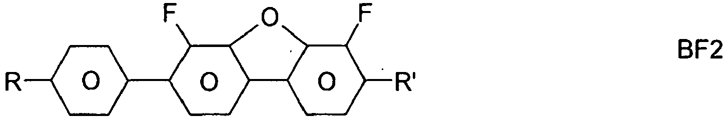 worin R und R' jeweils unabhängig voneinander einen geradkettigen Alkyl- oder Alkoxyrest mit 1-7 C-Atomen bedeuten.
q) FK-Medium, welches außer den erfindungsgemäßen polymerisierbaren Verbindungen, insbesondere der Formel I oder deren Unterformeln, sowie den Comonomeren, keine Verbindungen enthält, die eine endständige Vinyloxygruppe (-O-CH=CH₂) aufweisen.
r) FK-Medium, welches 1 bis 5, vorzugsweise 1, 2 oder 3 polymerisierbare Verbindungen, vorzusgweise ausgewählt aus erfindungsgemäßen polymerisierbare Verbindungen, insbesondere der Formel I oder deren Unterformeln, enthält.
s) FK-Medium, worin der Anteil an polymerisierbaren Verbindungen, insbesondere der Formel I oder deren Unterformeln, im Gesamtgemisch 0,05 bis 5 %, vorzugsweise 0,1 bis 1 % beträgt.
t) FK-Medium, welches 1 bis 8, vorzugsweise 1 bis 5 Verbindungen der Formel CY1, CY2, PY1 und/oder PY2 enthält. Der Anteil dieser Verbindungen im Gesamtgemisch beträgt vorzugsweise 5 bis 60 %, besonders bevorzugt 10 bis 35 %. Der Gehalt dieser einzelnen Verbindungen beträgt vorzugsweise jeweils 2 bis 20 %.
u) FK-Medium, welches 1 bis 8, vorzugsweise 1 bis 5 Verbindungen der Formel CY9, CY10, PY9 und/oder PY1 0 enthält. Der Anteil dieser Verbindungen im Gesamtgemisch beträgt vorzugsweise 5 bis 60 %, besonders bevorzugt 10 bis 35 %. Der Gehalt dieser einzelnen Verbindungen beträgt vorzugsweise jeweils 2 bis 20 %.
v) FK-Medium, welches 1 bis 10, vorzugsweise 1 bis 8 Verbindungen der Formel ZK enthält, insbesondere Verbindungen der Formel ZK1, ZK2 und/oder ZK6. Der Anteil dieser Verbindungen im Gesamtgemisch beträgt vorzugsweise 3 bis 25 %, besonders bevorzugt 5 bis 45 %. Der Gehalt dieser einzelnen Verbindungen beträgt vorzugsweise jeweils 2 bis 20 %.
w) FK-Medium, worin der Anteil an Verbindungen der Formel CY, PY und ZK im Gesamtgemisch mehr als 70 %, vorzugsweise mehr als 80 % beträgt,
x) PSA-VA-Anzeige, worin der pretilt-Winkel vorzugsweise ≤ 85°, besonderes bevorzugt ≤ 80° beträgt.

In einer zweiten bevorzugten Ausführungsform der vorliegenden Erfindung enthält das FK-Medium eine FK-Hostmischung basierend auf Verbindungen mit positiver dielektrischer Anisotropie. Solche FK-Medien eignen sich besonders zur Verwendung in PSA-OCB-, PSA-TN-, PSA-Posi-VA-, PSA-IPS- und PSA-FFS-Anzeigen. Besonders bevorzugte Ausführungsformen für solche FK-Medien werden im Folgenden genannt:

FK-Medium, welches eine oder mehrere Verbindungen ausgewählt aus der Gruppe bestehend aus folgenden Formeln enthält:

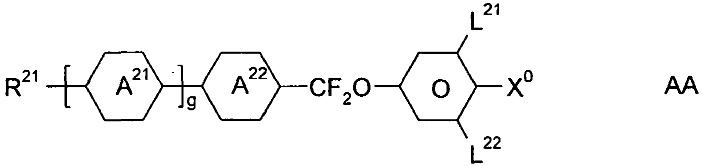

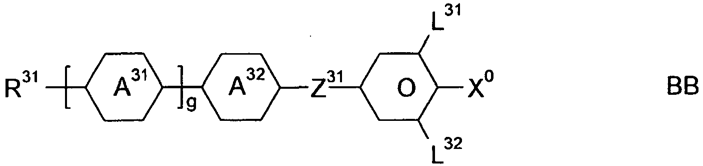

FK-Medium, welches zusätzlich zu den Verbindungen der Formel AA und/ oder BB eine oder mehrere Verbindungen der folgenden Formel enthält:

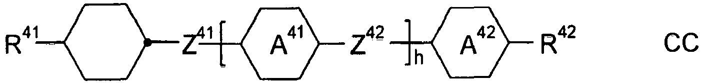

In den Formeln AA, BB und CC besitzen die einzelnen Reste folgende Bedeutung: 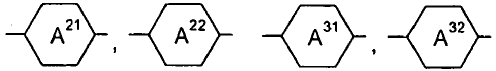 jeweils unabhängig voneinander, und bei jedem Auftreten gleich oder verschieden 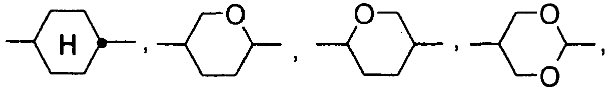 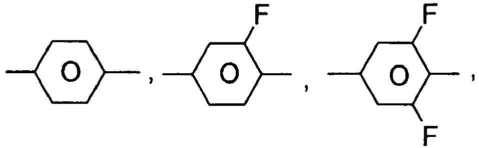 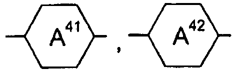 jeweils unabhängig voneinander, und bei jedem Auftreten gleich oder verschieden 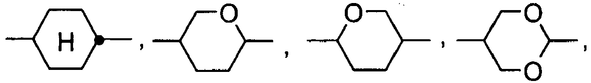 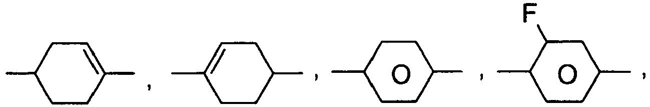
- R²¹, R³¹, R⁴¹, R⁴²: jeweils unabhängig voneinander Alkyl, Alkoxy, Oxaalkyl oder Fluoralkyl mit 1 bis 9 C-Atomen oder Alkenyl mit 2 bis 9 C-Atomen,
- X⁰: F, Cl, halogeniertes Alkyl oder Alkoxy mit 1 bis 6 C-Atomen oder halogeniertes Alkenyl oder Alkenyloxy mit 2 bis 6 C-Atomen,
- Z³¹: -CH₂CH₂-, -CF₂CF₂-, -COO-, *trans-* -CH=CH-, *trans-*-CF=CF-, -CH₂O- oder eine Einfachbindung, vorzugsweise -CH₂CH₂-, -COO-, *trans-* -CH=CH- oder eine Einfachbindung, besonders bevorzugt -COO-, *trans-* -CH=CH- oder eine Einfachbindung,
- Z⁴¹ Z⁴²: -CH₂CH₂-, -COO-, *trans-* -CH=CH-, *trans-* -CF=CF-, - CH₂O-, -CF₂O-, -C≡C- oder eine Einfachbindung, vorzugsweise eine Einfachbindung,
- L²¹, L²², L³¹, L³²: H oder F,
- g: 1, 2 oder 3,
- h: 0, 1, 2 oder 3.

X⁰ bedeutet vorzugsweise F, Cl, CF₃, CHF₂, OCF₃, OCHF₂, OCFHCF₃, OCFHCHF₂, OCFHCHF₂, OCF₂CH₃, OCF₂CHF₂, OCF₂CHF₂, OCF₂CF₂CHF₂, OCF₂CF₂CHF₂, OCFHCF₂CF₃, OCFHCF₂CHF₂, OCF₂CF₂CF₃, OCF₂CF₂CClF₂, OCClFCF₂CF₃ oder CH=CF₂, besonders bevorzugt F oder OCF₃

Die Verbindungen der Formel AA sind vorzugsweise ausgewählt aus der Gruppe bestehend aus den folgenden Formeln: 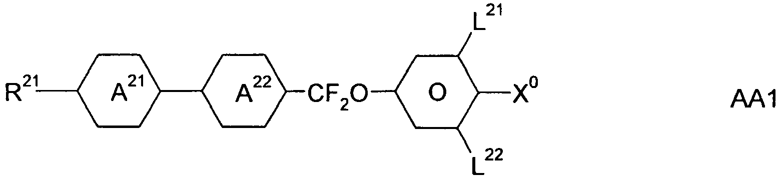 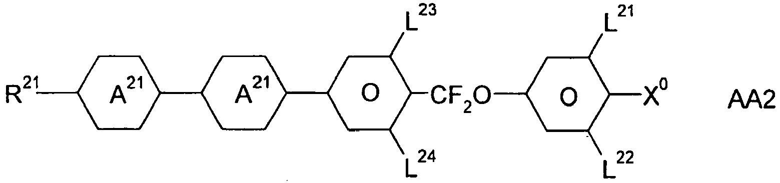 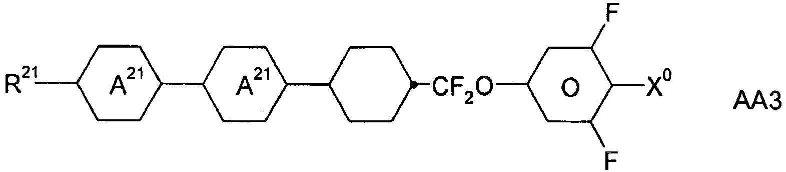 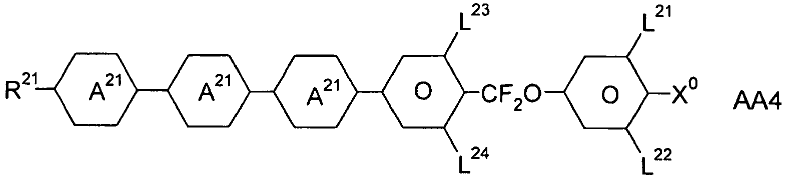 worin A²¹, R²¹, X°, L²¹ und L²² die in Formel AA angegebene Bedeutung besitzen, L²³ und L²⁴ jeweils unabhängig voneinander H oder F bedeuten, und X⁰ vorzugsweise F bedeutet. Besonders bevorzugt sind Verbindungen der Formeln AA1 und AA2.

Besonders bevorzugte Verbindungen der Formel AA1 sind ausgewählt aus der Gruppe bestehend aus folgenden Unterformeln: 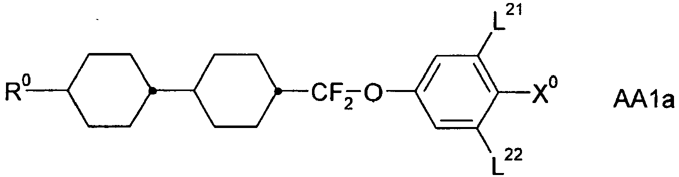 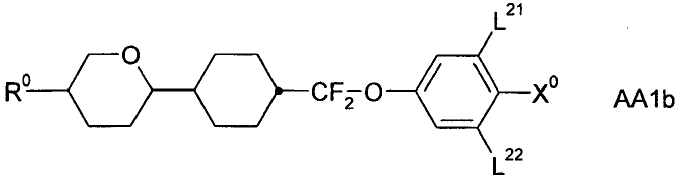 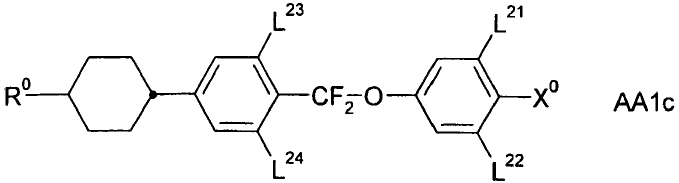 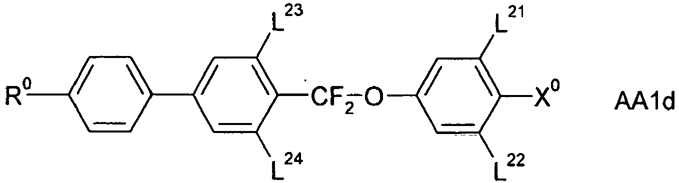 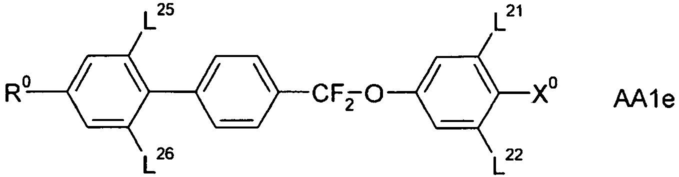 worin R⁰ eine der für R²¹ in Formel AA1 angegebenen Bedeutungen besitzt, X⁰, L²¹ und L²² die in Formel AA1 angegebene Bedeutung besitzen, L²³, L²⁴, L²⁵ und L²⁶ jeweils unabhängig voneinander H oder F bedeuten, und X⁰ vorzugsweise F bedeutet.

Ganz besonders bevorzugte Verbindungen der Formel AA1 sind ausgewählt aus der Gruppe bestehend aus folgenden Unterformeln: 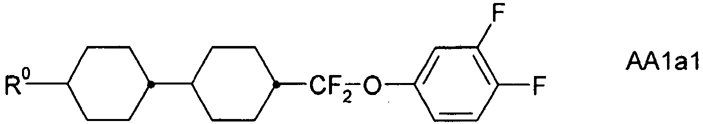 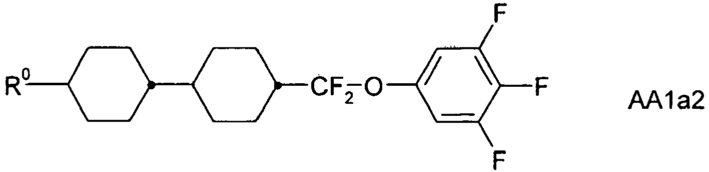 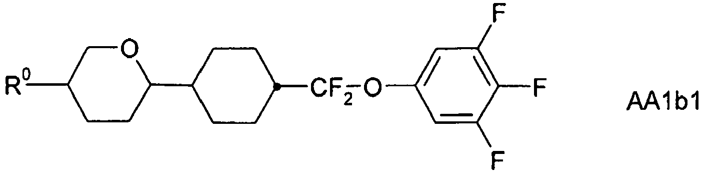 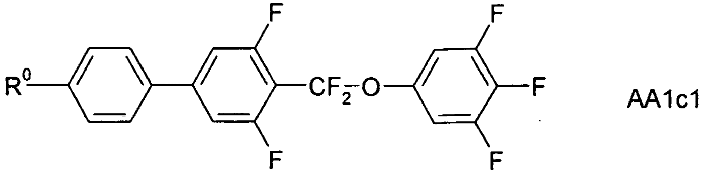 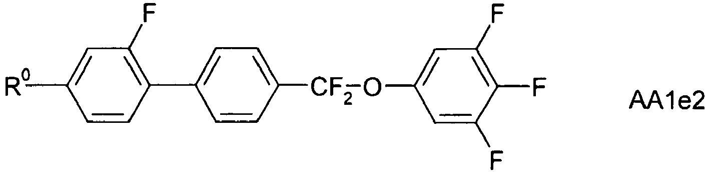 wortn R⁰ die für R²¹ in Formel AA1 angegebene Bedeutung besitzt.

Besonders bevorzugte Verbindungen der Formel AA2 sind ausgewählt aus der Gruppe bestehend aus folgenden Unterformeln: 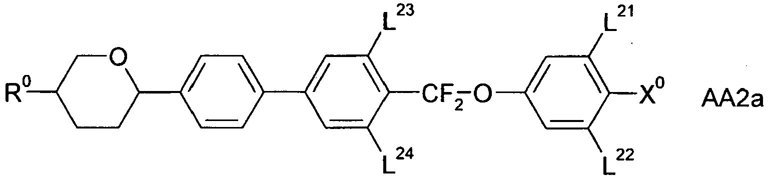 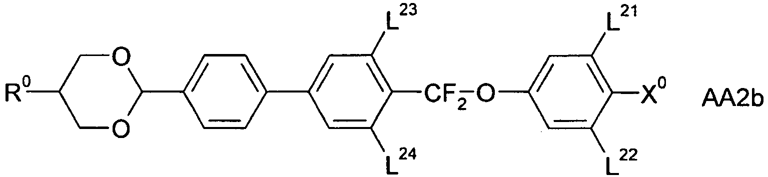 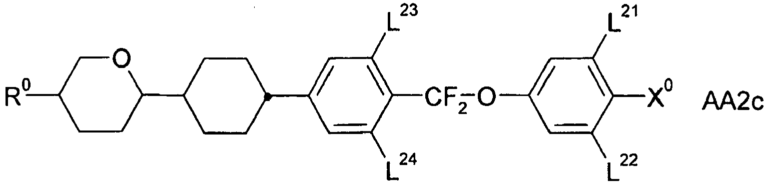 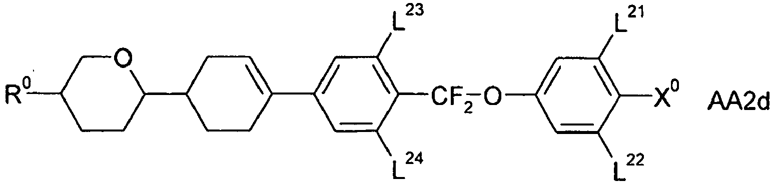 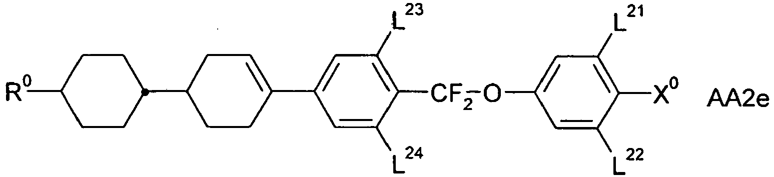 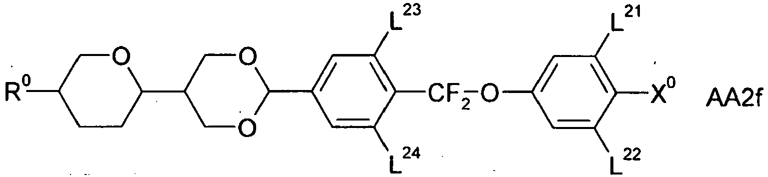 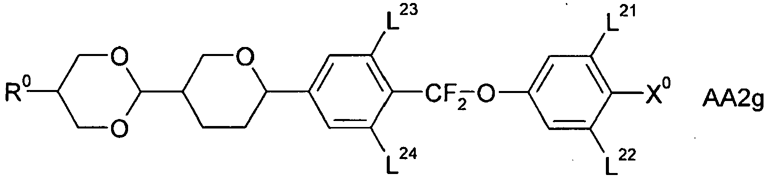 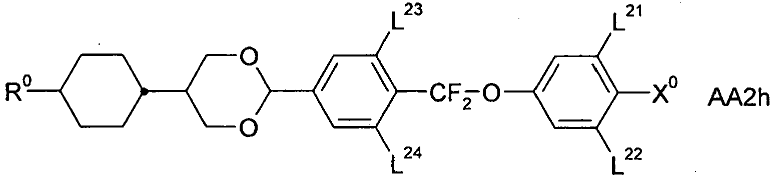 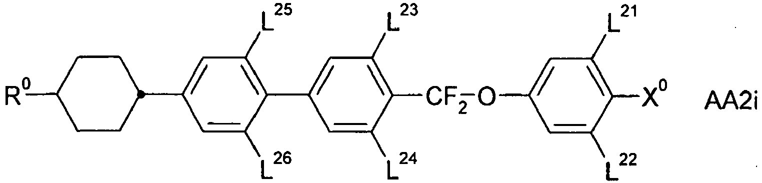 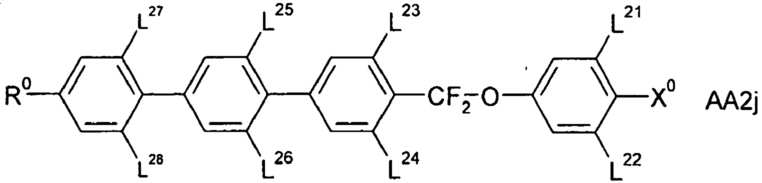 worin R⁰ die für R²¹ in Formel AA1 angegebene Bedeutung besitzt, X⁰, L²¹ und L²² die in Formel AA angegebene Bedeutung besitzen, L²³, L²⁴, L²⁵ und L²⁶ jeweils unabhängig voneinander H oder F bedeuten, und X⁰ vorzugsweise F bedeutet.

Ganz besonders bevorzugte Verbindungen der Formel AA2 sind ausgewählt aus der Gruppe bestehend aus folgenden Unterformeln: 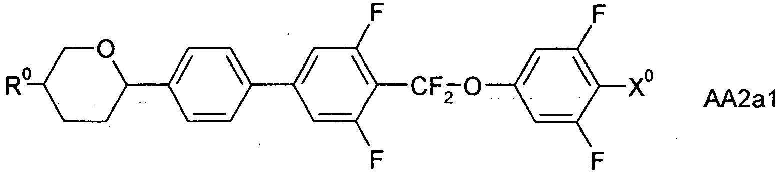 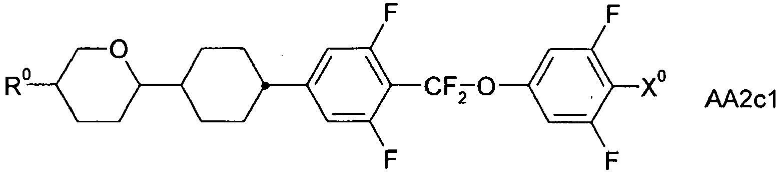 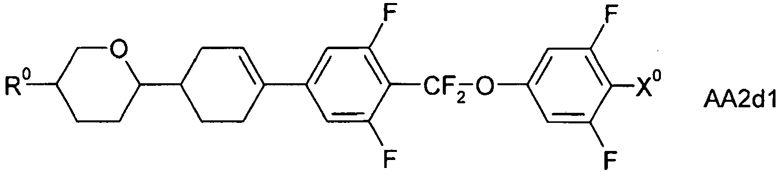 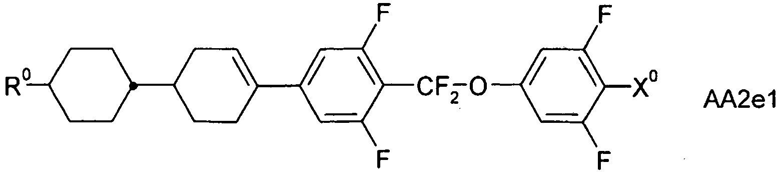 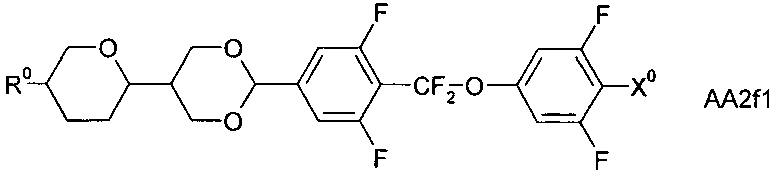 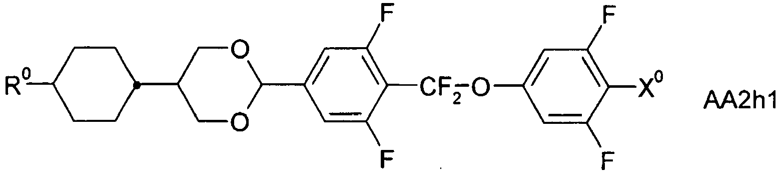 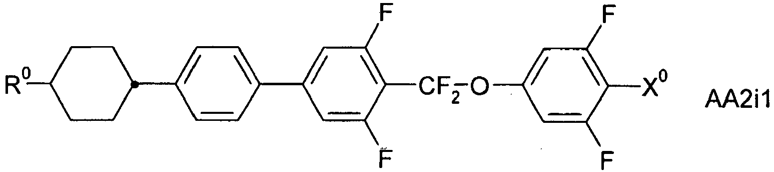 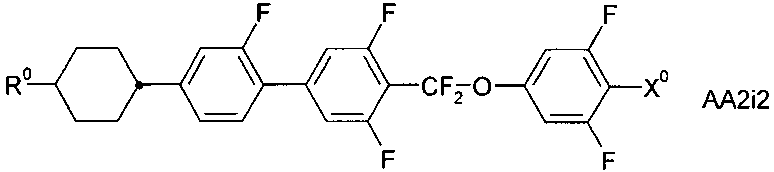 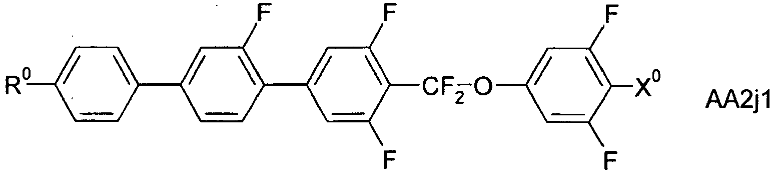 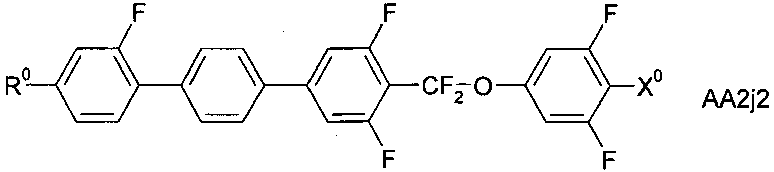 worin R⁰ die für R²¹ in Formel AA1 angegebene Bedeutung besitzt.

Besonders bevorzugte Verbindungen der Formel AA3 sind ausgewählt aus der Gruppe bestehend aus folgenden Unterformeln: 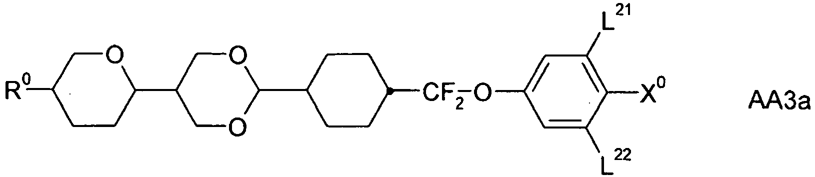 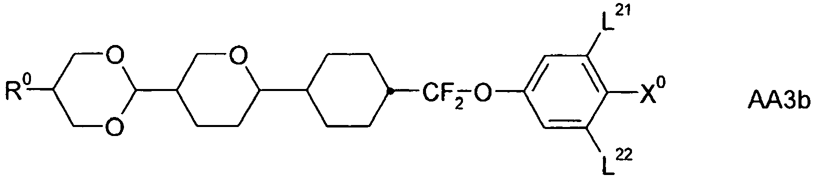 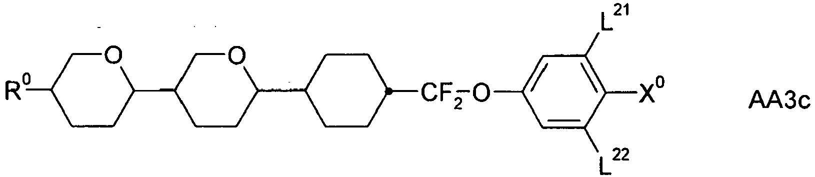 worin R⁰ die für R²¹ in Formel AA1 angegebene Bedeutung besitzt, X⁰, L²¹ und L²² die in Formel AA3 angegebene Bedeutung besitzen, und X⁰ vorzugsweise F bedeutet.

Besonders bevorzugte Verbindungen der Formel AA4 sind ausgewählt aus der Gruppe bestehend aus folgenden Unterformeln: 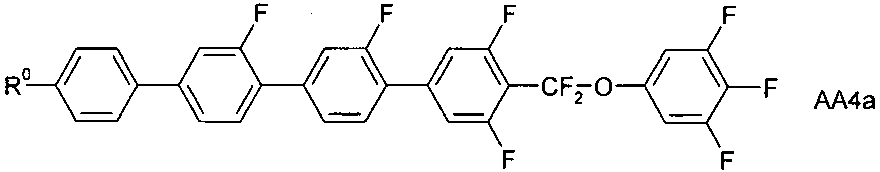 worin R⁰ die für R²¹ in Formel AA1 angegebene Bedeutung besitzt.

Die Verbindungen der Formel BB sind vorzugsweise ausgewählt aus der Gruppe bestehend aus den folgenden Formeln: 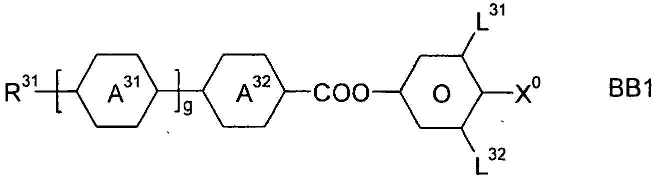 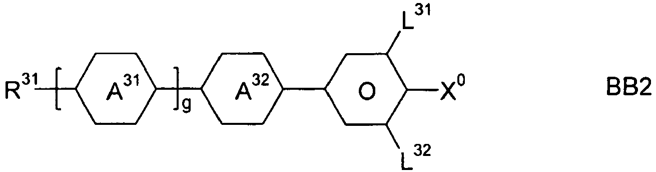 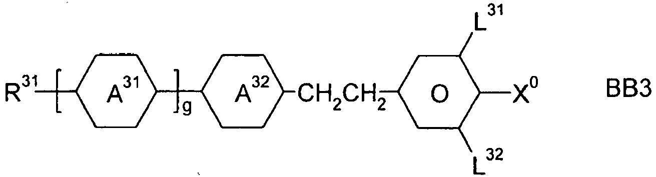 worin A³¹, A³², R³¹, X⁰, L³¹ und L³² die in Formel BB angegebene Bedeutung besitzen, und X⁰ vorzugsweise F bedeutet. besonders bevorzugt sind Verbindungen der Formeln BB1 und BB2.

Besonders bevorzugte Verbindungen der Formel BB1 sind ausgewählt aus der Gruppe bestehend aus folgenden Unterformeln: 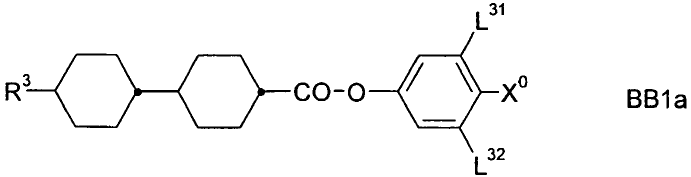 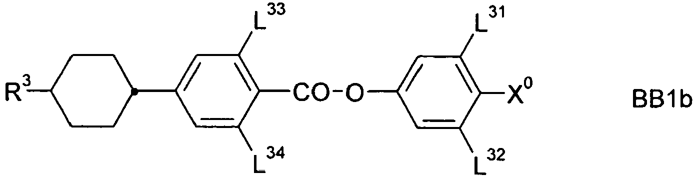 worin R³ die für R³¹ in Formel BB1 angegebene Bedeutung besitzt, X⁰, L³¹ und L³² die in Formel BB1 angegebene Bedeutung besitzen, und X⁰ vorzugsweise F bedeutet.

Ganz besonders bevorzugte Verbindungen der Formel BB1a sind ausgewählt aus der Gruppe bestehend aus folgenden Unterformeln: 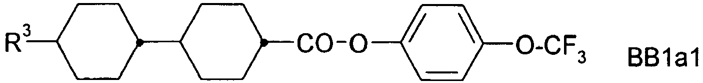 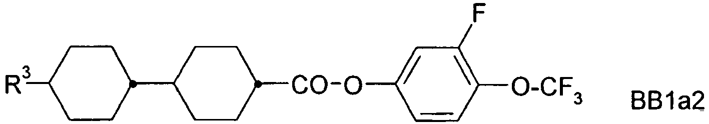 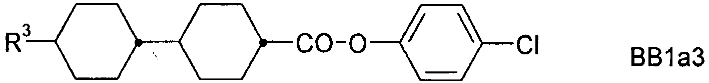 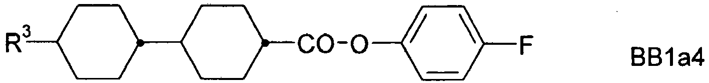 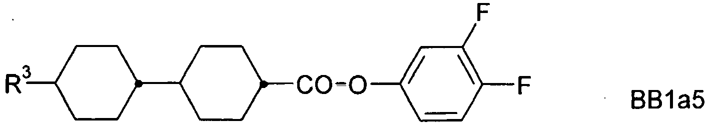 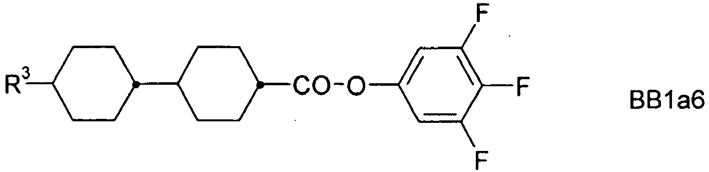 worin R³ die für R³¹ in Formel BB1 angegebene Bedeutung besitzt.

Ganz besonders bevorzugte Verbindungen der Formel BB1 b sind ausgewählt aus der Gruppe bestehend aus folgenden Unterformeln: 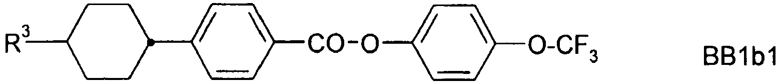 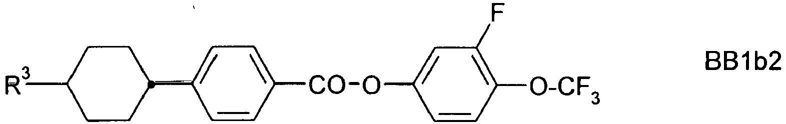 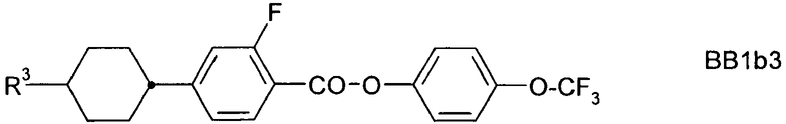 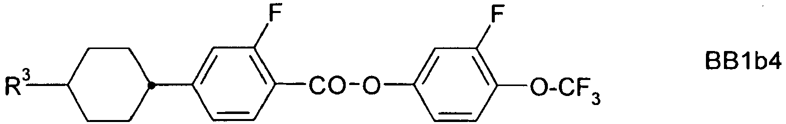 worin R³ die für R³¹ in Formel BB1 angegebene Bedeutung besitzt.

Besonders bevorzugte Verbindungen der Formel BB2 sind ausgewählt aus der Gruppe bestehend aus folgenden Unterformeln: 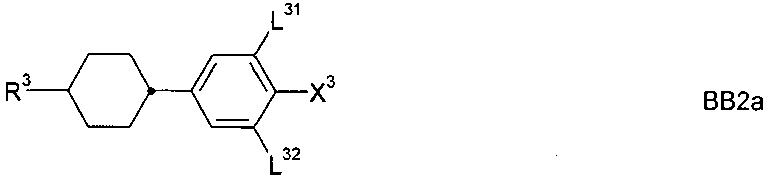 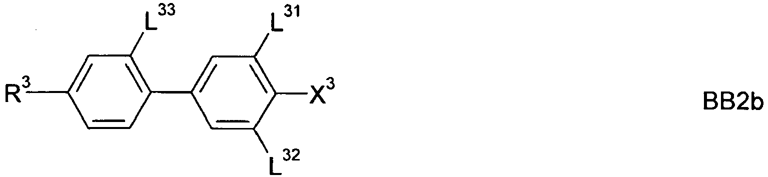 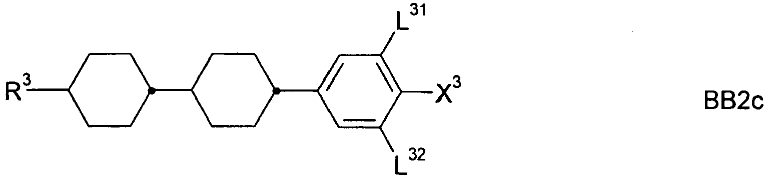 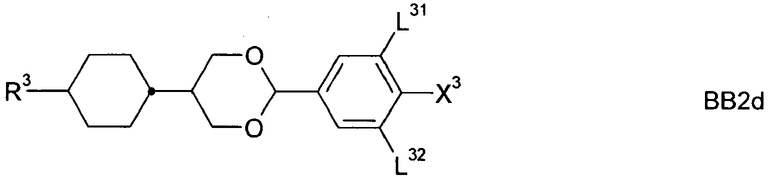 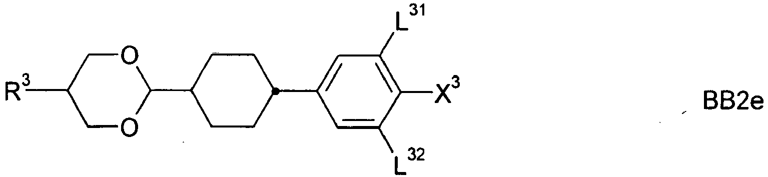 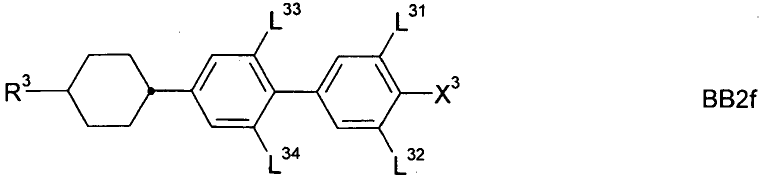 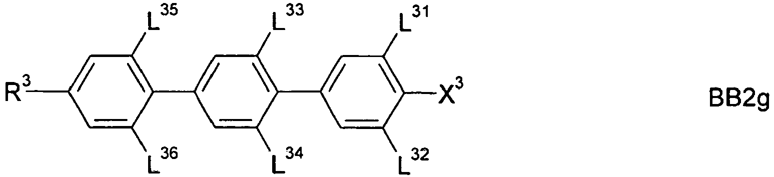 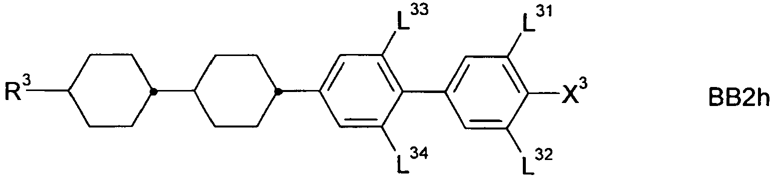 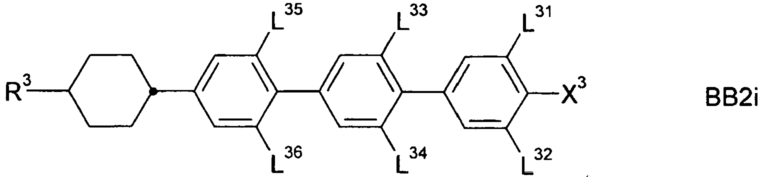 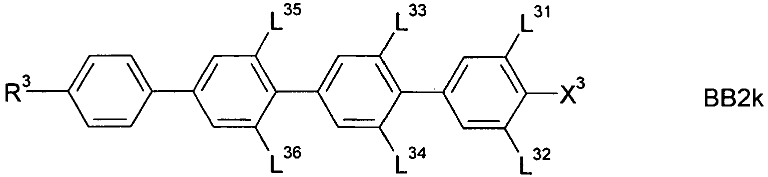 worin R⁰ eine der für R²¹ in Formel BB2 angegebenen Bedeutungen besitzt, X⁰, L³¹ und L³² die in Formel BB2 angegebene Bedeutung besitzen, L³³, L³⁴, L³⁵ und L³⁶ jeweils unabhängig voneinander H oder F bedeuten, und X⁰ vorzugsweise F bedeutet.

Ganz besonders bevorzugte Verbindungen der Formel BB2a sind ausgewählt aus der Gruppe bestehend aus folgenden Unterformeln: 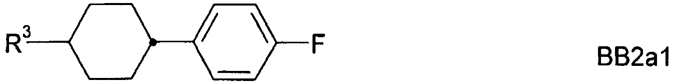 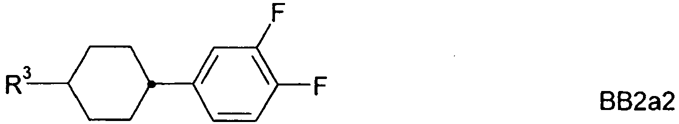 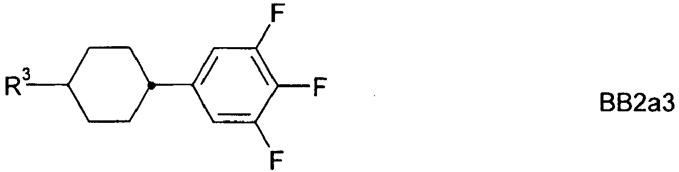 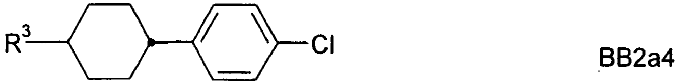 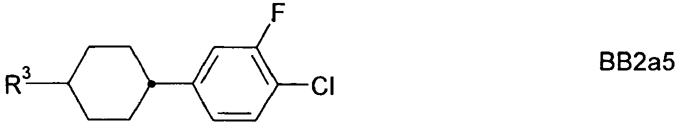 worin R³ die für R³¹ in Formel BB2 angegebene Bedeutung besitzt.

Ganz besonders bevorzugte Verbindungen der Formel BB2b sind ausgewählt aus der Gruppe bestehend aus folgenden Unterformeln: 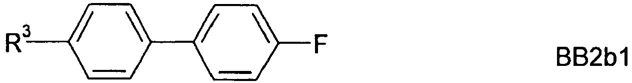 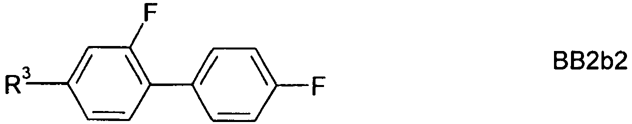 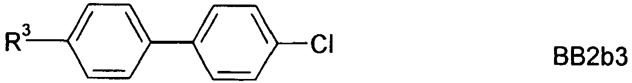 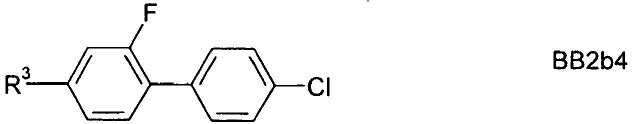 worin R³ die für R³¹ in Formel BB2 angegebene Bedeutung besitzt.

Ganz besonders bevorzugte Verbindungen der Formel BB2c sind ausgewählt aus der Gruppe bestehend aus folgenden Unterformeln: 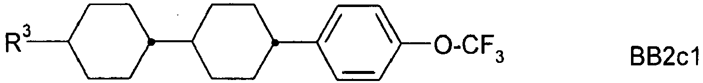 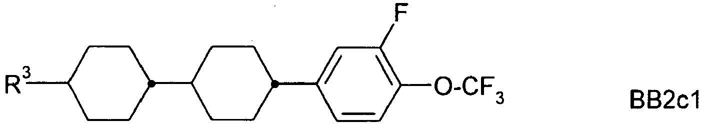 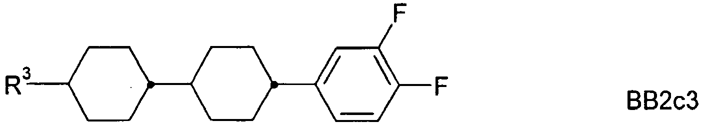 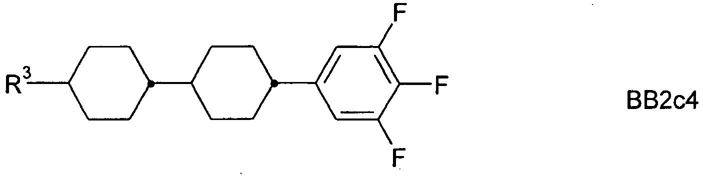 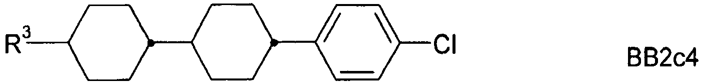 worin R³ die für R³¹ in Formel BB2 angegebene Bedeutung besitzt.

Ganz besonders bevorzugte Verbindungen der Formeln BB2d und BB2e sind ausgewählt aus der Gruppe bestehend aus folgenden Unterformeln: 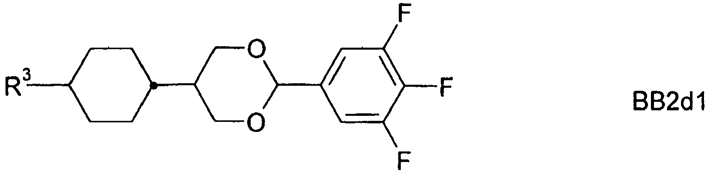 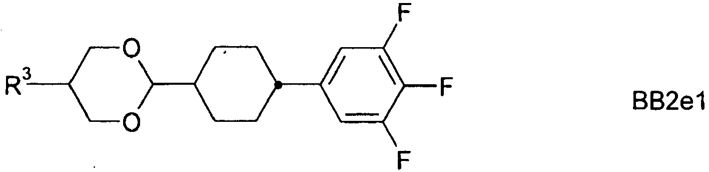 worin R³ die für R³¹ in Formel BB2 angegebene Bedeutung besitzt.

Ganz besonders bevorzugte Verbindungen der Formel BB2f sind ausgewählt aus der Gruppe bestehend aus folgenden Unterformeln: 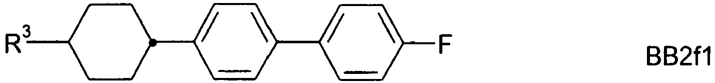 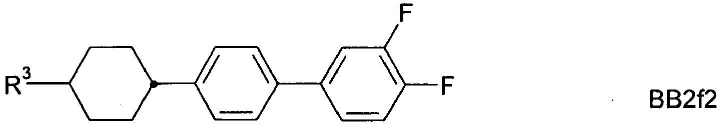 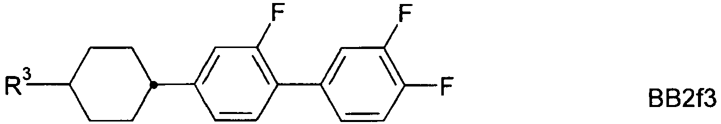 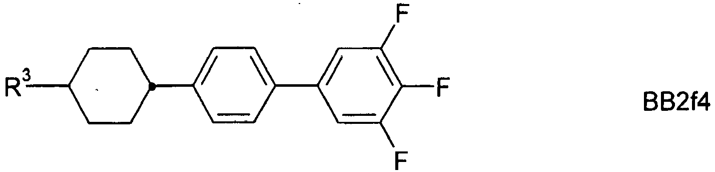 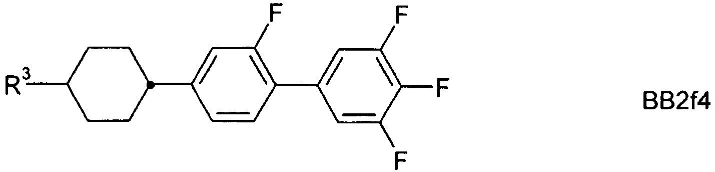 worin R³ die für R³¹ in Formel BB2 angegebene Bedeutung besitzt.

Ganz besonders bevorzugte Verbindungen der Formel BB2g sind ausgewählt aus der Gruppe bestehend aus folgenden Unterformeln: 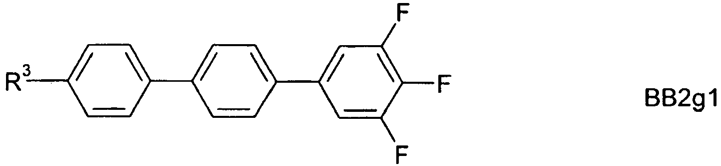 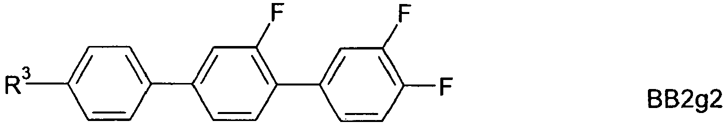 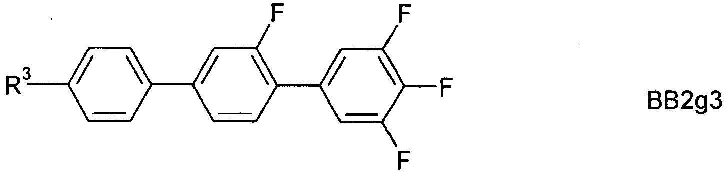 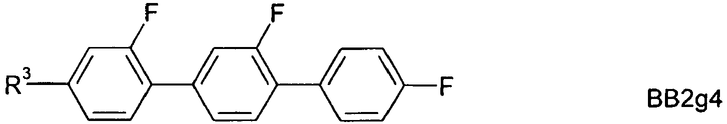 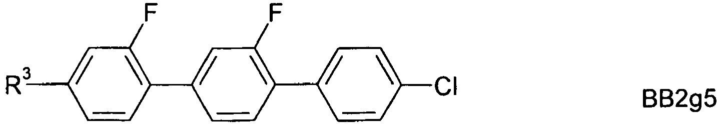 worin R³ die für R³¹ in Formel BB2 angegebene Bedeutung besitzt.

Ganz besonders bevorzugte Verbindungen der Formel BB2h sind ausgewählt aus der Gruppe bestehend aus folgenden Unterformeln: 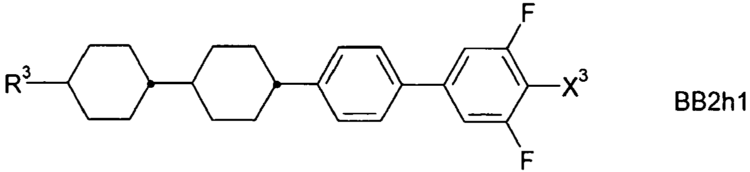 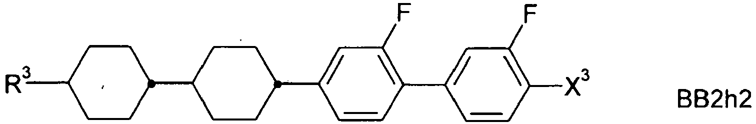 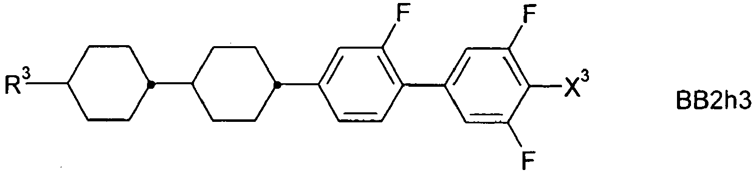 worin R³ die für R³¹ in Formel BB2 angegebene Bedeutung besitzt.

Ganz besonders bevorzugte Verbindungen der Formel BB2i sind ausgewählt aus der Gruppe bestehend aus folgenden Unterformeln: 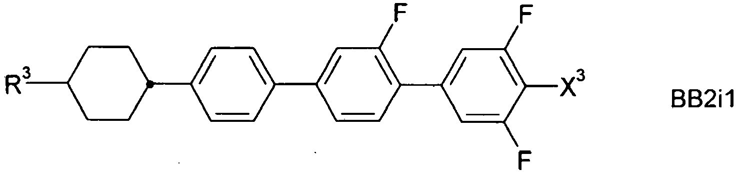 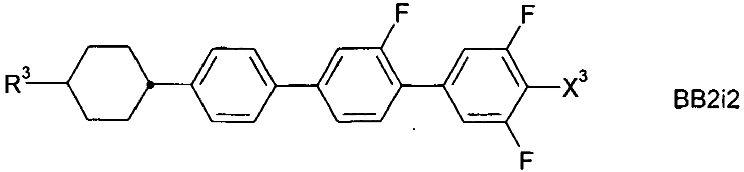 worin R³ die für R³¹ in Formel BB2 angegebene Bedeutung besitzt.

Ganz besonders bevorzugte Verbindungen der Formel BB2k sind ausgewählt aus der Gruppe bestehend aus folgenden Unterformeln: 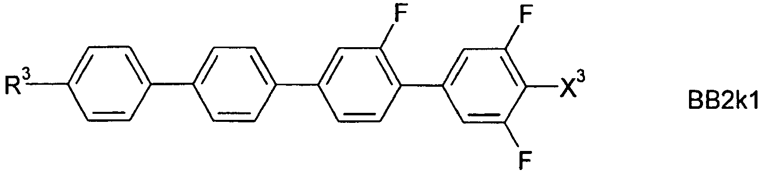 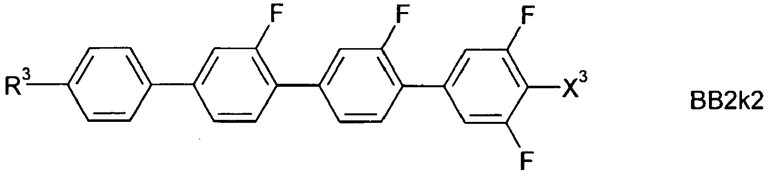 worin R³ die für R³¹ in Formel BB2 angegebene Bedeutung besitzt.

Alternativ zu oder zusätzlich den Verbindungen der Formel BB1 und/oder BB2 kann das FK-Medium auch eine oder mehrere Verbindungen der Formel BB3 wie oben definiert enthalten.

Besonders bevorzugte Verbindungen der Formel BB3 sind ausgewählt aus der Gruppe bestehend aus folgenden Unterformeln: 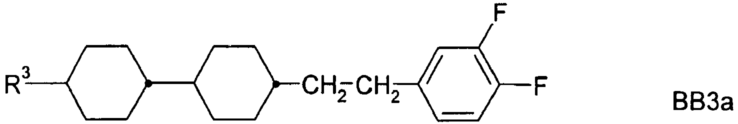 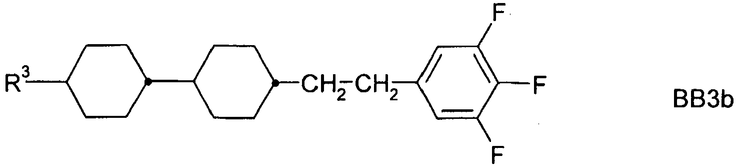 worin R³ die für R³¹ in Formel BB3 angegebene Bedeutung besitzt.

Vorzugsweise enthält das FK-Medium gemäß dieser zweiten bevorzugten Ausführungsform, zusätzlich zu den Verbindungen der Formel AA und/oder BB, eine oder mehrere dielektrisch neutrale Verbindungen mit einer dielektrischen Anisotropie von -1.5 bis +3, welche ausgewählt sind aus der Gruppe bestehend Verbindungen der Formel CC wie oben definiert.

Besonders bevorzugte Verbindungen der Formel CC sind ausgewählt aus der Gruppe bestehend aus folgenden Unterformeln: 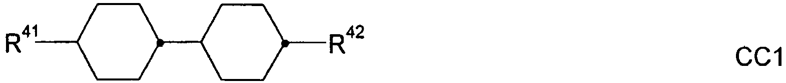 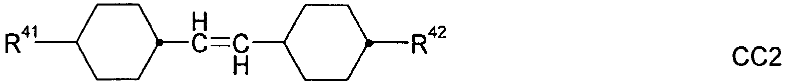 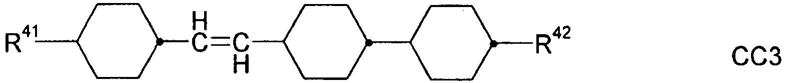 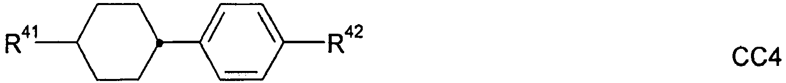 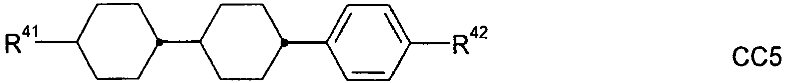 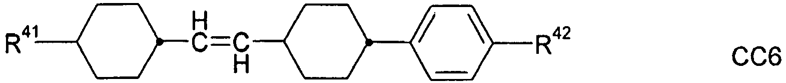 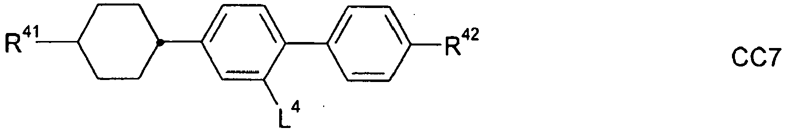 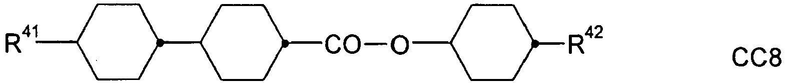 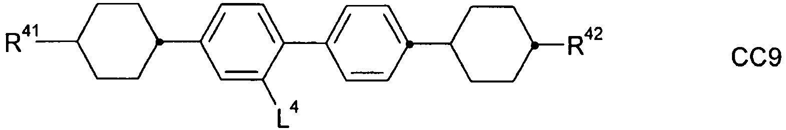 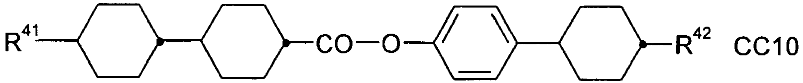 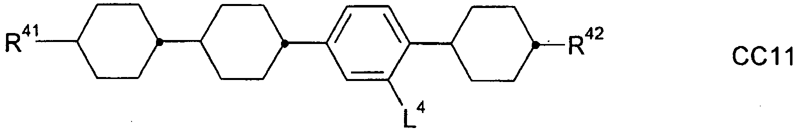 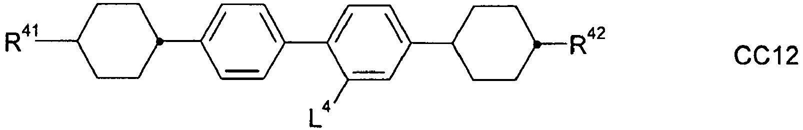 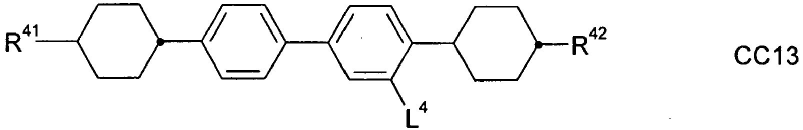 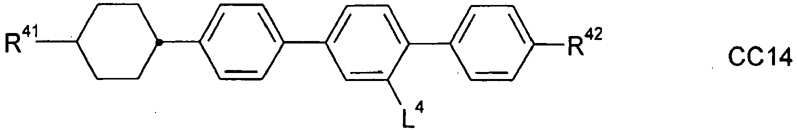 worin R⁴¹ und R⁴² die in Formel CC angegebene Bedeutung besitzen, und vorzugsweise jeweils unabhängig voneinander Alkyl, Alkoxy, fluoriertes Alkyl oder fluorertes Alkoxy mit 1 bis 7 C-Atomen, oder Alkenyl, Alkenyloxy, Alkoxyalkyl oder fluoriertes Alkenyl mit 2 bis 7 C-Atomen, und L⁴ H oder F bedeuten.

Vorzugsweise enthält das FK-Medium gemäß dieser zweiten bevorzugten Ausführungsform, zusätzlich oder alternativ zu den dielektrisch neutralen Verbindungen der Formel CC, eine oder mehrere dielektrisch neutrale Verbindungen mit einer dielektrischen Anisotropie von -1.5 bis +3, welche ausgewählt sind aus der Gruppe bestehend aus Verbindungen der Formel DD. 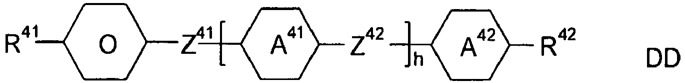 worin A⁴¹, A⁴², Z⁴¹, Z⁴², R⁴¹, R⁴² und h die in Formel CC angegebene Bedeutung besitzen.

Besonders bevorzugte Verbindungen der Formel DD sind ausgewählt aus der Gruppe bestehend aus folgenden Unterformeln: 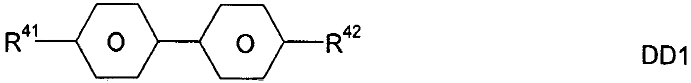 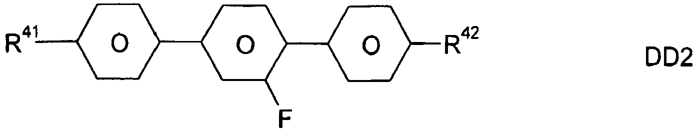 worin R⁴¹ und R⁴² die in Formel DD angegebene Bedeutung besitzen und R⁴¹ vorzugsweise Alkyl bedeutet, und in Formel DD1 R⁴² vorzugsweise Alkenyl, besonders bevorzugt -(CH₂)₂-CH=CH-CH₃ bedeutet, und in Formel DD2 R⁴² vorzugsweise Alkyl, -(CH₂)₂-CH=CH₂ oder -(CH₂)₂-CH=CH-CH₃ bedeutet.

Die Konzentration der Verbindungen der Formel AA und BB im erfindungsgemäßen FK-Medium beträgt vorzugsweise von 2% bis 60%, besonders bevorzugt von 3% bis 35%, ganz besonders bevorzugt von 4bis 30% in der Gesamtmischung.

Die Konzentration der Verbindungen der Formel CC und DD im erfindungsgemäßen FK-Medium beträgt vorzugsweise von 2% bis 70%, insbesondere von 5% bis 65%, besonders bevorzugt von 10% bis 60%, und ganz besonders bevorzugt von 10%, vorzugsweise 15%, bis 55% in der Gesamtmischung.

Die Kombination von niedermolekularen Verbindungen als Bestandteil der FK-Hostmischung gemäß den oben genannten bevorzugten Ausführungsformen mit den oben beschriebenen polymerisierten Verbindungen bewirkt in den erfindungsgemäßen FK-Medien niedrige Schwellenspannungen, niedrige Rotationsviskositäten und sehr gute Tieftemperaturstabilitäten bei gleichbleibend hohen Klärpunkten und hohen HR-Werten, und erlaubt die schnelle Einstellung eines besonders niedrigen pretilt-Winkels in PSA-Anzeigen. Insbesondere zeigen die FK-Medien in PSA-Anzeigen im Vergleich zu den Medien aus dem Stand der Technik deutlich verringerte Schaltzeiten, insbesondere auch der Graustufenschaltzeiten.

Vorzugsweise weist die Flüssigkristallmischung einen nematischen Phasenbereich von mindestens 80 K, besonders bevorzugt von mindestens 100 K, und eine Rotationsviskosität von nicht mehr als 250, vorzugsweise nicht mehr als 200 mPas, bei 20°C auf.

In den erfindungsgemäßen Anzeigen des VA-Typs sind die Moleküle in der Schicht des FK-Mediums im ausgeschalteten Zustand senkrecht zu den Elektrodenflächen (homöotrop) oder gekippt homöotrop (engl. "tilted") orientiert. Bei Anlegen einer elektrischen Spannung an die Elektroden findet eine Umorientierung der FK-Moleküle mit den Moleküllängsachsen parallel zu den Elektrodenflächen statt.

Erfindungsgemäße FK-Medien der ersten bevorzugten Ausführungsform, insbesondere solche zur Verwendung in Anzeigen des PSA-VA-Typs, weisen eine negative dielektrische Anisotropie Δε auf, vorzugsweise von etwa -0,5 bis -10, insbesondere von etwa -2,5 bis -7,5 bei 20°C und 1 kHz.

Die Doppelbrechung Δn in erfindungsgemäßen FK-Medien der ersten bevorzugten Ausführungsform, insbesondere in solchen zur Verwendung in Anzeigen des PSA-VA-Typs, liegt vorzugsweise unter 0,16, besonders bevorzugt zwischen 0,06 und 0,14, insbesondere zwischen 0,07 und 0,12.

In den erfindungsgemäßen Anzeigen des OCB-Typs sind die Moleküle in der Schicht des FK-Mediums eine "bend"-Orientierung auf. Bei Anlegen einer elektrischen Spannung findet eine Umorientierung der FK-Moleküle mit den Moleküllängsachsen senkrecht zu den Elektrodenflächen statt.

Erfindungsgemäße FK-Medien zur Verwendung in Anzeigen des PSA-OCB-Typs sind vorzugsweise solche mit einer positiven dielektrischen Anisotropie Δε gemäß der zweiten bevorzugten Ausführungsform, und besitzen vorzugsweise eine dielektrische Anisotropie Δε von etwa +4 bis +17 bei 20°C und 1 kHz.

Die Doppelbrechung Δn in erfindungsgemäßen FK-Medien der zweiten bevorzugten Ausführungsform zur Verwendung in Anzeigen des OCB-Typs liegt vorzugsweise zwischen 0,14 und 0,22, insbesondere zwischen 0,16 und 0,22.

Erfindungsgemäße FK-Medien der zweiten bevorzugten Ausführungsform, insbesondere solche zur Verwendung in Anzeigen des PSA-TN-, PSAposi-VA-, PSA-IPS- und PSA-FFS-Typs, weisen eine positive dielektrische Anisotropie Δε auf, vorzugsweise von +2 bis +30, besonders bevorzugt von +2 bis +17, ganz besonders bevorzugt von +3 bis +15, bei 20°C und 1 kHz.

Die Doppelbrechung Δn in erfindungsgemäßen FK-Medien der zweiten bevorzugten Ausführungsform, insbesondere in solchen zur Verwendung in Anzeigen des PSA-TN-, PSA-IPS-, und PSA-FFS-Typs, liegt vorzugsweise zwischen 0,07 und 0,15, insbesondere zwischen 0,08 und 0,13.

Die erfindungsgemäßen FK-Medien können auch weitere, dem Fachmann bekannte und in der Literatur beschriebene Zusätze oder Additive enthalten, wie beispielsweise Polymerisationsinitiatoren, Inhibitoren, Stabilisatoren, oberflächenaktive Substanzen oder chirale Dotierstoffe. Diese können polymerisierbar oder unpolymerisierbar sein. Polymerisierbare Additive werden dementsprechend der polymerisierbaren Komponente oder Komponente A) zugerechnet. Unpolymerisierbare Additive werden dementsprechend der unpolymerisierbaren Komponente oder Komponente B) zugerechnet.

Die FK.Medien können beispielsweise einen oder mehrere chirale Dotierstoffe enthalten, vorzusgweise solche ausgewählt aus der Gruppe bestehend aus Verbindungen der nachfolgenden Tabelle B.

Ferner können den FK.Medien beispielsweise 0 bis 15 Gew.-% pleochroitische Farbstoffe zugesetzt werden, ferner Nanopartikel, Leitsalze, vorzugsweise Ethyl-dimethyldodecylammonium-4-hexoxybenzoat, Tetrabutylammoniumtetraphenylborat oder Komplexsalze von Kronenethern (vgl. z.B. Haller et al., Mol. Cryst. Liq. Cryst. 24, 249-258 (1973)) zur Verbesserung der Leitfähigkeit, oder Substanzen zur Veränderung der dielektrischen Anisotropie, der Viskosität und/oder der Orientierung der nematischen Phasen. Derartige Substanzen sind z.B. in DE-A 22 09 127, 22 40 864, 23 21 632, 23 38 281, 24 50 088, 26 37 430 und 28 53 728 beschrieben.

Die einzelnen Komponenten der bevorzugten Ausführungsformen a)-z) der erfindungsgemäßen FK-Medien sind entweder bekannt, oder ihre Herstellungsweisen sind für den einschlägigen Fachmann aus dem Stand der Technik ohne weiteres abzuleiten, da sie auf in der Literatur beschriebenen Standardverfahren basieren. Entsprechende Verbindungen der Formel CY werden beispielsweise in EP-A-0 364 538 beschrieben. Entsprechende Verbindungen der Formel ZK werden beispielsweise in DE-A-26 36 684 und DE-A-33 21 373 beschrieben.

Die Herstellung der erfindungsgemäß verwendbaren FK-Medien erfolgt in an sich üblicher Weise, beispielsweise indem man eine oder mehrere der oben genannten Verbindungen mit einer oder mehreren polymerisierbaren Verbindungen wie oben definiert, und ggf. mit weiteren flüssigkristallinen Verbindungen und/oder Additiven mischt. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Das Verfahren zur Herstellung der erfindungsgemäßen FK-Medien ist ein weiterer Gegenstand der Erfindung.

Es versteht sich für den Fachmann von selbst, daß die erfindungsgemäßen FK-Medien auch Verbindungen enthalten können, worin beispielsweise H, N, O, Cl, F durch die entsprechenden Isotope ersetzt sind.

Der Aufbau der erfindungsgemäßen FK-Anzeigen entspricht der für PSA-Anzeigen üblichen Geometrie, wie er im eingangs zitierten Stand der Technik beschrieben ist. Es sind Geometrien ohne Protrusions bevorzugt, insbesondere diejenigen, bei denen darüber hinaus die Elektrode auf der Colour Filter-Seite unstrukturiert ist und lediglich die Elektrode auf der TFT-Seite Schlitze aufweist. Besonders geeignete und bevorzugte Elektrodenstrukturen für PSA-VA-Anzeigen sind beispielsweise in US 2006/0066793 A1 beschrieben.

Die folgenden Beispiele erläutern die vorliegende Erfindung ohne sie zu begrenzen. Sie zeigen dem Fachmann jedoch bevorzugte Mischungskonzepte mit bevorzugt einzusetzenden Verbindungen und deren jeweiligen Konzentrationen sowie deren Kombinationen miteinander. Außerdem illustrieren die Beispiele, welche Eigenschaften und Eigenschftskombinationen zugänglich sind.

Folgende Abkürzungen werden verwendet:
(n, m, z: jeweils unabhängig voneinander 1, 2, 3, 4, 5 oder 6)

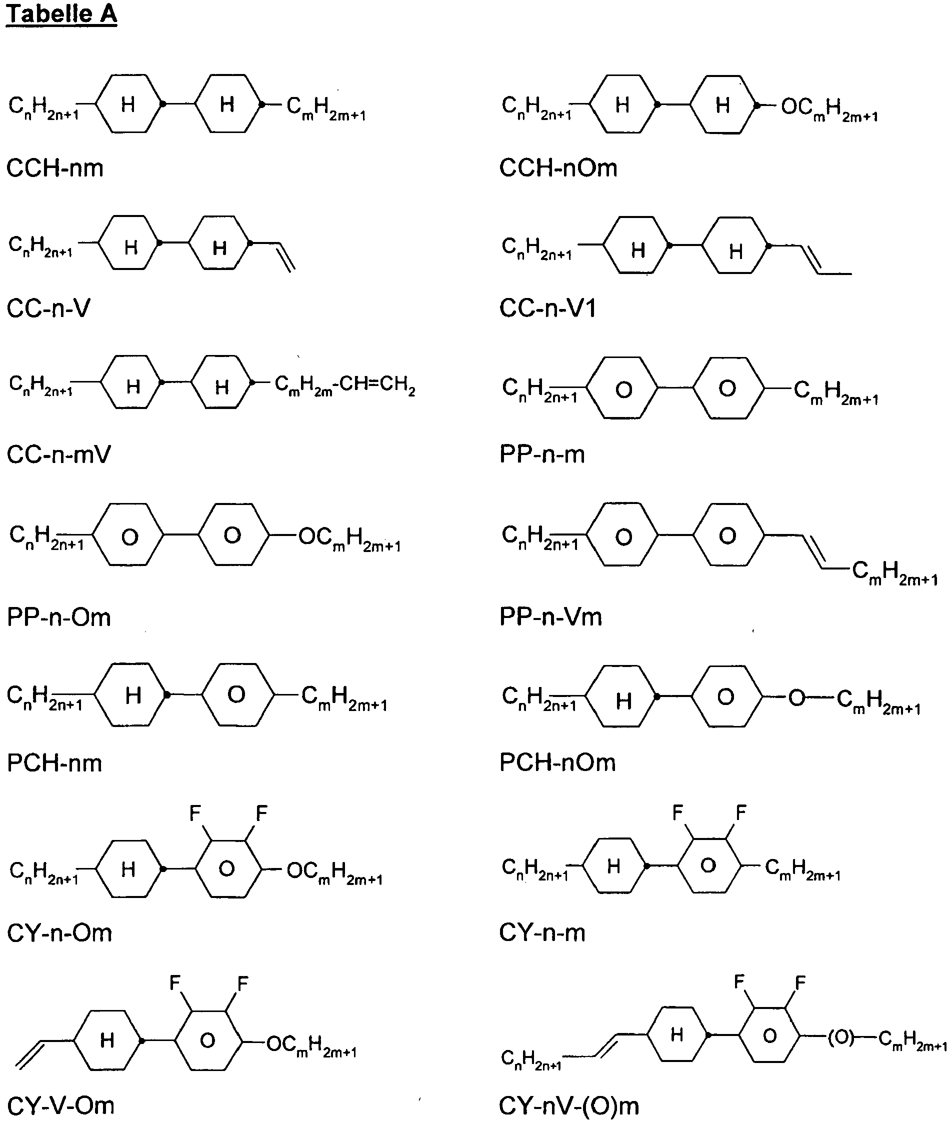

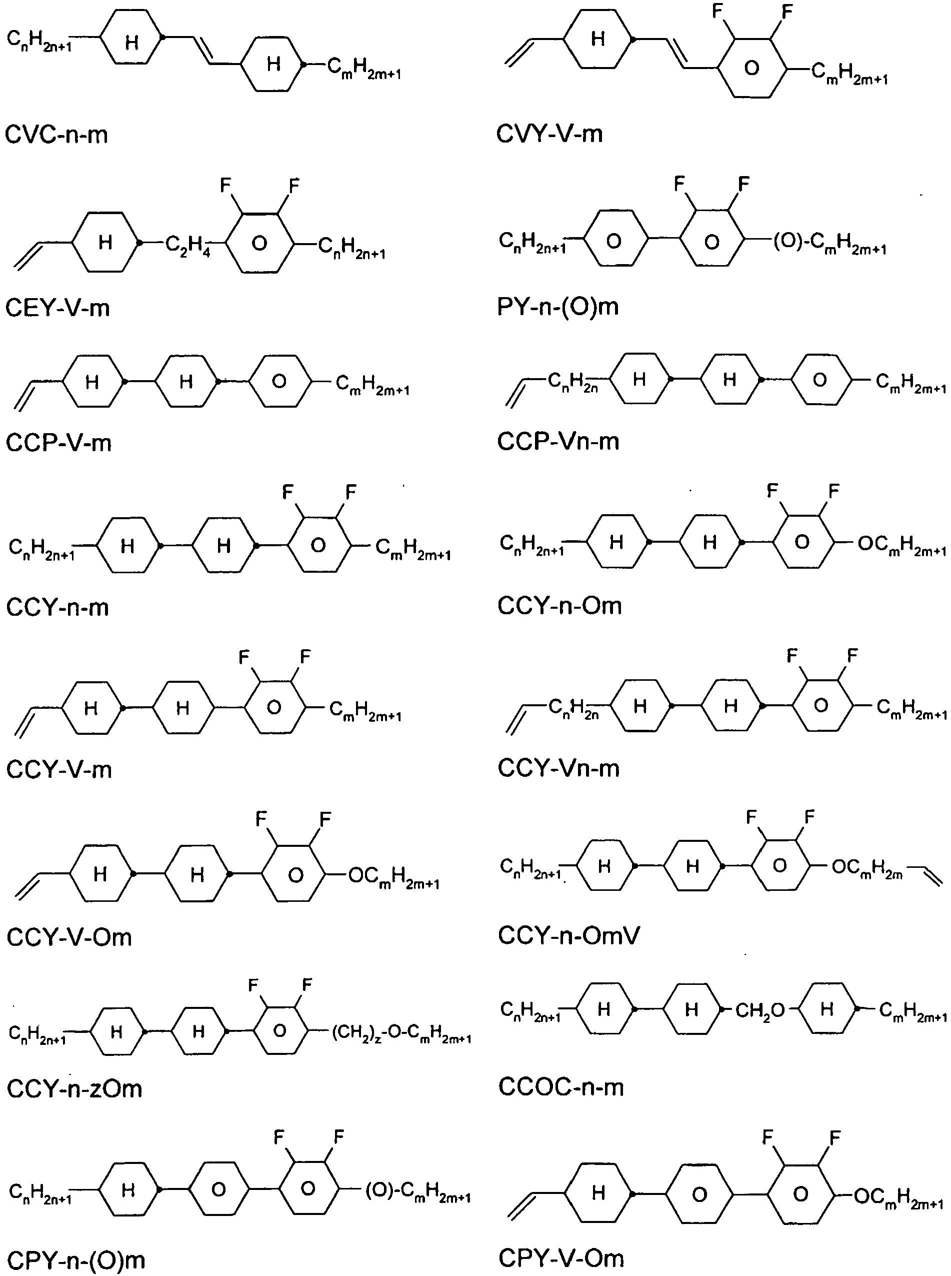

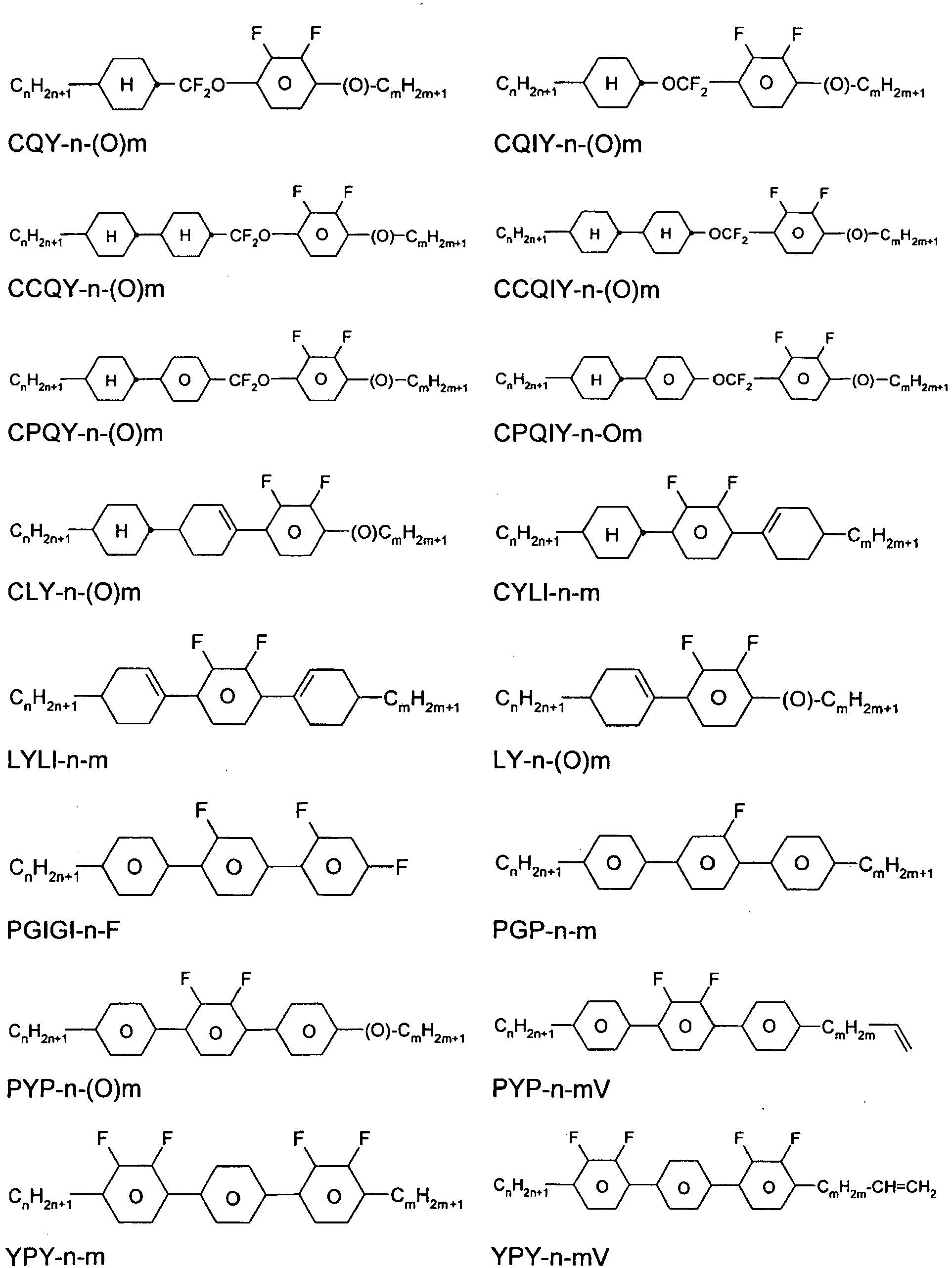

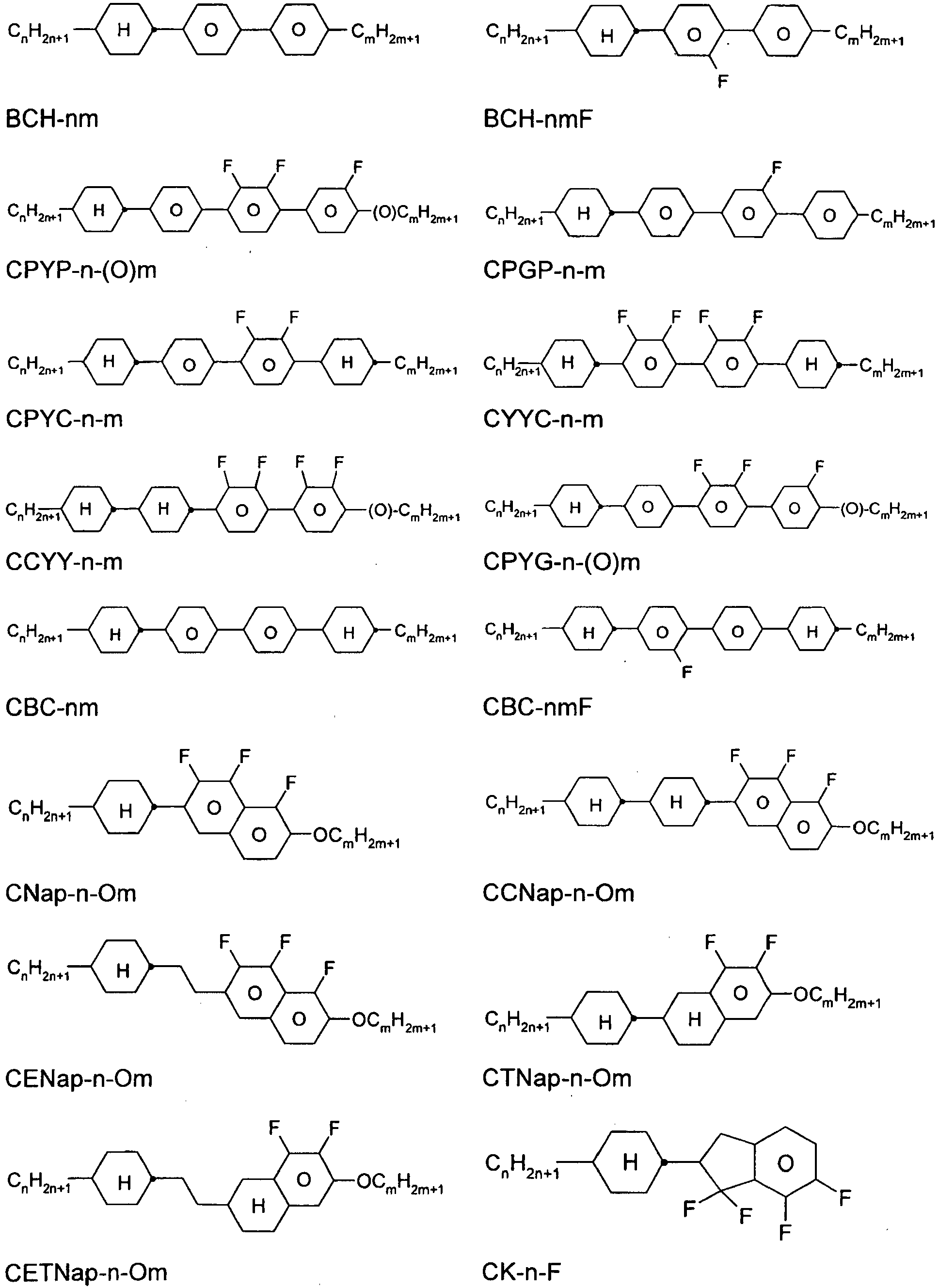

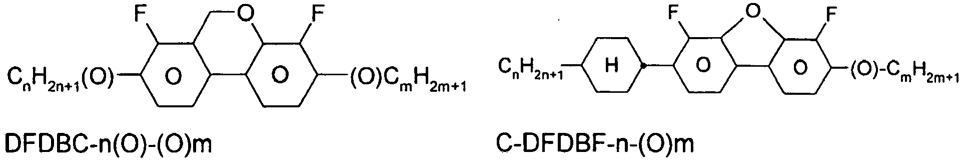

In einer bevorzugten Ausführungsform der vorliegenden Erfindung enthalten die erfindungsgemäßen FK-Medien eine oder mehrere Verbindungen ausgewählt aus der Guppe bestehend aus Verbindungen der Tabelle A.

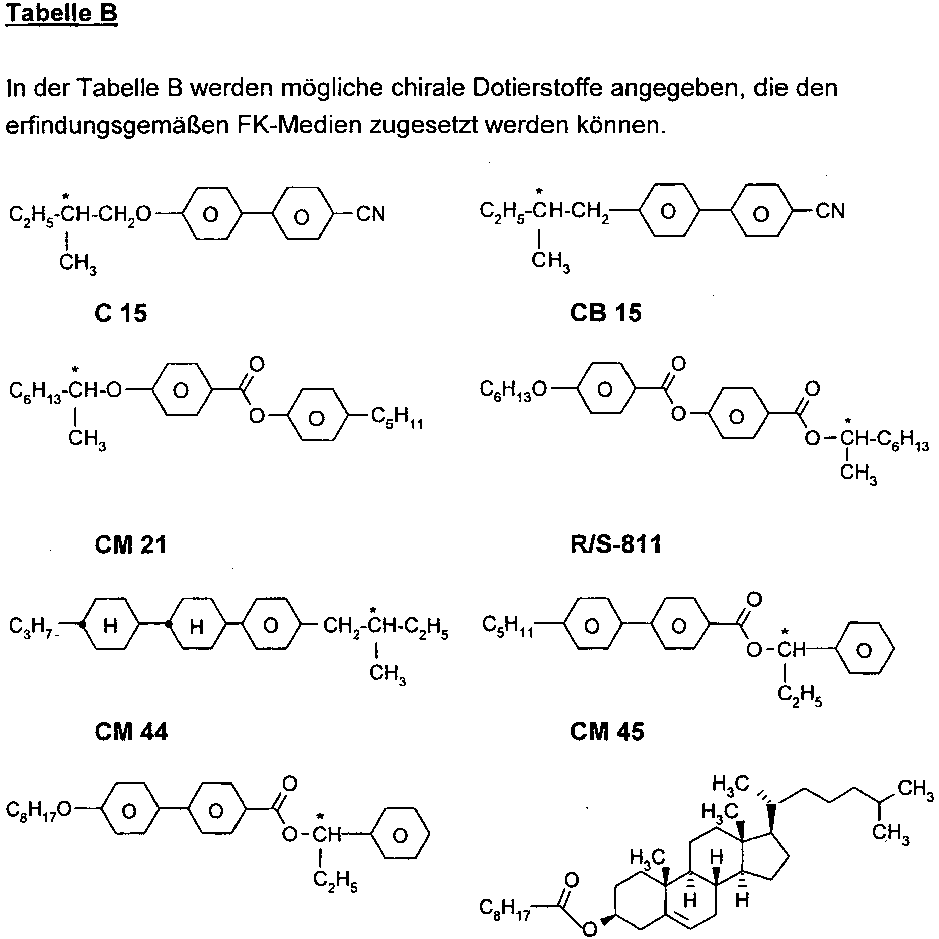

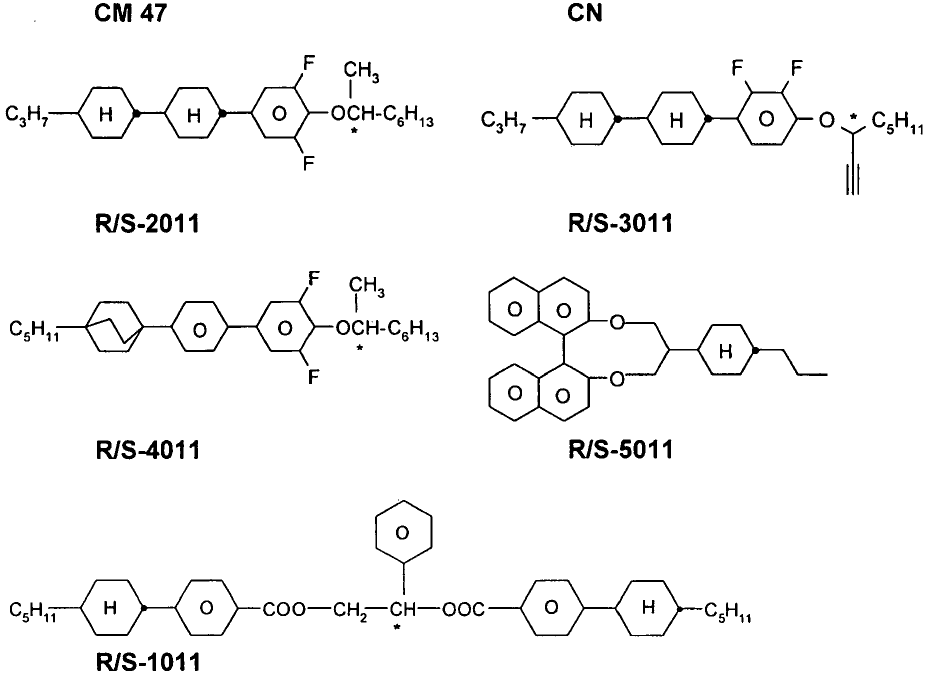

Vorzugsweise enthalten die FK-Medien 0 bis 10 Gew.%, insbesondere 0,01 bis 5 Gew.%, besonders bevorzugt 0,1 bis 3 Gew.% an Dotierstoffen. Vorzugsweise enthalten die FK-Medien einen oder mehrere Dotierstoffe ausgewählt aus der Guppe bestehend aus Verbindungen der Tabelle B.

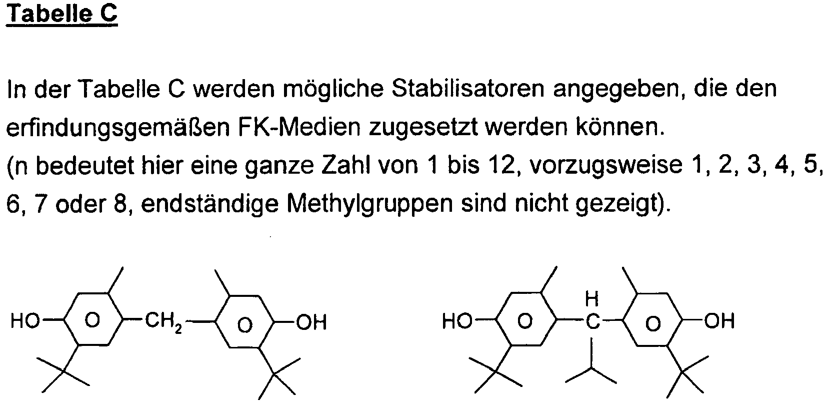

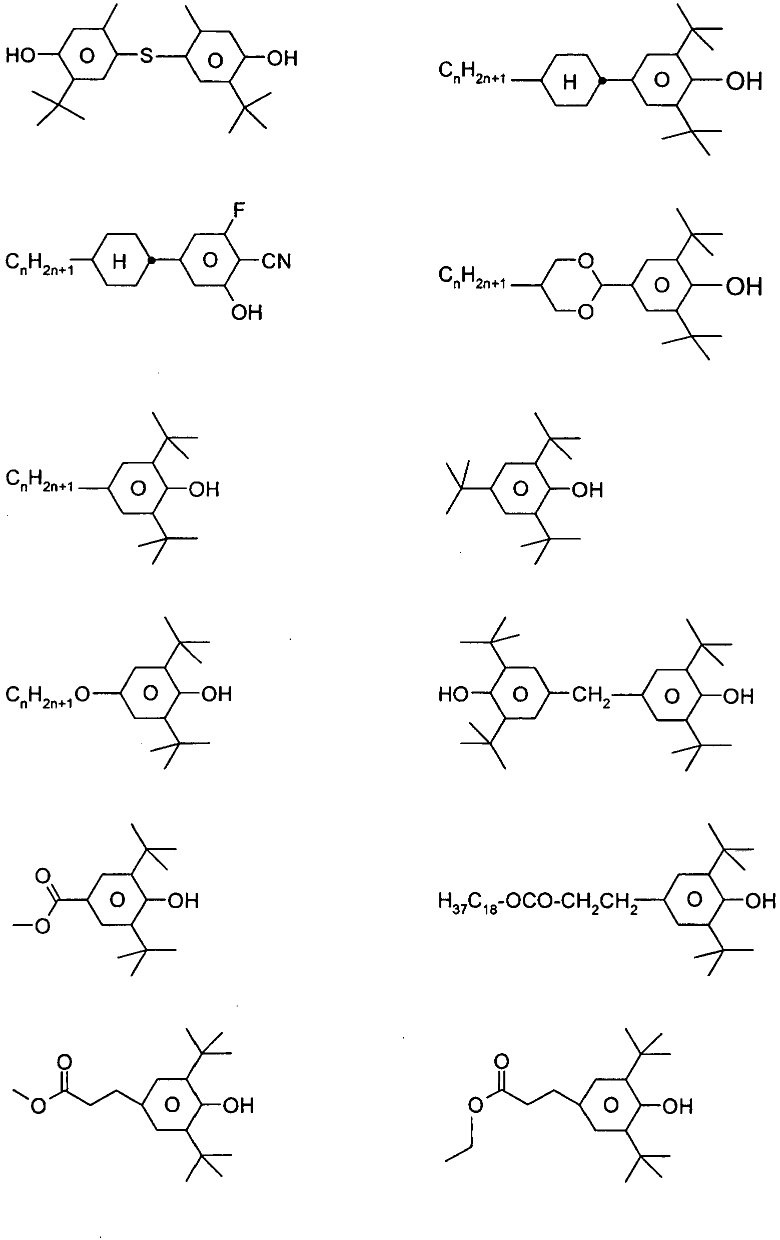

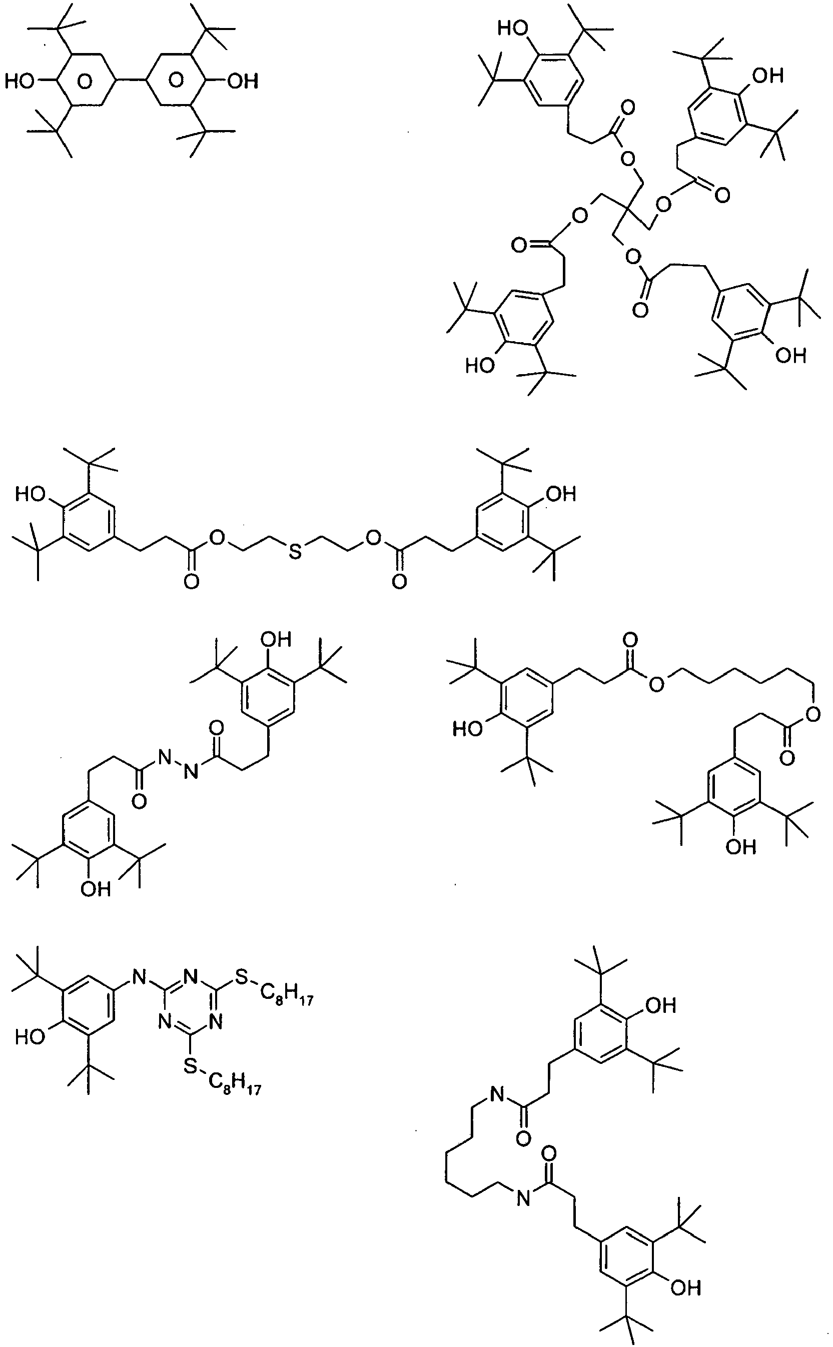

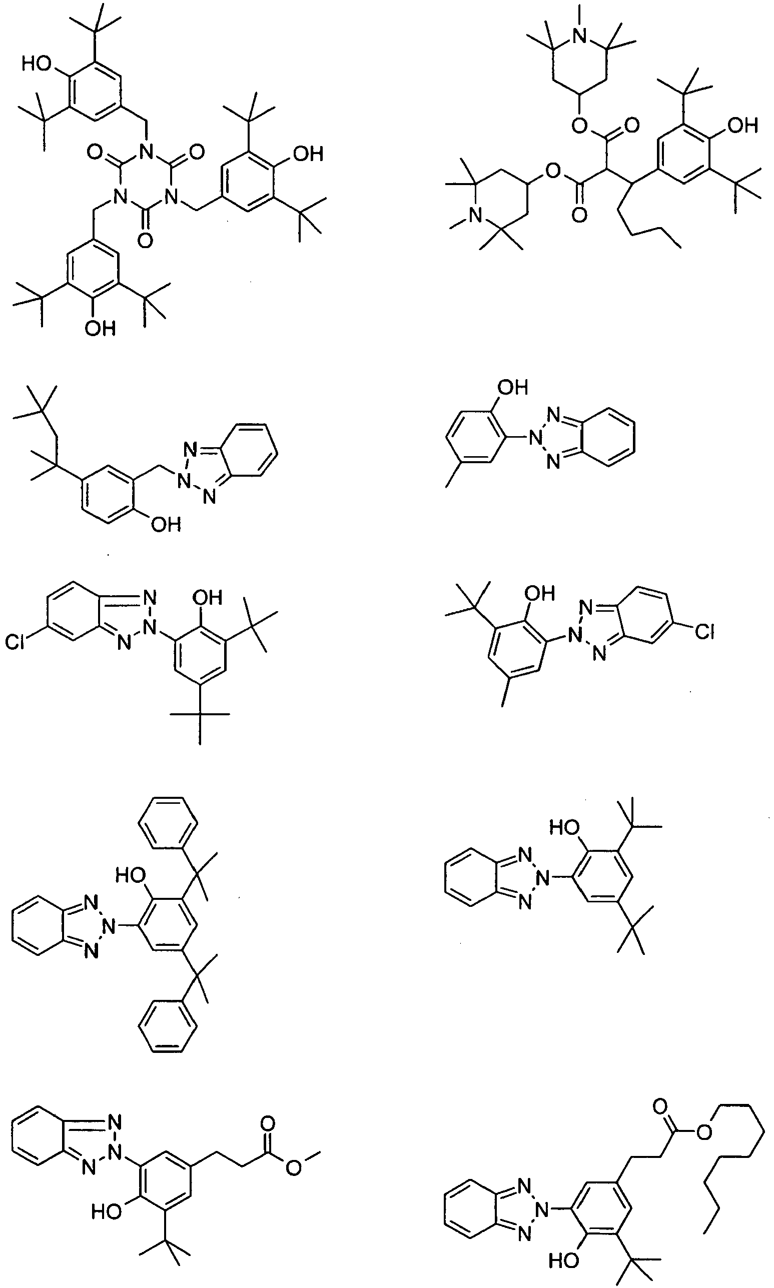

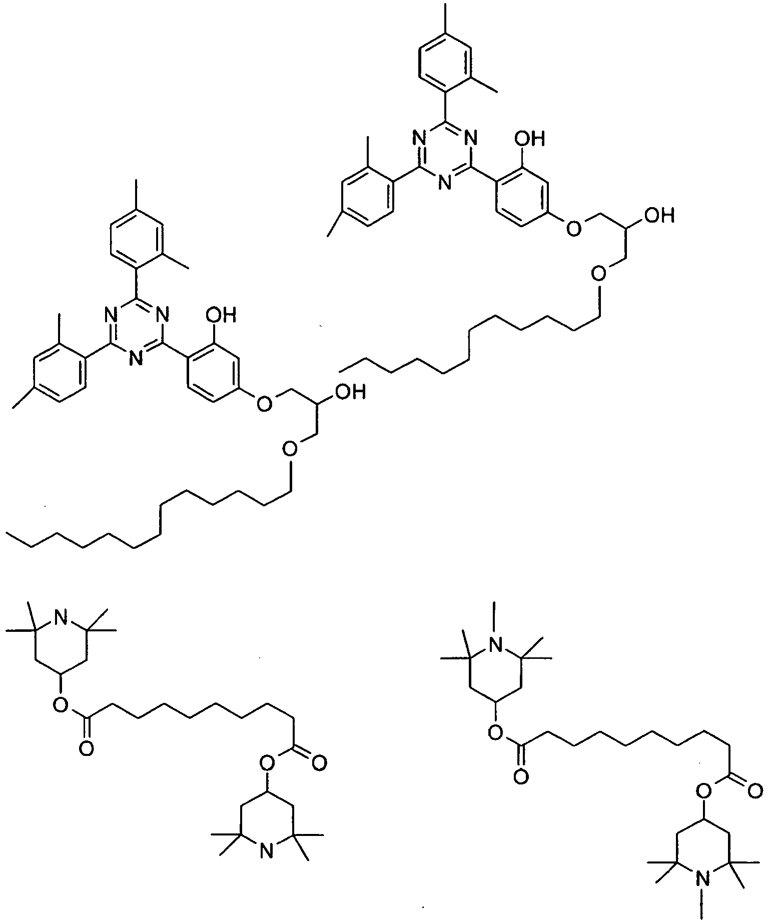

Vorzugsweise enthalten die FK-Medien 0 bis 10 Gew.%, insbesondere 1ppm bis 5 Gew.%, besonders bevorzugt 1ppm bis 1 Gew.% an Stabilisatoren. Vorzugsweise enthalten die FK-Medien einen oder mehrere Stabilisatoren ausgewählt aus der Gruppe bestehend aus Verbindungen der Tabelle C.

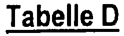

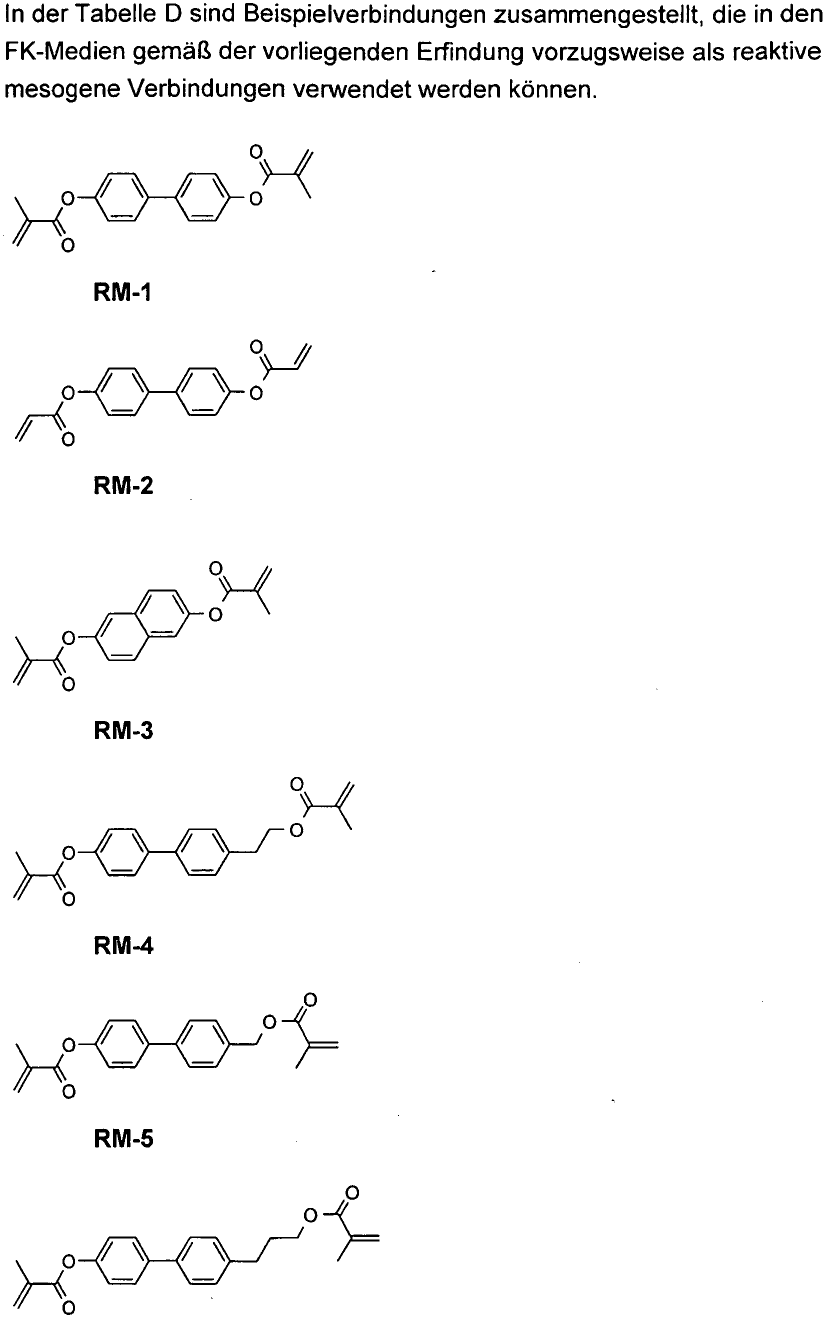

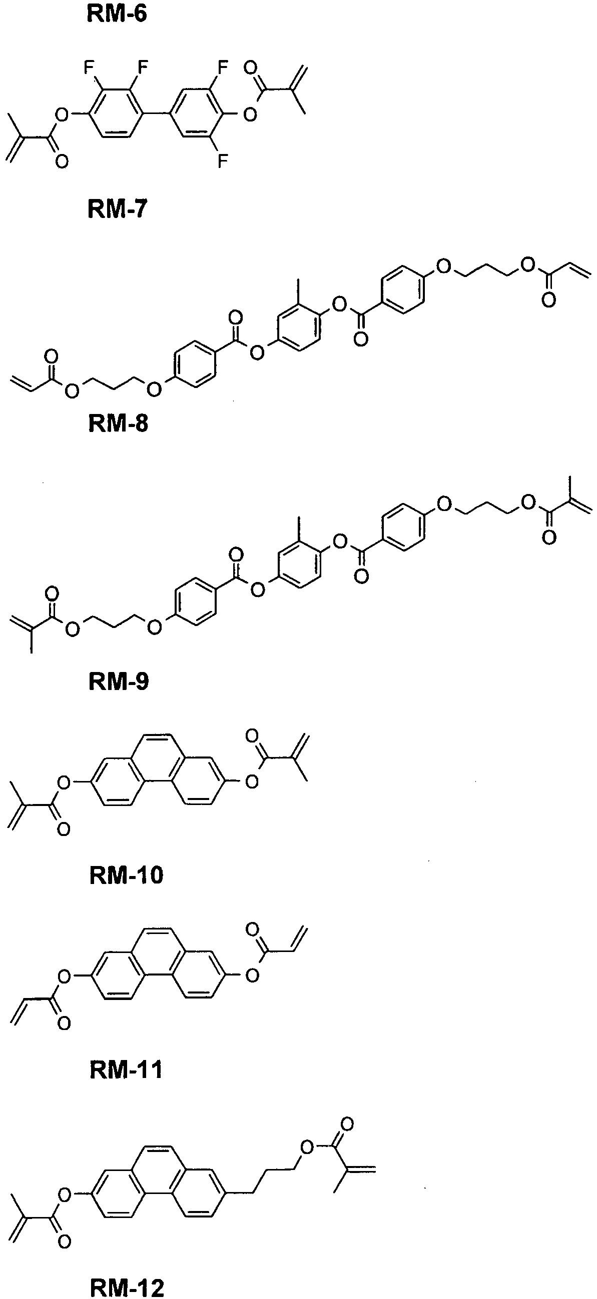

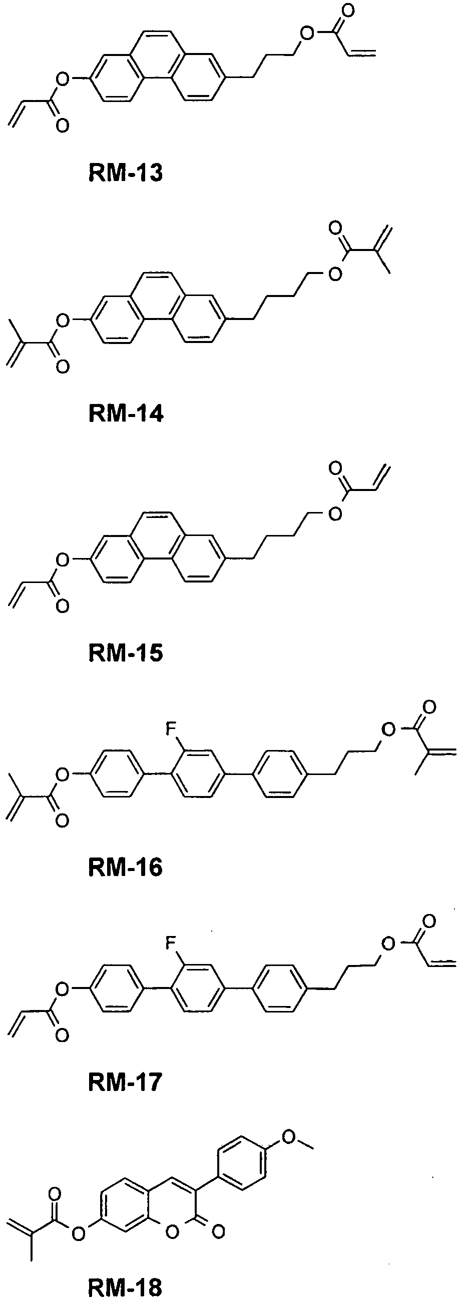

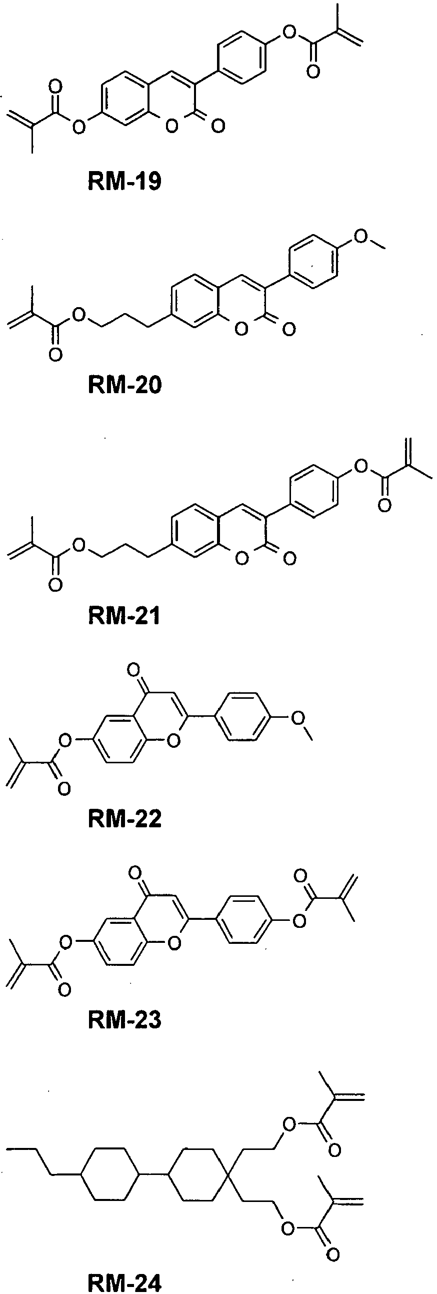

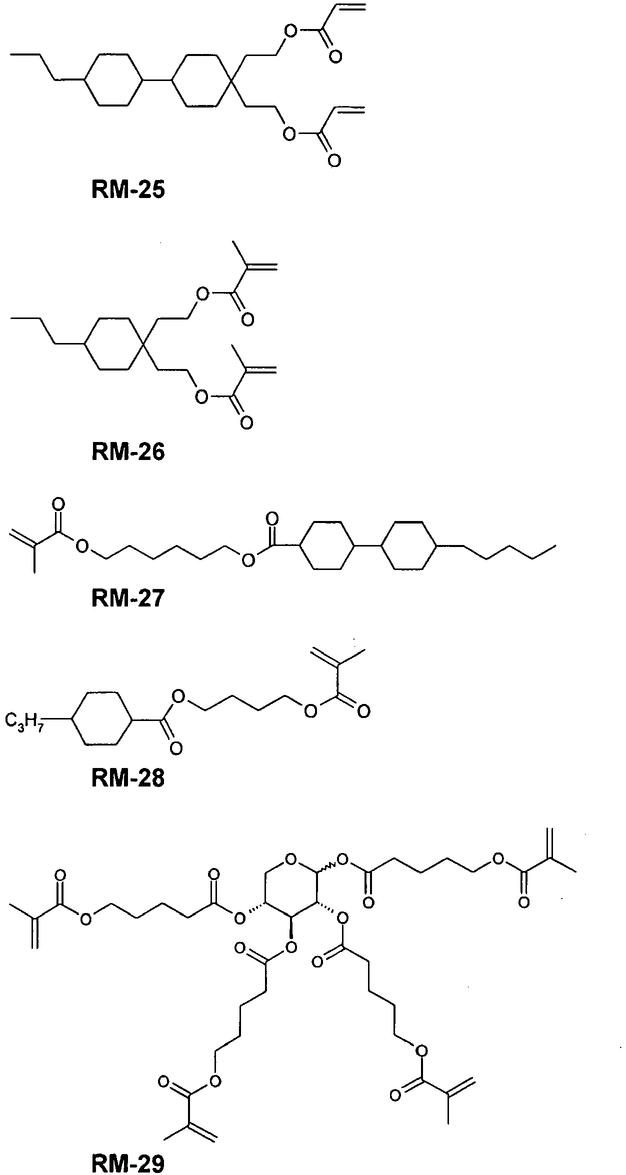

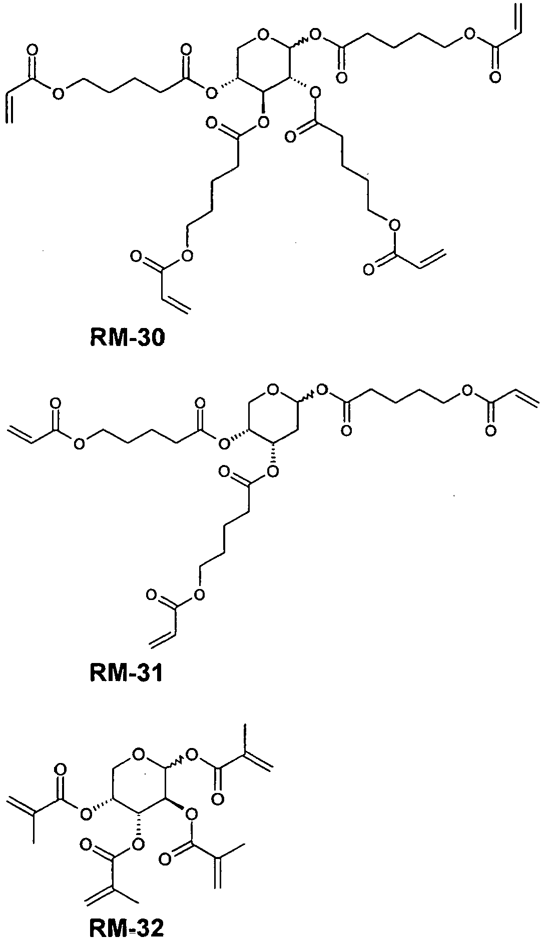

In einer bevorzugten Ausführungsform der vorliegenden Erfindung enthalten die mesogenen Medien eine oder mehrere Verbindungen ausgewählt aus der Gruppe der Verbindungen der Tabelle D.

Außerdem werden folgende Abkürzungen und Symbole verwendet:
- Vₒ: Schwellenspannung, kapazitiv [V] bei 20°C,
- nₑ: außerordentlicher Brechungsindex bei 20°C und 589 nm,
- nₒ: ordentlicher Brechungsindex bei 20°C und 589 nm,
- Δn: optische Anisotropie bei 20°C und 589 nm,
- ε_{⊥}: dielektrische Permittivität senkrecht zum Direktor bei 20°C und 1 kHz,
- ε_{||}: dielektrische Permittivität parallel zum Direktor bei 20°C und 1 kHz,
- Δε: dielektrische Anisotropie bei 20°C und 1 kHz,
- Kp., T(N,I): Klärpunkt [°C],
- γ₁: Rotationsviskosität bei 20°C [mPa·s],
- K₁: elastische Konstante, "splay"-Deformation bei 20°C [pN],
- K₂: elastische Konstante, "twist"-Deformation bei 20°C [pN],
- K₃: elastische Konstante, "bend"-Deformation bei 20°C [pN].

Soweit nicht explizit anders vermerkt, sind in der vorliegenden Anmeldung alle Konzentrationen in Gewichtsprozent angegeben und beziehen sich auf die entsprechende Gesamtmischung, d.h. das FK-Medium enthaltend alle festen oder flüssigkristallinen Komponenten, ohne Lösungsmittel.

Soweit nicht explizit anders vermerkt, sind in der vorliegenden Anmeldung alle angegebenen Werte für Temperaturen, wie z. B. der Schmelzpunkt T(C,N), der Übergang von der smektischen (S) zur nematischen (N) Phase T(S,N) und der Klärpunkt T(N,I), in Grad Celsius (°C) angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar.

Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20°C und Δn wird bei 589 nm und Δε bei 1 kHz bestimmt, sofern nicht jeweils explizit anders angegeben.

Der Begriff "Schwellenspannung" bezieht sich für die vorliegende Erfindung auf die kapazitive Schwelle (V₀), auch Freedericksz-Schwelle genannt, sofern nicht explizit anders angegeben. In den Beispielen kann auch, wie allgemein üblich, die optische Schwelle für 10 % relativen Kontrast (V₁₀) angegeben werden.

Die zur Messung der kapazitiven Schwellenspannung verwendete Anzeige besteht aus zwei planparallelen Glasträgerplatten im Abstand von 20 µm, welche auf den Innenseiten jeweils ein Elektrodenschicht sowie eine darüberliegende, ungeriebene Orientierungsschicht aus Polyimid aufweisen, die eine homöotrope Randorientierung der Flüssigkristallmoleküle bewirken.

Die zur Messung der Tiltwinkel verwendete Anzeige bzw. Testzelle besteht aus zwei planparallelen Glasträgerplatten im Abstand von 4 µm, welche auf den Innenseiten jeweils eine Elektrodenschicht sowie eine darüberliegende Orientierungsschicht aus Polyimid aufweisen, wobei die beiden Polyimidschichten antiparallel zueinander gerieben werden und eine homöotrope Randorientierung der Flüssigkristallmoleküle bewirken.

Die polymerisierbaren Verbindungen werden in der Anzeige bzw. Testzelle durch Bestrahlung mit UVA-Licht (üblicherweise 365nm) einer definierten Intensität für eine vorgegebene Zeit polymerisiert, wobei gleichzeitig eine Spannung an die Anzeige angelegt wird (üblicherweise 10V bis 30V Wechselstrom, 1 kHz). In den Beispielen wird, falls nicht anders angegeben, eine Quecksilberdampflampe mit 50 mW/cm² verwendet, die Intensität wird mit einem Standard-UV-Meter (Fabrikat Ushio UNI meter) gemessen, der mit einem Bandpassfilter bei 365nm ausgerüstet ist.

Der Tiltwinkel wird per Drehkristall-Experiment (Autronic-Melchers TBA-105) bestimmt. Ein niedriger Wert (d.h. eine große Abweichung vom 90°-Winkel) entspricht dabei einem großen Tilt.

Der VHR -Wert wird wie folgt gemessen: Zur FK-Host-Mischung werden 0.3% einer polymerisierbaren monomeren Verbindung zugesetzt, und die dadurch entstandene Mischung in TN-VHR-Testzellen gefüllt (90° gerieben, Orientierungsschicht TN-Polyimid, Schichtdicke d ≈ 6 µm). Der HR-Wert wird nach 5min bei 100°C vor und nach 2h UV-Belastung (suntest) bei 1V, 60Hz, 64µs pulse bestimmt (Messgerät: Autronic-Melchers VHRM-105).

Zur Untersuchung der Tieftemperaturstabilität, auch als "LTS" (low temperature stability) bezeichnet, d.h. der Stabilität der FK-Mischung gegen spontane Auskristallisation einzelner Komponenten bei tiefen Temperaturen, werden Fläschchen mit 1g FK/RM-Mischung bei -10°C eingelagert und es wird regelmäßig überprüft, ob die Mischungen auskristallisiert waren.

### Beispiel 1:2-Methyl-acrylsäure-4-[4-(2-methyl-acryloyloxy)-phenylethinyl]-benzylester

### 1.1 4-(4-Hydroxymethyl-phenylethinyl)-phenol

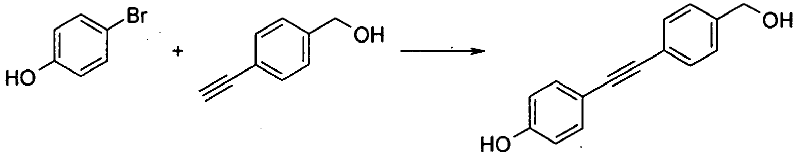

6,50 g (37,6 mmol) 4-Bromphenol und 5,00 g (37,8 mmol) 4-Ethinylbenzylalkohol werden in 70 ml THF und 10 ml Diisopropylamin vorgelegt und nach Zugabe von 1,50 g (2,14 mmol) Bis(triphenylphosphin)palldium(II)chlorid und 0,40 (2,10 mmol) Kupfer(I)iodid über Nacht unter Rückfluß erhitzt. Nach Zugabe von 100 ml Essigester wird mit 2 M Salzsäure angesäuert, die wäßrige Phase dreimal mit Essigester extrahiert und die vereinigten org. Phasen werden über Natriumsulfat getrocknet. Das Lösungsmittel wird i. Vak. entfernt und der Rückstand durch Chromatographie an Kieselgel gereinigt. Man erhält 4-(4-Hydroxymethyl-phenylethinyl)-phenol als farblosen Feststoff.

¹H-NMR (400 MHz, DMSO-d₆)
δ = 4,52 ppm (s, 2 H, CH₂), 5,27 (s, br., 1 H, OH), 6,79 (d, J = 8,7 Hz, 2 H, Ar-H), 7,34 (d, J = 8,1 Hz, 2 H, Ar-H), 7,36 (d, J = 8,7 Hz, 2 H, Ar-H), 7,45 (d, J = 8,1 Hz, 2 H, Ar-H), 9,93 (s, br., 1 H, OH).

### 1.2 2-Methyl-acrylsäure-4-[4-(2-methyl-acryloyloxy)-phenylethinyl]-benzylester

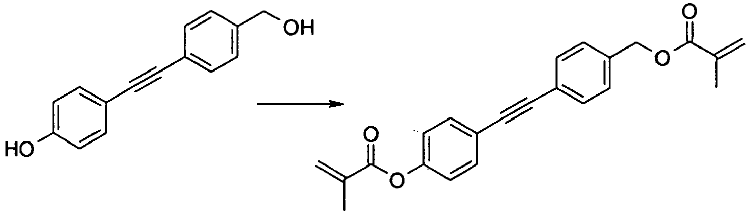

4-(4-Hydroxymethyl-phenylethinyl)-phenol und 2,5 ml Triethylamin werden in 40 ml Dichlormethan vorgelegt und unter Eiskühlung mit einer Lösung von 1,5 g (14 mmol) Acrylsäurechlorid versetzt. Nach 3 h wird der Ansatz über Kieselgel filtriert und das Lösungsmittel i. Vak. entfernt. Kristallisation des Rohproduktes aus Ethanol liefert den 2-Methyl-acrylsäure-4-[4-(2-methyl-acryloyloxy)-phenylethinyl]-benzylester als farblose Kristalle vom Schmp. 65 °C.

### Beispiel 2: 2-Methyl-acrylsäure-6-{4-[3-(2-methyl-acryloyloxy)-propyl]-phenylethinyl}-naphthalin-2-ylester

### 2.1 2-Triisopropylsilanyloxy-6-trimethylsilanylethinylnaphthalin

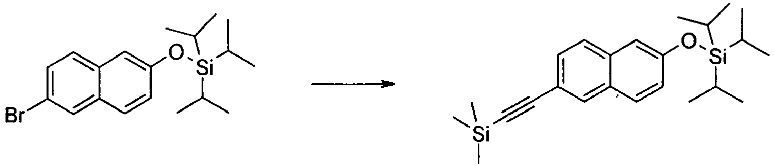

35,0 g (90,4 mmol) (6-Brom-naphthalin-2-yloxy)-triisopropyl-silan werden in 120 ml THF und 70 ml Diisopropylamin vorgelegt und nach Zugabe von 3,50 g (5,0 mmol) Bis(triphenylphosphin)palladium(II)chlorid und 1,0 g (5,3 mmol) Kupfer(I)iodid werden bei 70°C 25,0 g (0,255 mol) Trimethylsilylacetylen in 30 ml THF zutropfen gelassen. Der Ansatz wird 4 h bei 70 °C und über Nacht bei Raumtemp. rühren gelassen, mit 150 ml MTB-Ether versetzt, dreimal mit Wasser gewaschen, über Natriumsulfat getrocknet und i. Vak. eingeengt. Filtration des Rohproduktes mit Heptan über Kieselgel liefert 2-Triisopropylsilanyloxy-6-trimethylsilanylethinylnaphthalin, das ohne weiter Aufreinigung in der nächsten Stufe eingesetzt wird.

### 2.2 (6-Ethinyl-naphthalin-2-yloxy)-triisopropylsilan

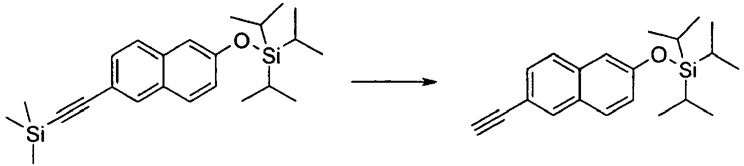

14,5 g (34,7 mmol) (6-Ethinyl-naphthalin-2-yloxy)-triisopropylsilan werden in 250 ml Methanol heiß gelöst und nach Zugabe von 5,2 g (37,6 mmol) Kaliumcarbonat 45 min bei Raumtemp. rühren gelassen. Der Ansatz wird mit MTB-Ether und ges. Natriumhydrogencarbonatlsg. versetzt, die org. Phase abgetrennt und die wäßr. Phase dreimal mit MTB-Ether extrahiert. Die vereinigten org. Phasen werden mit ges. Natriumchloridlsg. gewaschen und über Natriumsulfat getrocknet. Das Lösungsmittel wird i. Vak. entfernt und der Rückstand mit Heptan/Toluol (4:1) über Kieselgel filtriert. Man erhält (6-Ethinyl-naphthalin-2-yloxy)-triisopropylsilan als farbloses Öl.

### 2.3 3-(4-(6-Triisopropylsilanyloxy-naphthalin-2-ylethinyl)-phenyl]-propan-1-ol

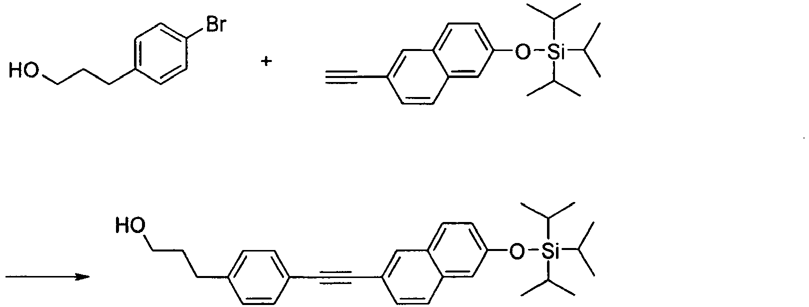

7,0 g (32,5 mmol) 3-(4-Brom-phenyl)-propan-1-ol werden in 70 ml THF und 30 ml Diisopropylamin vorgelegt und nach Zugabe von 1,0 g (1,4 mmol) Bis(triphenylphosphin)palladium(II)chlorid und 0,4 g (2,1 mmol) Kupfer(I)iodid werden bei 70°C 16,4 (50,5 mmol) (6-Ethinyl-naphthalin-2-yloxy)-triisopropylsilan in 30 ml THF zutropfen gelassen. Der Ansatz wird 5 h bei 70 °C rühren gelassen, mit 200 ml Essigester versetzt und dreimal mit Wasser gewaschen. Die vereinigten wäßr. Phasen werden mit Essigester extrahiert, die vereinigten org. Phasen über Natriumsulfat getrocknet und i. Vak. eingeengt. Chromatographie des Rohproduktes mit zunächst Toluol und dann Toluol/Essigester (7:3) an Kieselgel liefert 2-Triisopropylsilanyloxy-6-trimethylsilanylethinylnaphthalin als braunes Öl, das ohne weiter Aufreinigung in der nächsten Stufe eingesetzt wird.

### 2.4 6-[4-(3-Hydroxy-propyl)-phenylethinyl]-naphthalin-2-ol

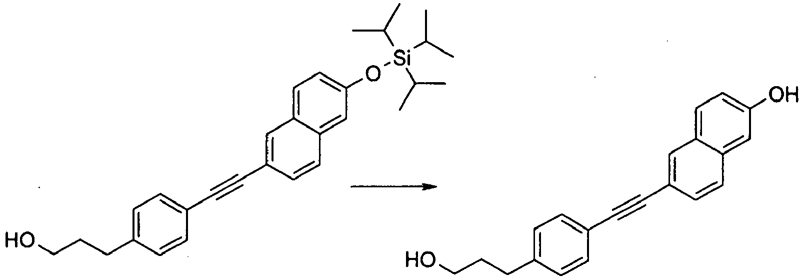

8,0 g (17,4 mmol) 3-[4-(6-Triisopropylsilanyloxy-naphthalin-2-ylethinyl)-phenyl]-propan-1-ol werden in 100 ml THF gelöst und unter Eiskühlung mit 22 ml (22 mmol) einer 1 M Lösung von Tetrabutylammoniumflorid in THF versetzt. Nach 30 min wird die Kühlung entfernt und der Ansatz 1 h bei Raumtemp. rühren gelassen. Der Ansatz wird auf Wasser gegeben, mit verd. Salzsäure angesäuert und dreimal mit Essigester extrahiert. Die vereinigten org. Phasen werden über Natriumsulfat getrocknet, das Lösungsmittel i. Vak. entfernt und der Rückstand mit Toluol/Essigester/Ethanol (60:39:1) an Kieselgel chromatographiert. Kristallisation des Rohproduktes aus Toluol liefert 6-[4-(3-Hydroxy-propyl)-phenylethinyl]-naphthalin-2-ol als farblosen Feststoff.

¹H-NMR (400 MHz, CDCl₃)
δ=1,91 ppm (m_{c}, 2 H, -CH₂CH₂CH₂-), 2,74 (t, J = 7,5 Hz, 2 H, Ar-CH₂-), 3,69 (m_{c}, 2 H, -CH2-OH), 5,18 (s, 1 H, OH), 7,12 (m_{c}, 2 H, Ar-H), 7,20 (AB-d, J = 8,2 Hz, 2 H, Ar-H), 7,49 (AB-d, J = 8,2 Hz, 2 H, Ar-H), 7,53 (dd, J = 8,5 Hz, J = 1,6 Hz, 1 H, Ar-H), 7,64 (d, J = 8,5 Hz, 1 H, Ar-H), 7,73 (d, J = 8,7 Hz, 1 H, Ar-H), 7,97 (d, br., J = 0,8 Hz, 1 H, Ar-H).

### 2.5 2-Methyl-acrylsäure-6-{4-[3-(2-methyl-acryloyloxy)-propyl]-phenylethinyl}-naphthalin-2-ylester

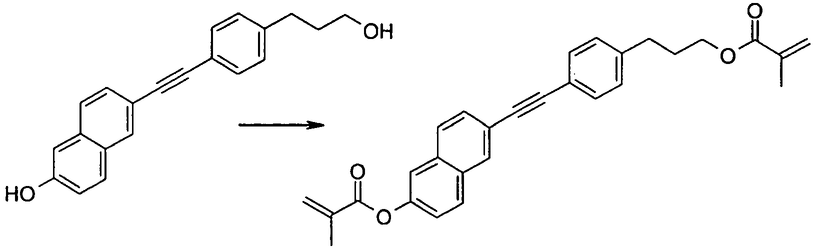

In Analogie zu Beispiel 1 erhält man aus 6-[4-(3-Hydroxy-propyl)-phenylethinyl]-naphthalin-2-ol den 2-Methyl-acrylsäure-6-{4-[3-(2-methyl-acryloyloxy)-propyl]-phenylethinyl}-naphthalin-2-ylester als farblose Kristalle vom Schmp. 85 °C.

### Beispiel 3: 2-Methyl-acrylsäure-4'-{4-[3-(2-methyl-acryloyloxy)-propyl]-phenylethynyl}-biphenyl-4-yl ester

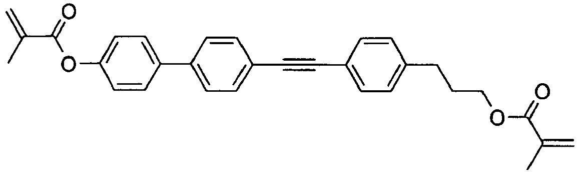

Ausgehend von 4-Brom-biphenyl-4-ol erhält man in Analogie zu dem in Beispiel 2 beschriebenen Syntheseverfahren den 2-Methyl-acrylsäure-4'-{4-[3-(2-methyl-acryloyloxy)-propyl]-phenylethynyl}-biphenyl-4-yl ester als farblose Kristalle vom Schmp. 123 °C.

### Anwendungsbeispiel 1

Die nematische FK-Mischung N1 wird wie folgt formuliert

| | | | |
|---|---|---|---|
| CCH-501 | 9,00 % | Kp. | + 70,0 |
| CCH-35 | 14,00 % | Δn | 0,0825 |
| PCH-53 | 8,00 % | Δε | - 3,5 |
| CY-3-04 | 14,00 % | ε_{\|\|} | 3,5 |
| CY-5-O4 | 13,00 % | K₃/K₁ | 1,00 |
| CCY-3-O2 | 8,00 % | γ₁ | 141 |
| CCY-5-O2 | 8,00 % | V₀ | 2,06 |
| CCY-2-1 | 9,00 % | | |
| CCY-3-1 | 9,00 % | | |
| CPY-2-O2 | 8,00 % | | |

Zur FK-Mischung N1 werden 0.3% einer polymerisierbaren monomeren Verbindung aus den unten aufgeführten Beispielen zugesetzt und die dadurch entstandenen Mischungen in VA-e/o-Testzellen gefüllt (antiparallel gerieben, Orientierungsschicht VA-Polyimid, Schichtdicke d ≈ 4µm). Unter Anlegen einer Spannung von 24 V (Wechselstrom) werden die Zellen in der angegebenen Zeit mit UV-Licht der Intensität 50 mW/cm² bestrahlt, dadurch erfolgt Polymerisation der monomeren Verbindung. Vor und nach der UV-Bestrahlung wird per Drehkristall-Experiment (Autronic-Melchers TBA-105) der Tiltwinkel bestimmt.

Zur Bestimmung der Polymerisationsgeschwindigkeit wird der Restgehalt an unpolymerisiertem RM (in Gew.%) in den Testzellen nach verschiedenen Belichtungszeiten mit der HPLC-Methode gemessen. Dazu wird jede Mischung jeweils unter den angegebenen Bedingungen in der Testzelle polymerisiert. Danach wird die Mischung mit Methylethylketon aus der Testzelle gespült und vermessen.

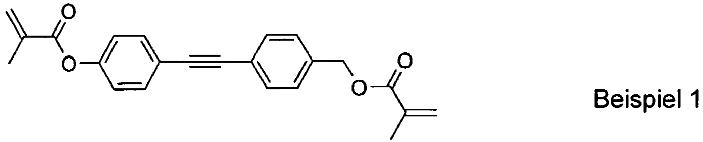

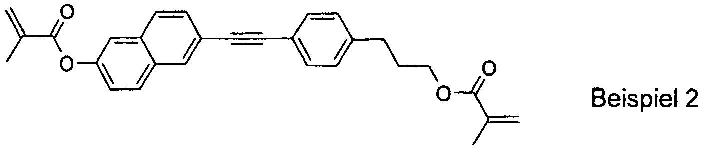

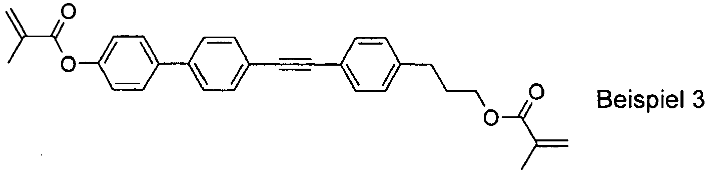

Die Tiltwinkelergebnisse und die RM-Konzentrationen nach verschiedenen Belichtungszeiten sind in Tabelle 1 zusammengefasst.

**Tabelle 1 (t = Belichtungszeit)**

| | Bsp. 1 | Bsp. 2 | Bsp. 3 | Bsp. 1 | Bsp. 2 |
|---|---|---|---|---|---|
| t/s | Tiltwinkel / ° | | | Rest-RM / % | |
| 0 | 88,5 | 87,8 | 88,4 | 0,300 | 0,300 |
| 30 | 88,8 | 83,3 | 85,1 | - | - |
| 60 | 86,7 | 67,7 | 82,8 | - | - |
| 120 | 81,0 | 35,6 | 78,6 | 0,131 | 0,000 |
| 240 | 68,9 | 25,0 | 75,2 | 0,030 | 0,000 |
| 360 | 55,4 | 21,8 | 72,5 | 0,013 | 0,000 |

Wie aus Tabelle 1 ersichtlich ist, können mit den erfindungsgemäßen Monomeren aus Beispiel 1, 2 und 3 sehr schnell ein kleiner Tiltwinkel nach Polymerisation sowie eine sehr schnelle Polymerisationsgeschwindigkeit erzielt werden.

## Patentansprüche

1. Verwendung von Verbindungen der Formel I
P-(Sₚ)ₛ₁-A¹-Z¹(-Z²-A³)ₘ-(Sp)ₛ₂-P I
worin die einzelnen Reste folgende Bedeutung besitzen
P bei jedem Auftreten gleich oder verschieden eine polymerisierbare Gruppe,
Sp bei jedem Auftreten gleich oder verschieden eine Abstandsgruppe,
s1, s2 jeweils unabhängig voneinander 0 oder 1,
A¹, A², A³ jeweils unabhängig voneinander 1,4-Phenylen, Naphthalin-1,4-diyl oder Naphthalin-2,6-diyl, wobei in diesen Gruppen auch eine oder mehrere CH-Gruppen durch N ersetzt sein können, Phenanthren-2,7-diyl, Anthracen-2,7-diyl, 9,10-Dihydrophenanthren-2,7-diyl, 9H-Fluoren,2,7-diyl, 9,9-Dimethylfluoren-2,7-diyl, Dibenzofuran-3,7-diyl, wobei alle diese Gruppen unsubstituiert oder durch L ein- oder mehrfach substituiert sein können,
m 0, 1 oder 2,
L bei jedem Auftreten gleich oder verschieden P-, P-Sp-, OH, CH₂OH, Halogen, -CN, -NO₂ , -NCO, -NCS, -OCN, -SCN, -C(=O)N(R^{x})₂, -C(=O)Y¹, -C(=O)R^{x}, -N(R^{x})₂, optional substituiertes Silyl oder eine optional substituierte Kohlenstoffgruppe oder Kohlenwasserstoffgruppe,
R^{x} P-, P-Sp-, H, Halogen, geradkettiges, verzweigtes oder cyclisches Alkyl mit 1 bis 25, vorzugsweise 1 bis 12 C-Atomen, worin auch eine oder mehrere nicht benachbarte CH₂-Gruppen durch -O-, -S-, -CO-, -CO-O-, -O-CO-, -O-CO-O- so ersetzt sein können, dass O-und/oder S-Atome nicht direkt miteinander verknüpft sind, und worin auch ein oder mehrere H-Atome durch F, Cl, P- oder P-Sp- ersetzt sein können,
Y¹ Halogen,
Z¹, Z² jeweils unabhängig voneinander -CO-O-, -OCO-, - CY=CY-, -C≡C- oder eine Einfachbindung,
Y bei jedem Auftreten gleich oder verschieden H oder F,
wobei mindestens einer der Reste Z¹ und Z² -C≡C- bedeutet,
in FK-Medien und FK-Anzeigen des PS- oder PSA-Typs.

2. Verwendung nach Anspruch 1, **dadurch gekennzeichnet, dass** in den Verbindungen der Formel I
A¹ und A² jeweils unabhängig voneinander 1,4-Phenylen, Naphthalin-2,6-diyl, Phenanthren-2,7-diyl oder Anthracen-2,7-diyl, wobei alle diese Gruppen unsubstituiert oder durch L ein- oder mehrfach substituiert sein können, und
L P-Sp-, OH, CH₂OH, F, Cl, Br, I, -CN, -NO₂, -NCO, -NCS, -OCN, -SCN, -C(=O)N(R^{x})₂, -C(=O)Y¹, -C(=O)R^{x}, -N(R^{x})₂, optional substituiertes Silyl, optional substituiertes Aryl mit 6 bis 20 C Atomen, geradkettiges oder verzweigtes Alkyl oder Alkoxy 1 bis 25, vorzugsweise 1 bis 12, C-Atomen, oder geradkettiges oder verzweigtes Alkenyl, Alkinyl, Alkylcarbonyl, Alkoxycarbonyl, Alkylcarbonyloxy oder Alkoxycarbonyloxy mit 2 bis 25, vorzugsweise 2 bis 12, C-Atomen, worin in allen diesen Gruppen auch ein oder mehrere H-Atome durch F, Cl, P oder P-Sp- ersetzt sein können,
bedeuten, wobei R^{x} und Y¹ die in Anspruch 1 angegebene Bedeutung besitzen.

3. Verwendung nach Anpruch 1 oder 2, **dadurch gekennzeichnet, dass** die Verbindungen der Formel I ausgewählt sind aus der Gruppe bestehend aus den folgenden Unterformeln 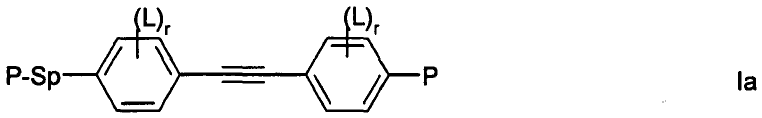 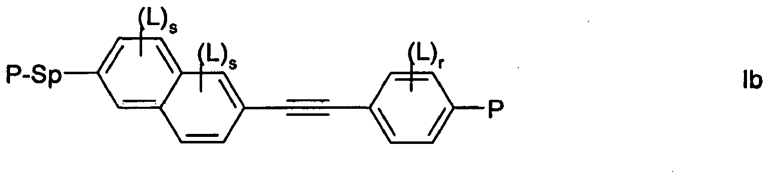 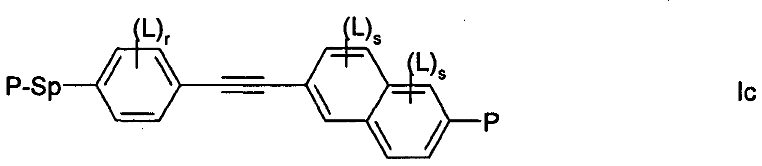 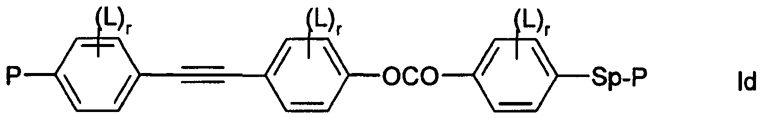 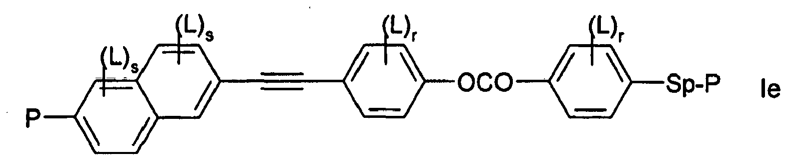 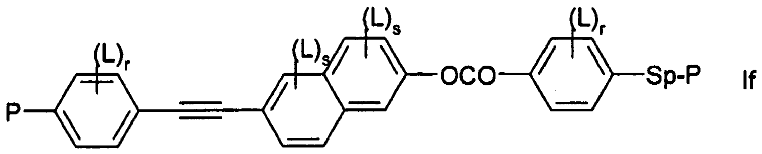 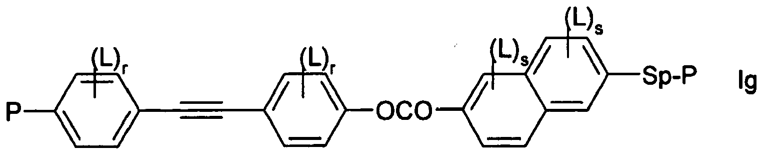 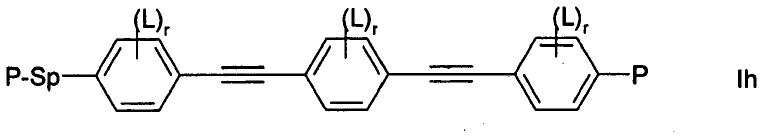 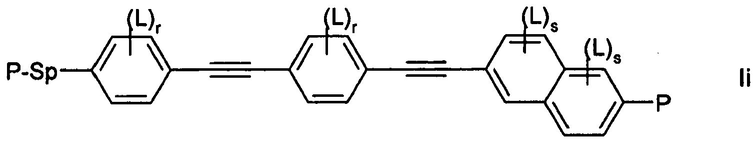 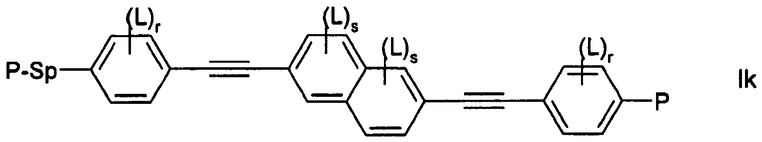 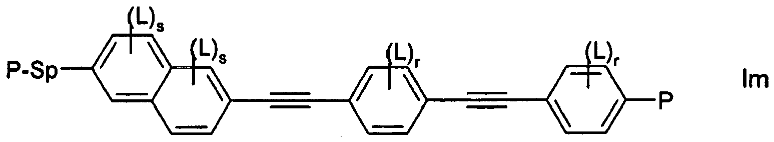 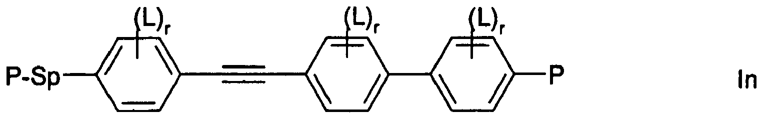 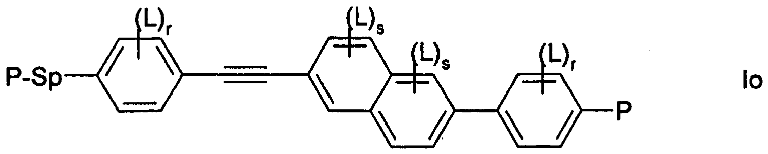 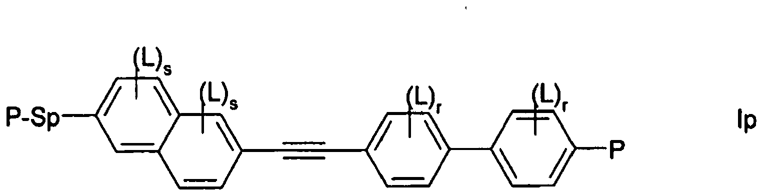 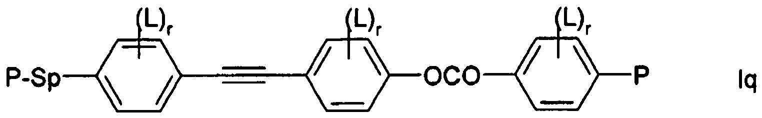 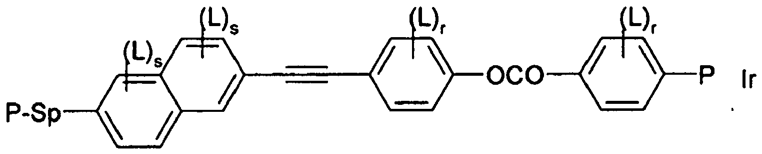 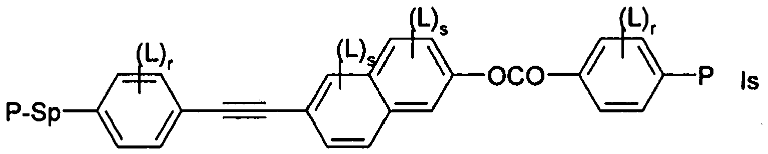 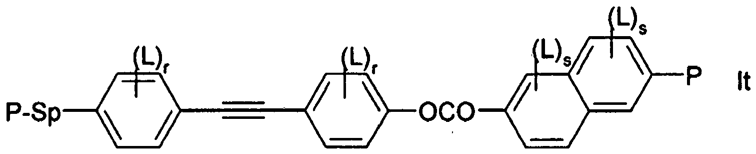 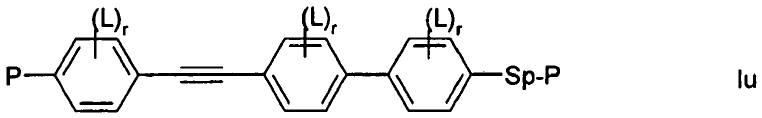 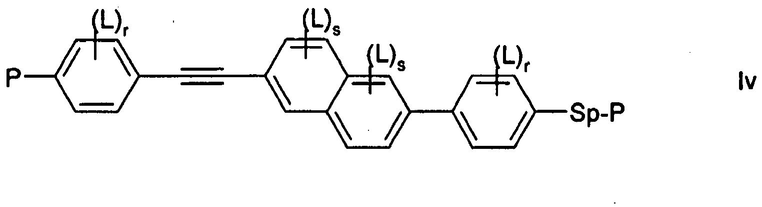 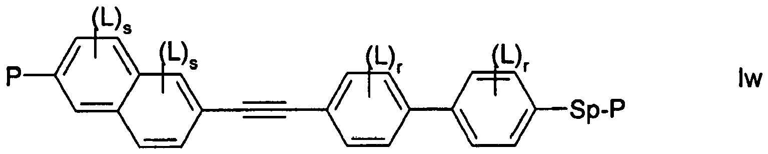 worin P, Sp und L die in Anspruch 1 angegebene Bedeutung besitzen, r 0, 1, 2, 3 oder 4 und s 0, 1, 2 oder 3 bedeuten.

4. Verwendung von Verbindungen nach einem oder mehreren der Ansprüche 1 bis 3 in FK-Anzeigen des PS- oder PSA-Typs, enthaltend eine FK-Zelle mit zwei Substraten und zwei Elektroden, wobei mindestens ein Substrat lichtdurchlässig ist und mindestens ein Substrat eine oder zwei Elektroden aufweist, sowie einer zwischen den Substraten befindlichen Schicht eines FK-Mediums enthaltend eine polymerisierte Komponente und eine niedermolekulare Komponente, wobei die polymerisierte Komponente erhältlich ist durch Polymerisation einer oder mehrerer polymerisierbarer Verbindungen zwischen den Substraten der FK-Zelle im FK-Medium, vorzugsweise unter Anlegen einer elektrischen Spannung an die Elektroden, wobei mindestens eine der polymerisierbaren Verbindungen eine polymerisierbare Verbindung nach einem oder mehreren der Ansprüche 1 bis 3 ist.

5. Verwendung nach einem oder mehreren der Ansprüche 1 bis 4, **dadurch gekennzeichnet, dass** das FK-Medium eine oder mehrere Verbindungen der Formel CY und/oder PY enthält: 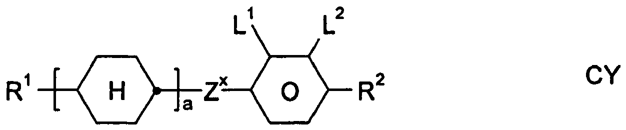 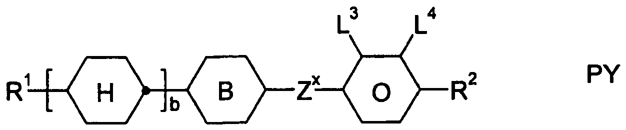 worin die einzelnen Reste folgende Bedeutung besitzen
a 1 oder 2,
b 0 oder 1, 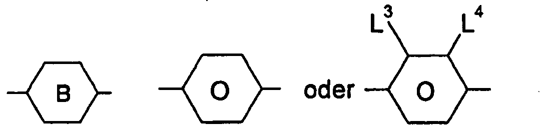
R¹ und R² jeweils unabhängig voneinander Alkyl mit 1 bis 12 C-Atomen, wobei auch eine oder zwei nicht benachbarte CH₂-Gruppen durch -O-, -CH=CH-, -CO-, -O-CO- oder -CO-O- so ersetzt sein können, dass O-Atome nicht direkt miteinander verknüpft sind,
Z^{x} -CH=CH-, -CH₂O-, -OCH₂-, -CF₂O-, -OCF₂-, -O-, -CH₂-, -CH₂CH₂-, oder eine Einfachbindung, vorzugsweise eine Einfachbindung,
L¹⁻⁴ jeweils unabhängig voneinander F, Cl, OCF₃, CF₃, CH₃, CH₂F, CHF₂.

6. Verwendung nach einem oder mehreren der Ansprüche 1 bis 5, **dadurch gekennzeichnet, dass** das FK-Medium eine oder mehrere Verbindungen der folgenden Formel enthält: 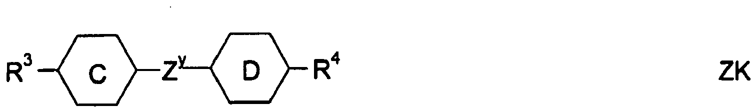 worin die einzelnen Reste folgende Bedeutung besitzen 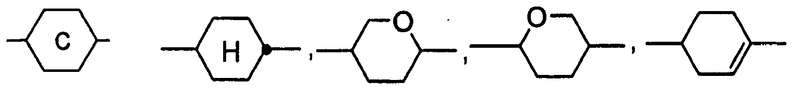 oder 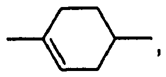 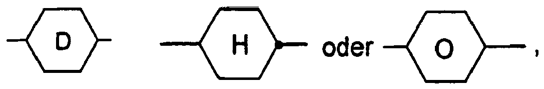
R³ und R⁴ jeweils unabhängig voneinander Alkyl mit 1 bis 12 C-Atomen, worin auch eine oder zwei nicht benachbarte CH₂-Gruppen durch -O-, -CH=CH-, -CO-, -O-CO- oder -CO-O- so ersetzt sein können, dass O-Atome nicht direkt miteinander verknüpft sind,
Z^{y} -CH₂CH₂-, -CH=CH-, -CF₂O-, -OCF₂-, -CH₂O-, -OCH₂-, -COO-, -OCO-, -C₂F₄-, -CF=CF- oder eine Einfachbindung.

7. FK-Anzeige nach einem oder mehreren der Ansprüche 1 bis 6.

8. FK-Anzeige nach Anspruch 8, **dadurch gekennzeichnet, dass** sie eine PSA-VA-, PSA-OCB-, PSA-IPS-, PSA-FFS- PSA-Posi-VA- oder PSA-TN-Anzeige ist.

9. FK-Medium enthaltend
- eine polymerisierbare Komponente A) enthaltend eine oder mehrere polymerisierbare Verbindungen, sowie
- eine flüssigkristalline Komponente B) enthaltend eine oder mehrere niedermolekulare Verbindungen,
**dadurch gekennzeichnet, dass** Komponente A) eine oder mehrere polymerisierbare Verbindungen nach einem oder mehreren der Ansprüche 1 bis 3 enthält, und Komponente B) eine oder mehrere Verbindungen ausgewählt aus Formel CY und PY nach Anspruch 6 enthält.

10. FK-Medium enthaltend
- eine polymerisierbare Komponente A) enthaltend eine oder mehrere polymerisierbare Verbindungen, sowie
- eine flüssigkristalline Komponente B) enthaltend eine oder mehrere niedermolekulare Verbindungen,
**dadurch gekennzeichnet, dass** Komponente A) eine oder mehrere polymerisierbare Verbindungen nach einem oder mehreren der Ansprüche 1 bis 3 enthält, wobei die Konzentration der polymerisierbaren Verbindungen im FK-Medium < 5 Gew.-% beträgt.

11. FK-Medium nach Anspruch 9 oder 10, worin Komponente B) eine oder mehrere Verbindungen ausgewählt aus Formel CY, PY und ZK nach Anspruch 6 oder 7 enthält.

12. Verfahren zu Herstellung einer FK-Anzeige des PS- oder PSA-Typs, indem man ein FK-Medium nach einem oder mehreren der Ansprüche 9 bis 11 in eine FK-Zelle mit zwei Substraten und zwei Elektroden, wobei mindestens ein Substrat lichtdurchlässig ist und mindestens ein Substrat eine oder zwei Elektroden aufweist, füllt, und die polymerisierbaren Verbindungen, vorzugsweise unter Anlegen einer elektrischen Spannung an die Elektroden, polymerisiert.

## Claims

1. Use of compounds of the formula I
P-(Sp)ₛ₁-A¹-Z¹-A²(-Z²-A³)ₘ-(Sp)ₛ₂-P I
in which the individual radicals have the following meaning:
P on each occurrence, identically or differently, denotes a polymerisable group,
Sp on each occurrence, identically or differently, denotes a spacer group,
s1, s2 each, independently of one another, denote 0 or 1,
A¹, A², A³ each, independently of one another, denote 1,4-phenylene, naphthalene-1 ,4-diyl or naphthalene-2,6-diyl, where, in addition, one or more CH groups in these groups may be replaced by N, phenanthrene-2,7-diyl, anthracene-2,7-diyl, 9,10-dihydrophenan-threne-2,7-diyl, 9H-fluorene-2,7-diyl, 9,9-dimethylfluor-ene-2,7-diyl, dibenzofuran-3,7-diyl, where all these groups may be unsubstituted or mono- or polysubstituted by L,
m denotes 0, 1 or 2,
L on each occurrence, identically or differently, denotes P-, P-Sp-, OH, CH₂OH, halogen, -CN, -NO₂, -NCO, -NCS, -OCN, -SCN, -C(=O)N(R^{x})₂, -C(=O)Y¹, -C(=O)R^{x}, -N(R^{x})₂, optionally substituted silyl or an optionally substituted carbon group or hydrocarbon group,
R^{x} denotes P-, P-Sp-, H, halogen, straight-chain, branched or cyclic alkyl having 1 to 25, preferably 1 to 12, C atoms, in which, in addition, one or more non-adjacent CH₂ groups may be replaced by -O-, -S-, -CO-, -CO-O-, -O-CO-, -O-CO-O- in such a way that O and/or S atoms are not linked directly to one another, and in which, in addition, one or more H atoms may be replaced by F, Cl, P- or P-Sp-,
Y¹ denotes halogen,
Z¹, Z² each, independently of one another, denote -CO-O-, -OCO-, -CY=CY-, -C≡C- or a single bond,
Y on each occurrence, identically or differently, denotes H or F,
where at least one of the radicals Z¹ and Z² denotes -C≡C-,
in LC media and LC displays of the PS or PSA type.

2. Use according to Claim 1, **characterised in that**, in the compounds of the formula I,
A¹ and A² each, independently of one another, denote 1,4-phenylene, naphthalene-2,6-diyl, phenanthrene-2,7-diyl or anthracene-2,7-diyl, where all these groups may be unsubstituted or mono- or polysubstituted by L, and
L denotes P-Sp-, OH, CH₂OH, F, Cl, Br, I, -CN, -NO₂, -NCO, -NCS, -OCN, -SCN, -C(=O)N(R^{x})₂, -C(=O)Y¹, -C(=O)R^{x}, -N(R^{x})₂, optionally substituted silyl, optionally substituted aryl having 6 to 20 C atoms, straight-chain or branched alkyl or alkoxy having 1 to 25, preferably 1 to 12, C atoms, or straight-chain or branched alkenyl, alkynyl, alkylcarbonyl, alkoxycarbonyl, alkylcarbonyloxy or alkoxycarbonyloxy having 2 to 25, preferably 2 to 12, C atoms, in which, in addition, one or more H atoms in all these groups may be replaced by F, Cl, P- or P-Sp-,
where R^{x} and Y¹ have the meaning indicated in Claim 1.

3. Use according to Claim 1 or 2, **characterised in that** the compounds of the formula I are selected from the group consisting of the following sub-formulae: 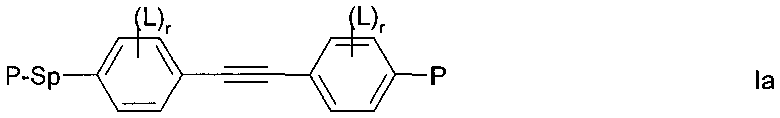 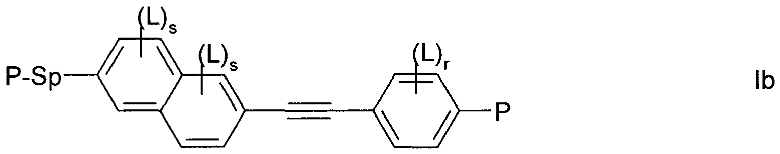 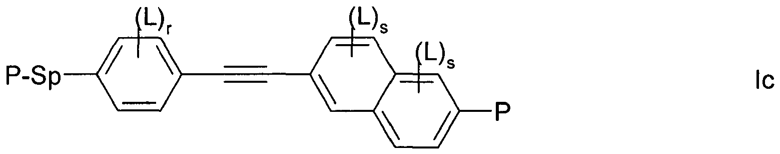 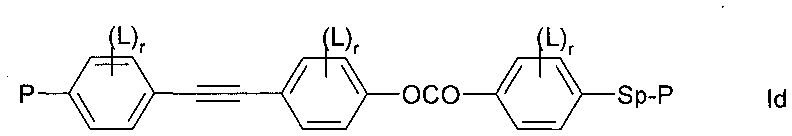 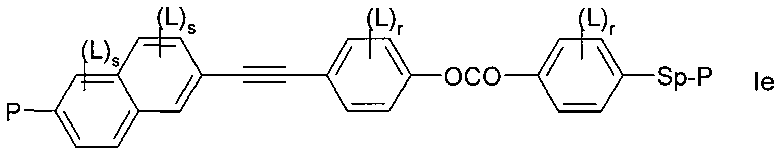 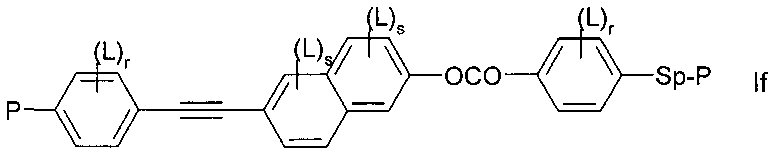 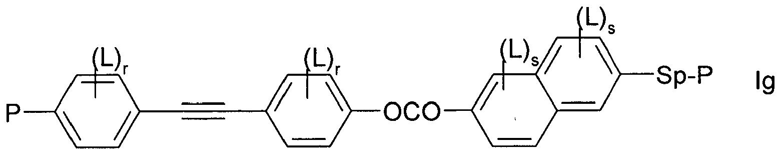 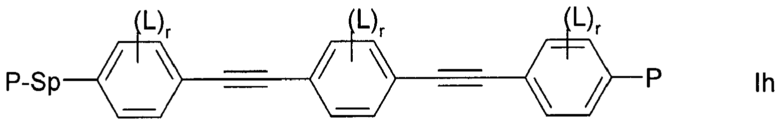 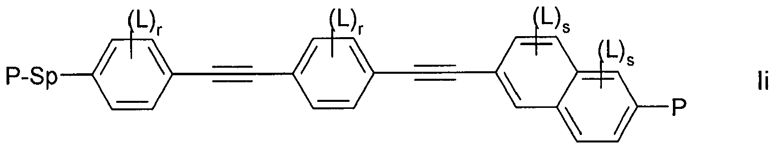 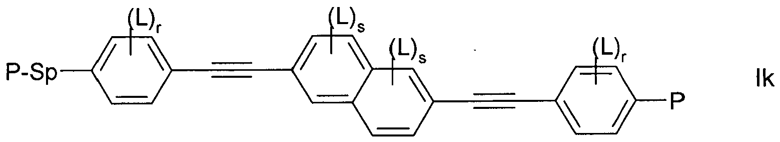 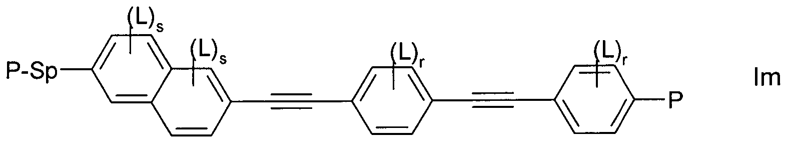 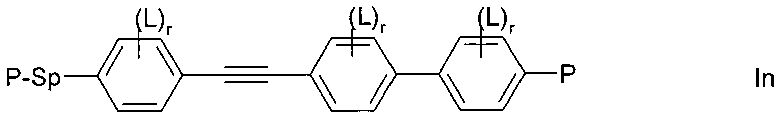 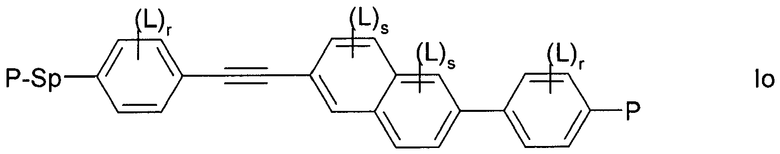 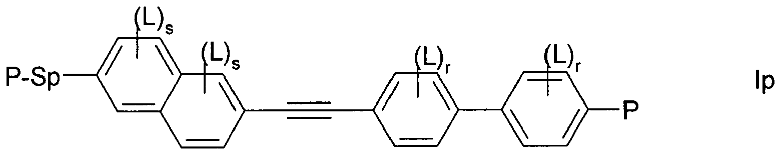 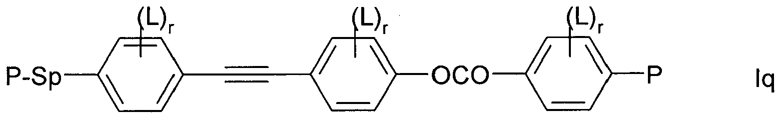 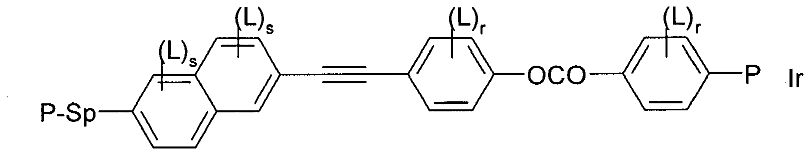 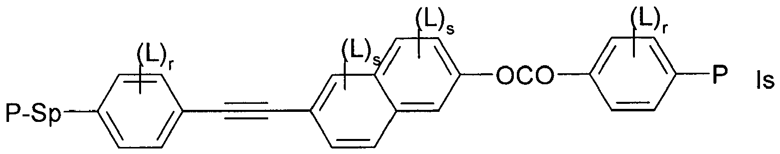 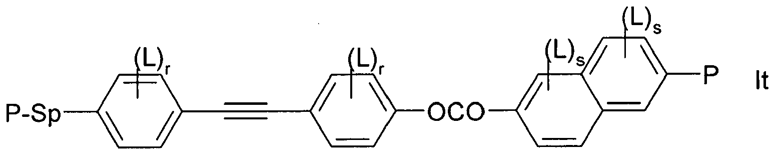 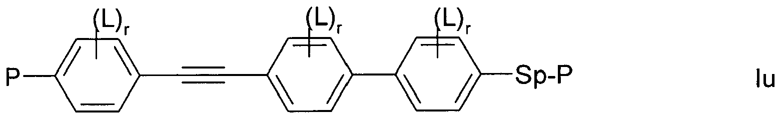 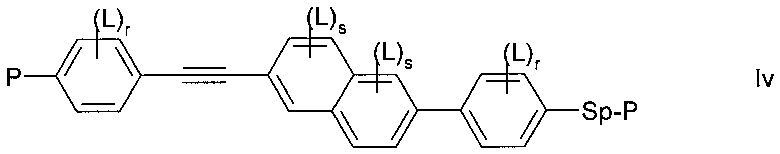 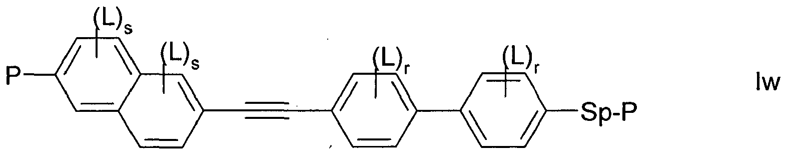 in which P, Sp and L have the meaning indicated in Claim 1, r denotes 0, 1, 2, 3 or 4, and s denotes 0, 1, 2 or 3.

4. Use of compounds according to one or more of Claims 1 to 3 in LC displays of the PS or PSA type containing an LC cell having two substrates and two electrodes, where at least one substrate is transparent to light and at least one substrate has one or two electrodes, and a layer, located between the substrates, of an LC medium comprising a polymerised component and a low-molecular-weight component, where the polymerised component is obtainable by polymerisation of one or more polymerisable compounds between the substrates of the LC cell in the LC medium, preferably with application of an electrical voltage to the electrodes, where at least one of the polymerisable compounds is a polymerisable compound according to one or more of Claims 1 to 3.

5. Use according to one or more of Claims 1 to 4, **characterised in that** the LC medium comprises one or more compounds of the formulae CY and/or PY: 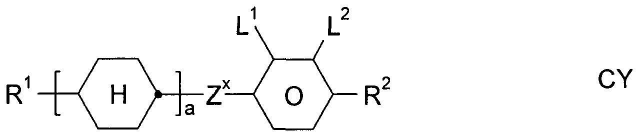 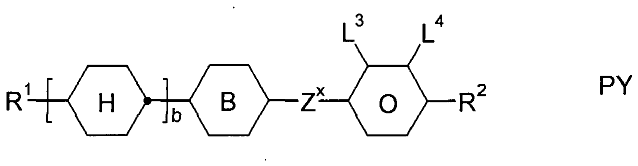 in which the individual radicals have the following meaning:
a denotes 1 or 2,
b denotes 0 or 1, 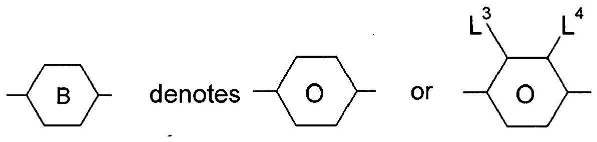
R¹ and R² each, independently of one another, denote alkyl having 1 to 12 C atoms, where, in addition, one or two non-adjacent CH₂ groups may be replaced by -O-, -CH=CH-, -CO-, -O-CO- or -CO-O- in such a way that O atoms are not linked directly to one another,
Z^{x} denotes -CH=CH-, -CH₂O-, -OCH₂-, -CF₂O-, -OCF₂-, -O-, -CH₂-, -CH₂CH₂- or a single bond, preferably a single bond,
L¹⁻⁴ each, independently of one another, denote F, Cl, OCF₃, CF₃, CH3, CH₂F, CHF₂.

6. Use according to one or more of Claims 1 to 5, **characterised in that** the LC medium comprises one or more compounds of the following formula: 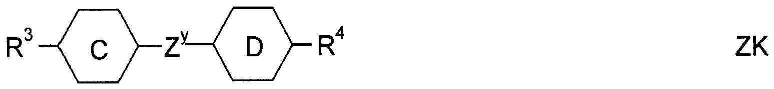 in which the individual radicals have the following meaning: 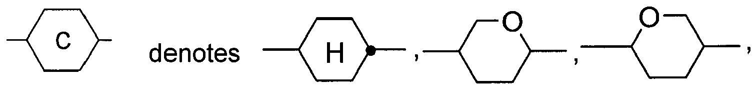 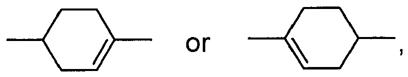 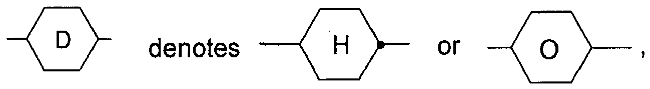
R³ and R⁴ each, independently of one another, denote alkyl having 1 to 12 C atoms, in which, in addition, one or two non-adjacent CH₂ groups may be replaced by -O-, -CH=CH-, -CO-, -O-CO- or -CO-O- in such a way that O atoms are not linked directly to one another,
Z^{y} denotes -CH₂CH₂-, -CH=CH-, -CF₂O-, -OCF₂-, -CH₂O-, -OCH₂-, -COO-, -OCO-, -C₂F₄-, -CF=CF- or a single bond.

7. LC display according to one or more of Claims 1 to 6.

8. LC display according to Claim 7, **characterised in that** it is a PSA-VA, PSA-OCB, PSA-IPS, PSA-FFS, PSA-positive-VA or PSA-TN display.

9. LC medium comprising
- a polymerisable component A) comprising one or more polymerisable compounds, and
- a liquid-crystalline component B) comprising one or more low-molecular-weight compounds,
**characterised in that** component A) comprises one or more polymerisable compounds according to one or more of Claims 1 to 3, and component B) comprises one or more compounds selected from formulae CY and PY according to Claim 5.

10. LC medium comprising
- a polymerisable component A) comprising one or more polymerisable compounds, and
- a liquid-crystalline component B) comprising one or more low-molecular-weight compounds,
**characterised in that** component A) comprises one or more polymerisable compounds according to one or more of Claims 1 to 3, where the concentration of the polymerisable compounds in the LC medium is < 5% by weight.

11. LC medium according to Claim 9 or 10, in which component B) comprises one or more compounds selected from formulae CY, PY and ZK according to Claim 5 or 6.

12. Process for the production of an LC display of the PS or PSA type in which an LC medium according to one or more of Claims 9 to 11 is introduced into an LC cell having two substrates and two electrodes, where at least one substrate is transparent to light and at least one substrate has one or two electrodes, and the polymerisable compounds are polymerised, preferably with application of an electrical voltage to the electrodes.

## Revendications

1. Utilisation de composés de la formule I
P-(Sp)ₛ₁-A¹-Z¹-A²(-Z²-A³)ₘ-(Sp)ₛ₂-P I
dans laquelle les radicaux individuels présentent la signification qui suit :
P représente, pour chaque occurrence, de manière identique ou différente, un groupe polymérisable,
Sp représente, pour chaque occurrence, de manière identique ou différente, un groupe d'espaceur,
s1, s2 représentent, chacun indépendamment de l'autre, 0 ou 1,
A¹, A², A³ représentent, chacun indépendamment des autres, 1,4-phénylène, naphtalène-1,4-diyle ou naphtalène-2,6-diyle, où, en outre, un ou plusieurs groupes CH dans ces groupes peut/peuvent être remplacé(s) par N, phénanthrène-2,7-diyle, anthracène-2,7-diyle, 9,10-dihydrophénanthrène-2,7-diyle, 9H-fluorène-2,7-diyle, 9,9-diméthylfluorène-2,7-diyle, dibenzofuran-3,7-diyle, où tous ces groupes peuvent être non substitués ou mono- ou polysubstitués par L,
m représente 0, 1 ou 2,
L représente, pour chaque occurrence, de manière identique ou différente, P-, P-Sp-, OH, CH₂OH, halogène, -CN, -NO₂, -NCO, -NCS, -OCN, -SCN, -C(=O)N(R^{x})₂, -C(=O)Y¹, -C(=O)R^{x}, -N(R^{x})₂, silyle en option substitué ou un groupe carbone ou un groupe hydrocarbone en option substitué,
R^{x} représente P-, P-Sp-, H, halogène, alkyle en chaîne droite, ramifié ou cyclique comportant de 1 à 25, de façon préférable de 1 à 12, atomes de C, où, en outre, un ou plusieurs groupes CH₂ non adjacents peut/ peuvent être remplacé(s) par -O-, -S-, -CO-, -CO-O-, -O-CO-, -O-CO-O- de telle sorte que des atomes de O et/ou de S ne soient pas liés directement les uns aux autres, et où, en outre, un ou plusieurs atomes de H peut/peuvent être remplacé(s) par F, Cl, P- ou P-Sp-,
Y¹ représente halogène,
Z¹, Z² représentent, chacun indépendamment de l'autre, -CO-O-, -OCO-, -CY=CY-, -C≡C- ou une liaison simple,
Y représente, pour chaque occurrence, de manière identique ou différente, H ou F,
où au moins l'un des radicaux Z¹ et Z² représente -C≡C-,
dans des milieux LC et des affichages LC du type PS ou PSA.

2. Utilisation selon la revendication 1, **caractérisée en ce que**, pour les composés de la formule I,
A¹ et A² représentent, chacun indépendamment de l'autre, 1,4-phénylène, naphtalène-2,6-diyle, phénanthrène-2,7-diyle ou anthracène-2,7-diyle, où tous ces groupes peuvent être non substitués ou mono- ou polysubstitués par L, et
L représente P-Sp-, OH, CH₂OH, F, Cl, Br, l, -CN, -NO₂, -NCO, -NCS, -OCN, -SCN, -C(=O)N(R^{x})₂, -C(=O)Y₁, -C(=O)R^{x}, -N(R^{x})₂, silyle en option substitué, aryle en option substitué comportant de 6 à 20 atomes de C, alkyle ou alcoxy en chaîne droite ou ramifié comportant de 1 à 25, de façon préférable de 1 à 12, atomes de C, ou alkényle, alkynyle, alkylcarbonyle, alcoxycarbonyle, alkylcarbonyloxy ou alcoxycarbonyloxy en chaîne droite ou ramifié comportant de 2 à 25, de façon préférable de 2 à 12, atomes de C, où, en outre, un ou plusieurs atomes de H dans tous ces groupes peut/peuvent être remplacé(s) par F, CI, P- ou P-Sp-,
où R^{x} et Y¹ présentent la signification indiquée selon la revendication 1.

3. Utilisation selon la revendication 1 ou 2, **caractérisée en ce que** les composés de la formule I sont choisis parmi le groupe constitué par les sous-formules qui suivent : 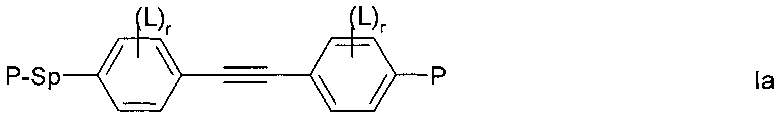 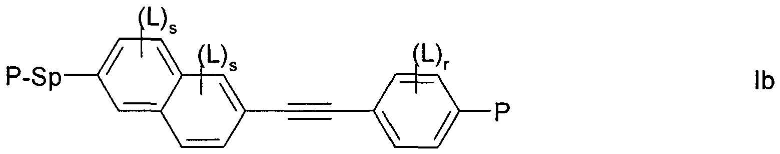 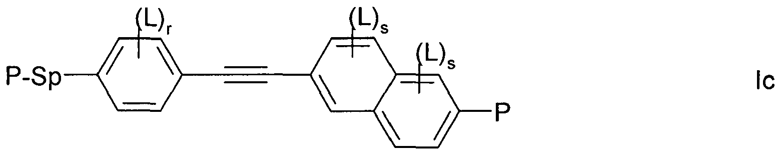 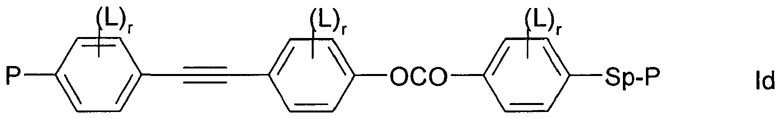 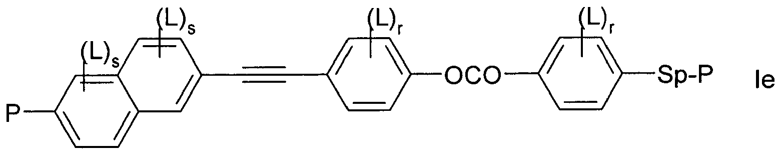 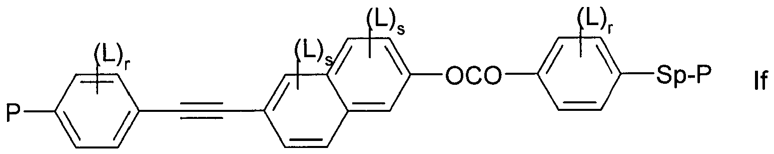 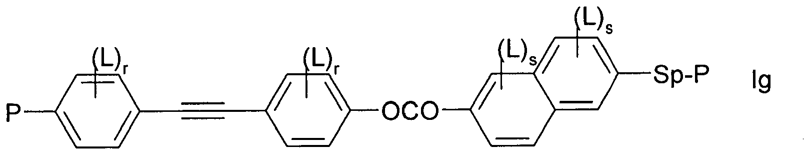 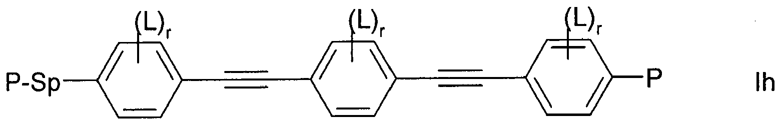 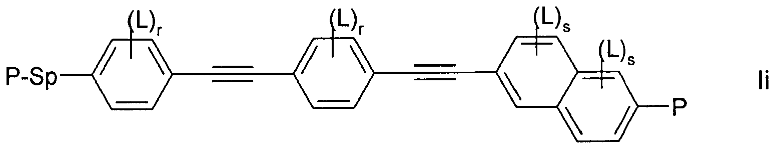 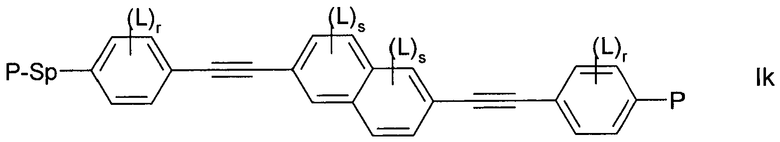 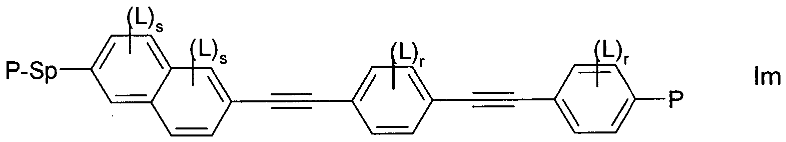 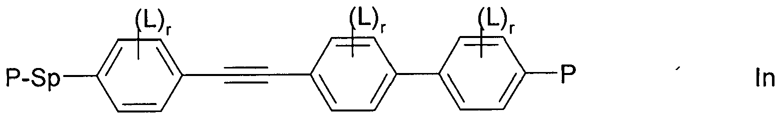 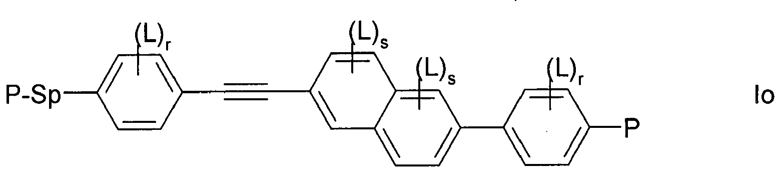 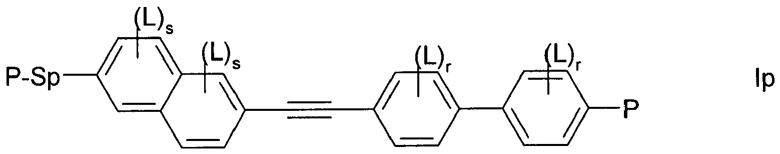 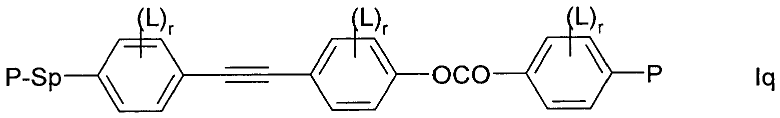 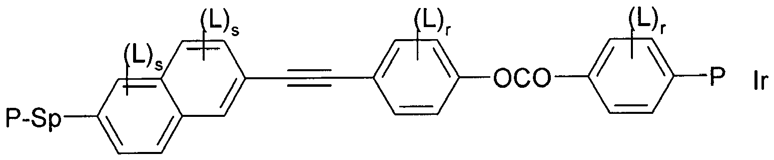 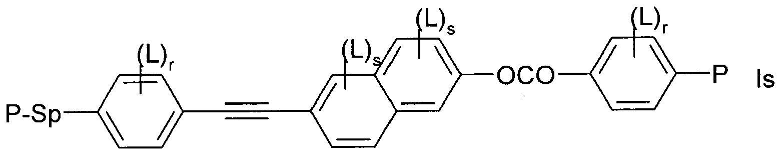 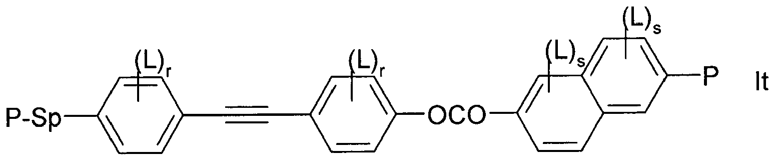 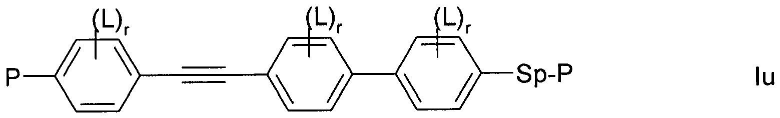 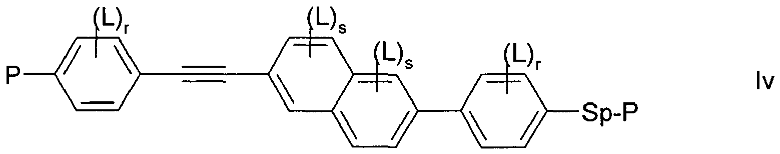 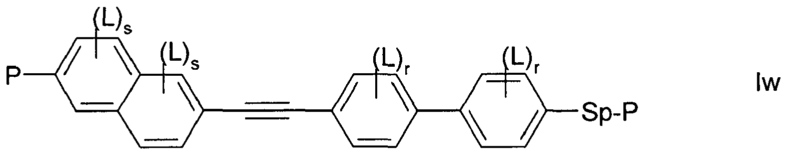 dans lesquelles P, Sp et L présentent la signification indiquée selon la revendication 1, r représente 0, 1, 2, 3 ou 4, et s représente 0, 1, 2 ou 3.

4. Utilisation de composés selon une ou plusieurs des revendications 1 à 3 dans des affichages LC du type PS ou PSA contenant une cellule LC comportant deux substrats et deux électrodes, où au moins un substrat est transparent vis-à-vis de la lumière et au moins un substrat comporte une ou deux électrodes, et une couche, située entre les substrats, d'un milieu LC comprenant un composant polymérisé et un composant de poids moléculaire faible, où le composant polymérisé peut être obtenu par polymérisation d'un ou de plusieurs composé(s) polymérisable(s) entre les substrats de la cellule LC dans le milieu LC, de façon préférable moyennant l'application d'une tension électrique entre les électrodes, où au moins l'un des composés polymérisables est un composé polymérisable selon une ou plusieurs des revendications 1 à 3.

5. Utilisation selon une ou plusieurs des revendications 1 à 4, **caractérisée en ce que** le milieu LC comprend un ou plusieurs composés des formules CY et/ou PY : 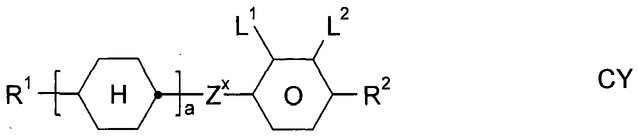 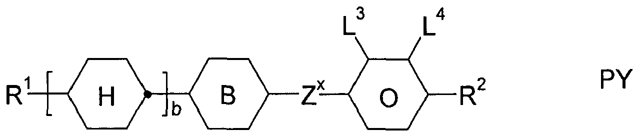 dans lesquelles les radicaux individuels présentent la signification qui suit :
a représente 1 ou 2,
b représente 0 ou 1, 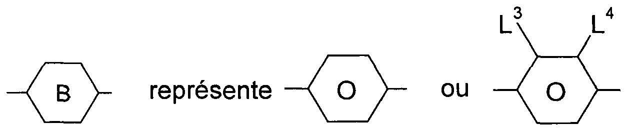
R¹ et R² représentent, chacun indépendamment de l'autre, alkyle comportant de 1 à 12 atomes de C, où, en outre, un ou deux groupes CH₂ non adjacents peut/peuvent être remplacé(s) par -O-, -CH=CH-, -CO-, -O-CO- ou -CO-O- de telle sorte que des atomes de O ne soient pas liés directement les uns aux autres,
Z^{x} représente -CH=CH-, -CH₂O-, -OCH₂-, -CF₂O-, -OCF₂-, -O-, -CH₂-, -CH₂CH₂- ou une liaison simple, de façon préférable une liaison simple,
L¹⁻⁴ représentent, chacun indépendamment des autres, F, CI, OCF₃, CF₃, CH₃, CH₂F, CHF₂.

6. Utilisation selon une ou plusieurs des revendications 1 à 5, **caractérisée en ce que** le milieu LC comprend un ou plusieurs composés de la formule qui suit : 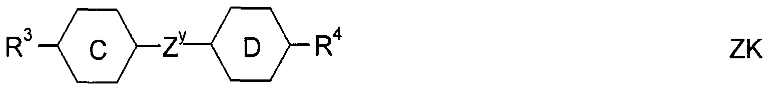 dans laquelle les radicaux individuels présentent la signification qui suit : 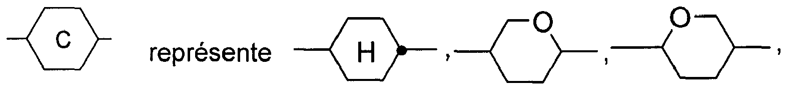 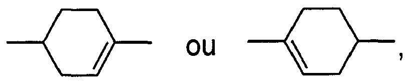 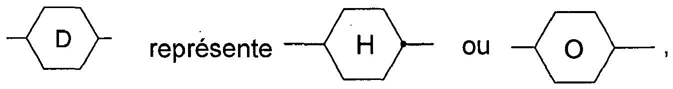
R³ et R⁴ représentent, chacun indépendamment de l'autre, alkyle comportant de 1 à 12 atomes de C, où, en outre, un ou deux groupes CH₂ non adjacents peut/peuvent être remplacé(s) par -O-, -CH=CH-, -CO-, -O-CO- ou -CO-O- de telle sorte que des atomes de O ne soient pas liés directement les uns aux autres,
Z^{y} représente -CH₂CH₂-, -CH=CH-, -CF₂O-, -OCF₂-, -CH₂O-, -OCH₂-, -COO-, -OCO-, -C₂F₄-, -CF=CF- ou une liaison simple.

7. Affichage LC selon une ou plusieurs des revendications 1 à 6.

8. Affichage LC selon la revendication 7, **caractérisé en ce qu'**il s'agit d'un affichage PSA-VA, PSA-OCB, PSA-IPS, PSA-FFS, PSA-positif-VA ou PSA-TN.

9. Milieu LC comprenant :
- un composant polymérisable A) comprenant un ou plusieurs composés polymérisables, et
- un composant cristallin liquide B) comprenant un ou plusieurs composés de poids moléculaire faible,
**caractérisé en ce que** le composant A) comprend un ou plusieurs composés polymérisables selon une ou plusieurs des revendications 1 à 3, et le composant B) comprend un ou plusieurs composés choisis parmi les formules CY et PY selon la revendication 5.

10. Milieu LC comprenant :
- un composant polymérisable A) comprenant un ou plusieurs composés polymérisables, et
- un composant cristallin liquide B) comprenant un ou plusieurs composés de poids moléculaire faible,
**caractérisé en ce que** le composant A) comprend un ou plusieurs composés polymérisables selon une ou plusieurs des revendications 1 à 3, où la concentration des composés polymérisables dans le milieu LC est < 5% en poids.

11. Milieu LC selon la revendication 9 ou 10, dans lequel le composant B) comprend un ou plusieurs composés choisis parmi les formules CY, PY et ZK selon la revendication 5 ou 6.

12. Procédé pour la fabrication d'un affichage LC du type PS ou PSA dans lequel un milieu LC selon une ou plusieurs des revendications 9 à 11 est introduit à l'intérieur d'une cellule LC comportant deux substrats et deux électrodes, où au moins un substrat est transparent vis-à-vis de la lumière et au moins un substrat comporte une ou deux électrodes, et les composés polymérisables sont polymérisés, de façon préférable moyennant l'application d'une tension électrique entre les électrodes.
